# EUROPEAN PATENT APPLICATION

(11) **EP 3 517 132 A1**
(43) Date of publication of application: **31.07.2019**
(21) Application number: 18211172.4
(22) Date of filing: 14.03.2014
(51) Int. Cl.: A61K 47/68, A61P 35/00

(54) **ANTI-EGFR ANTIBODY DRUG CONJUGATE FORMULATIONS**

(30) Priority: 15.03.2013 US 201361790490 P
(62) Divisional of application: 14722450.5
(71) Applicant: AbbVie Deutschland GmbH & Co KG, 65205 Wiesbaden (DE); AbbVie Inc., North Chicago, IL 60064 (US)
(72) Inventor: TSCHOEPE, Markus, 67258 Hessheim (DE); KALETA, Katharina, 67071 Ludwigshafen (DE); KUMAR, Vineet, Storrs, CT Connecticut 06268 (US)
(74) Representative: Miller, David James

(57) **Abstract**

The invention provides a stable formulation comprising an anti-EGFR antibody drug conjugate (ADC), including an anti-EGFR antibody, e.g., antibody 1, conjugated to an auristatin, e.g., MMAF, histidine, a sugar, and a surfactant.

## Description

### RELATED APPLICATIONS

This application claims the benefit of U.S. Provisional Application No. 61/790,490, filed on March 15, 2013. The contents of the aforementioned priority document are hereby incorporated by reference in its entirety.

### INTRODUCTION

Human epidermal growth factor receptor (also known as HER-1 or Erb-B1, and referred to herein as "EGFR") is a 170 kDa transmembrane receptor encoded by the c-erbB protooncogene, and exhibits intrinsic tyrosine kinase activity (Modjtahedi et al., Br. J. Cancer 73:228-235 (1996); Herbst and Shin, Cancer 94:1593-1611 (2002)). SwissProt database entry P00533 provides the sequence of EGFR. EGFR regulates numerous cellular processes via tyrosine-kinase mediated signal transduction pathways, including, but not limited to, activation of signal transduction pathways that control cell proliferation, differentiation, cell survival, apoptosis, angiogenesis, mitogenesis, and metastasis (Atalay et al., Ann. Oncology 14:1346-1363 (2003); Tsao and Herbst, Signal 4:4-9 (2003); Herbst and Shin, Cancer 94:1593-1611 (2002); Modjtahedi et al., Br. J. Cancer 73:228-235 (1996)).

Overexpression of EGFR has been reported in numerous human malignant conditions, including cancers of the bladder, brain, head and neck, pancreas, lung, breast, ovary, colon, prostate, and kidney. (Atalay et al., Ann. Oncology 14:1346-1363 (2003); Herbst and Shin, Cancer 94:1593-1611 (2002) Modjtahedi et al., Br. J. Cancer 73:228-235 (1996)). In many of these conditions, the overexpression of EGFR correlates or is associated with poor prognosis of the patients. (Herbst and Shin, Cancer 94:1593-1611 (2002) Modjtahedi et al., Br. J. Cancer 73:228-235 (1996)). EGFR is also expressed in the cells of normal tissues, particularly the epithelial tissues of the skin, liver, and gastrointestinal tract, although at generally lower levels than in malignant cells (Herbst and Shin, Cancer 94:1593-1611 (2002)).

A significant proportion of tumors containing amplifications of the EGFR gene (i.e., multiple copies of the EGFR gene) also co-express a truncated version of the receptor (Wikstrand et al. (1998) J. Neurovirol. 4, 148-158) known as de2-7 EGFR, ΔEGFR, or Δ2-7 (terms used interchangeably herein) (Olapade-Olaopa et al. (2000) Br. J. Cancer. 82, 186-94). The rearrangement seen in the de2-7 EGFR results in an in-frame mature mRNA lacking 801 nucleotides spanning exons 2-7 (Wong et al. (1992) Proc. Natl. Acad. Sci. U.S.A. 89, 2965-9; Yamazaki et al. (1990) Jpn. J. Cancer Res. 81, 773-9; Yamazaki et al. (1988) Mol. Cell. Biol. 8, 1816-20; Sugawa et al. (1990) Proc. Natl. Acad. Sci. U.S.A. 87, 8602-6). The corresponding EGFR protein has a 267 amino acid deletion comprising residues 6-273 of the extracellular domain and a novel glycine residue at the fusion junction (Sugawa et al., 1990). This deletion, together with the insertion of a glycine residue, produces a unique junctional peptide at the deletion interface (Sugawa et al., 1990).

The de2-7 EGFR has been reported in a number of tumor types including glioma, breast, lung, ovarian and prostate (Wikstrand et al. (1997) Cancer Res. 57, 4130-40; Olapade-Olaopa et al. (2000) Br. J. Cancer. 82, 186-94; Wikstrand, et al. (1995) Cancer Res. 55, 3140-8; Garcia de Palazzo et al. (1993) Cancer Res. 53, 3217-20). While this truncated receptor does not bind ligand, it possesses low constitutive activity and imparts a significant growth advantage to glioma cells grown as tumor xenografts in nude mice (Nishikawa et al. (1994) Proc. Natl. Acad. Sci. U.S.A. 91, 7727-31) and is able to transform NIH3T3 cells (Batra et al. (1995) Cell Growth Differ. 6, 1251-9) and MCF-7 cells. The cellular mechanisms utilized by the de2-7 EGFR in glioma cells are not fully defined but are reported to include a decrease in apoptosis (Nagane et al. (1996) Cancer Res. 56, 5079-86) and a small enhancement of proliferation (Nagane et al., 1996).

As expression of this truncated receptor is restricted to tumor cells it represents a highly specific target for antibody therapy. Accordingly, there is a need in the art for anti-EGFR antibodies and formulations that can provide effective treatment to those in need thereof.

Antibody drug conjugates (ADC) represent a new class of therapeutics comprising an antibody conjugated to a cytotoxic drug via a chemical linker. A number of ADCs are being tested in clinical trials today, including trastuzumab (Herceptin (anti-HER2 antibody) linked to DM1; Genentech / Roche) and glembatumumab vedotin (CDX-011; an anti-GPNMB antibody linked to MMAE; Celldex Therapeutics). The therapeutic concept of ADCs is to use an antibody as a vehicle to deliver a cytotoxic drug to a tumor cell by means of binding to a target surface antigen. ADCs have more complex and heterogeneous structures than the unconjugated antibodies. Accordingly, ADCs present a challenge for formulating for therapeutic purposes.

### SUMMARY OF INVENTION

The present invention provides formulations comprising antibody-drug conjugates (ADCs) and methods relating to the use of such conjugates to treat disorders requiring treatment with an anti-epidermal growth factor receptor (anti-EGFR) ADC, such as cancer.

The invention provides a formulation comprising an anti-Epidermal Growth Factor Receptor (EGFR) antibody drug conjugate (ADC), a sugar, histidine, and a surfactant, wherein the formulation has a pH of about 5 - 7, and wherein the anti-EGFR ADC comprises an anti-EGFR antibody, or antigen-binding portion thereof, conjugated to an auristatin.

The invention provides a formulation comprising an anti-Epidermal Growth Factor Receptor (EGFR) antibody drug conjugate (ADC), a sugar, a succinate or citrate and/or phosphate buffer, and a surfactant, wherein the formulation has a pH of about 5 - 7, and wherein the anti-EGFR ADC comprises an anti-EGFR antibody, or antigen-binding portion thereof, conjugated to an auristatin. In one embodiment, the formulation is lyophilized.

In one embodiment, the formulation comprises a surfactant which is a polysorbate, *e.g*., polysorbate 80, or a poloxamer. In one embodiment, the formulation comprises 0.05-0.15 mg/ml of a polysorbate.

In another embodiment, the formulation comprises about 60-80 mg/ml of a sugar. In one embodiment, the sugar is selected from the group consisting of mannitol, sorbitol, sucrose, and trehalose.

In one embodiment, the formulation of the invention comprises about 5-25 mM histidine.

In another further embodiment, the formulation is lyophilized. In one embodiment, the formulation is lyophilized and the sugar is sucrose. In another embodiment of the invention, the lyophilized formulation does not comprise lactobionic acid. In another embodiment of the invention, the lyophilized formulation does not comprise a sugar acid.

In one embodiment, the formulation of the invention is aqueous, and comprises about 1-40 mg/ml of an anti-EGFR ADC.

In a further embodiment, the formulation of the invention comprises 1-150 mg of the anti-EGFR ADC. In one embodiment, the formulation of the invention comprises 90-110 mg of an anti-EGFR ADC.

In one embodiment, the formulation of the invention has a pH of about 5 to 7. In a further embodiment, the formulation has a pH of about 5.5 to 6.5.

In one embodiment, the auristatin is Monomethylauristatin F (MMAF). In one embodiment, the formulation of the invention comprises an anti-EGFR ADC comprising an anti-EGFR antibody, or antigen-binding portion thereof, conjugated to Monomethylauristatin F (MMAF). In another embodiment, the MMAF is conjugated to the antibody with a maleimidocaproyl linker.

The invention further provides a lyophilized formulation comprising an anti-Epidermal Growth Factor Receptor (EGFR) antibody drug conjugate (ADC) comprising an anti-EGFR antibody, or antigen-binding portion thereof, conjugated to an auristatin, a sugar, *e.g.,* sucrose, a surfactant, *e.g.,* a polysorbate, such as polysorbate 80, and histidine. In one embodiment, the lyophilized formulation comprises 1-20 mg of histidine, about 320-410 mg of a sugar, about 0.1 to 0.9 mg of a surfactant, and about 1-150 mg of the anti-EGFR ADC.

The invention also provides an aqueous formulation comprising about 1 - 100 mg/ml of an anti-Epidermal Growth Factor Receptor (EGFR) antibody drug conjugate (ADC) comprising an anti-EGFR antibody, or antigen-binding portion thereof, conjugated to an auristatin, about 1 -10 mg/mL histidine, about 50 -90 mg/ml of a sugar, *e.g*., sucrose, and about 0.01 - 0.2 mg/ml of a surfactant, *e.g*., polysorbate 80.

In one embodiment, the formulation of the invention comprises an anti-EGFR ADC wherein the anti-EGFR antibody is humanized.

In another embodiment, the formulation of the invention comprises an anti-EGFR ADC wherein the anti-EGFR antibody comprises a heavy chain variable region comprising the amino acid sequence set forth in SEQ ID NO: 13, and a light chain variable region comprising the amino acid sequence set forth in SEQ ID NO: 18.

In another embodiment, the formulation of the invention comprises an anti-EGFR ADC wherein the anti-EGFR antibody comprises a heavy chain variable region having the amino acid sequence set forth in SEQ ID NO: 13, a heavy chain constant region having the amino acid sequence as set forth in SEQ ID NO: 14, a light chain variable region having the amino acid sequence set forth in SEQ ID NO: 18, and a light chain constant region having the amino acid sequence as set forth in SEQ ID NO: 19,

In a further embodiment, the formulation of the invention comprises an anti-EGFR ADC wherein the antibody comprises a heavy chain variable region comprising complementarity domain regions (CDRs) comprising the amino acid sequences set forth in SEQ ID NOS: 15, 16, and 17, and comprises a light chain variable region comprising CDRs comprising the amino acid sequences set forth in SEQ ID NOS: 20, 21, and 22.

In another embodiment of the invention, the anti-EGFR ADC comprises CDRs (*i.e.,* light chain CDR1, CDR2, and CDR3) described in the light chain variable region set forth in the amino acid sequence of SEQ ID NO: 18, and CDRs (*i.e.,* heavy chain CDR1, CDR2, and CDR3) described in the amino acid sequence of SEQ ID NO: 13.

The invention further provides a lyophilized formulation comprising sucrose, polysorbate 80, and histidine, and wherein the antibody comprises a heavy chain variable region comprising complementarity domain regions (CDRs) comprising the amino acid sequences set forth in SEQ ID NOS: 15, 16, and 17, and comprises a light chain variable region comprising CDRs comprising the amino acid sequences set forth in SEQ ID NOS: 20, 21, and 22. In one embodiment, the formulation comprises 1-110 mg of the anti-EGFR ADC.

In one embodiment, the formulation of the invention contains an ADC mixture comprising anti-EGFR ADCs having an average DAR of about 3 or an ADC mixture comprising anti-EGFR ADCs having a DAR of about 2-4.

In one embodiment, the formulation of the invention comprises an anti-EGFR ADC comprising an anti-EGFR antibody, or antigen-binding portion thereof, conjugated to monomethyl auristatin F (MMAF), wherein said ADC 1-MMAF comprises a heavy chain variable region comprising complementarity domain regions (CDRs) comprising the amino acid sequences set forth in SEQ ID NOS: 15, 16, and 17, and comprises a light chain variable region comprising CDRs comprising the amino acid sequences set forth in SEQ ID NOS: 20, 21, and 22, sucrose, histidine, and polysorbate 80, wherein the formulation comprises an ADC mixture having an average DAR of about 3 or an ADC mixture having a DAR of about 2-4. In a further embodiment of the invention, the formulation is lyophilized. In yet a further invention, the anti-EGFR antibody is linked to MMAF via a maleimidocaproyl linker. In still a further embodiment, the anti-EGFR antibody, or antigen-binding portion thereof, comprises a heavy chain variable region comprising the amino acid sequence set forth in SEQ ID NO: 13, and a light chain variable region comprising the amino acid sequence set forth in SEQ ID NO: 18.

In one embodiment, the formulation of the invention comprises an anti-EGFR ADC comprising an anti-EGFR antibody, or antigen-binding portion thereof, conjugated to monomethyl auristatin E (MMAE), wherein said ADC 1-MMAE comprises a heavy chain variable region comprising complementarity domain regions (CDRs) comprising the amino acid sequences set forth in SEQ ID NOS: 15, 16, and 17, and comprises a light chain variable region comprising CDRs comprising the amino acid sequences set forth in SEQ ID NOS: 20, 21, and 22, sucrose, histidine, and polysorbate 80, wherein the formulation comprises an ADC mixture having an average DAR of about 3 or an ADC mixture having a DAR of about 2-4. In a further embodiment of the invention, the formulation is lyophilized. In still a further embodiment, the anti-EGFR antibody, or antigen-binding portion thereof, comprises a heavy chain variable region comprising the amino acid sequence set forth in SEQ ID NO: 13, and a light chain variable region comprising the amino acid sequence set forth in SEQ ID NO: 18.

In one embodiment, the formulation of the invention is a pharmaceutical formulation.

The invention also features a method of preparing a formulation, wherein the method comprises lyophilizing an aqueous formulation having a pH ranging from about 5 to 7 and comprising 1-20 mg of histidine, about 320-410 mg of the sugar, about 0.1 to 0.9 mg of the surfactant, and about 1-150 mg of the anti-EGFR ADC.

The invention further provides a method for treating a subject comprising administering a therapeutically effective amount of an anti-EGFR ADC in the formulations described herein to a subject, wherein the subject has a disorder requiring treatment with the anti-EGFR ADC. In one embodiment, the disorder requiring treatment with the anti-EGFR ADC is cancer, *e.g*., glioblastoma, non-small cell lung cancer, lung cancer, colon cancer, head and neck cancer, breast cancer, squamous cell tumors, anal cancer, skin cancer, and vulvar cancer.

In one embodiment, the formulation of the invention is used to treat solid tumors likely to over-express the Epidermal Growth Factor Receptor (EGFR) or squamous non-small cell lung cancer (NSCLC).

In one embodiment, the formulation of the invention is used to treat a cancer selected from the group consisting of squamous tumors (including, squamous tumors of the lung, head and neck, cervical, etc.), glioblastoma, glioma, non-small cell lung cancer, lung cancer, colon cancer, head and neck cancer, breast cancer, squamous cell tumors, anal cancer, skin cancer, and vulvar cancer.

In one embodiment, the formulation of the invention is used to treat glioblastoma multiforme.

In one embodiment, the formulation of the invention is used to treat a solid tumor having overexpression of EGFR. In one embodiment, the formulation of the invention is used to treat a subject having an advanced solid tumor likely to overexpress EGFR.

In one embodiment, the formulations of the invention are administered intravenously.

The invention also provides kits comprising the formulations described herein.

### BRIEF DESCRIPTION OF THE DRAWINGS

**Figure 1** is a diagram that shows the structure of MMAE coupled to an antibody of interest (mAb) by a linker (GN Linkers + Auristatin: vcMMAE).
**Figure 2** is a diagram that shows the structure of MMAF coupled to an antibody of interest (mAb) by a linker (Linkers + Auristatin: mcMMAF).
**Figure 3** **(A and B)** graphically depict the onset of unfolding temperature as determined with dynamic scanning fluorescence (A) and differential scanning calorimetry (B). ◆ antibody 1; ■ Antibody Drug Conjugate (ADC) 1- MMAF; ▲ ADC 1- MMAE.
**Figure 4** **(A and B)** graphically depicts the presence of unstructured elements in antibody 1, ADC 1- MMAF and ADC 1- MMAE using Fourier Transform Infrared Spectroscopy (FTIR) (A) and near UV- Circular Dichroism (CD) (B). ◆ antibody 1; ■ ADC 1- MMAF; ▲ ADC 1-MMAE.
**Figure 5** graphically depicts the aggregation propensity of ADC 1- MMAE at low concentrations, compared to ADC 1- MMAF and antibody 1.
**Figure 6** graphically depicts the aggregation propensity of ADC 1- MMAE at high concentrations, compared to ADC 1- MMAF and antibody 1.
**Figure 7** shows a schematic of the serum stability assay, and describes the *in vitro* serum stability of a number of molecules, including antibody 1, the ADC 1- MMAF and ADC 1-MMAE.
**Figure 8** **(A and B)** graphically depicts freeze/thaw stability, indicated by percentage of monomer present, as determined by SEC among antibody 1, ADC 1- MMAF and ADC 1-MMAE formulations in 15 mM histidine buffer at pH 5.75 (A) and 1 mg/ml pH 7.0 10 mM citrate and 10 mM phosphate buffer.
**Figure 9** **(A and B)** is a graph quantifying subvisible particle formation during freeze-thawing as determined by micro flow imaging (MFI).

### DETAILED DESCRIPTION

### I. Definitions

In order that the present invention may be more readily understood, certain terms are first defined. In addition, it should be noted that whenever a value or range of values of a parameter are recited, it is intended that values and ranges intermediate to the recited values are also intended to be part of this invention.

The terms "anti-Epidermal Growth Factor antibody drug conjugate" or "anti-EGFR antibody drug conjugate" and "anti-EGFR ADC", used interchangeably herein, refer to an antibody-drug conjugate comprising an antibody that specifically binds to EGFR, whereby the antibody is conjugated to a drug, *e.g.,* a cytotoxic agent such as an auristatin (*e.g.,* monomethyl auristatin F). In one embodiment, the anti-EGFR antibody drug conjugate is the ADC 1-MMAF, which is antibody 1 conjugated to MMAF via a maleimidocaproyl (mc) linkage. Amino acid sequences corresponding to the light and heavy chains of antibody 1 are provided in SEQ ID NOs: 13 to 22. Unless otherwise specified, the term "ADC1-MMAF" as used herein in inclusive of purified ADC1-MMAF (also referred to as ADC1-MMAFp).

The term "auristatin", as used herein, refers to a family of antimitotic agents. Auristatin derivatives are also included within the definition of the term "auristatin". Examples of auristatins include, but are not limited to, auristatin E (AE), monomethyl auristatin E (MMAE), monomethyl auristatin F (MMAF), and synthetic analogs of dolastatin.

The term "anti-EGFR antibody" is meant to refer to an antibody that specifically binds to EGFR. An antibody "which binds" an antigen of interest, *i.e.,* EGFR, is one capable of binding that antigen with sufficient affinity such that the antibody is useful in targeting a cell expressing the antigen.

The term "ADC mixture," as used herein, refers to a composition containing a heterogeneous DAR distribution of ADCs. In one embodiment, an ADC mixture contains anti-EGFR ADCs having a distribution of DARs of 1 to 8, *e.g.,* 2, 4, 6, and 8 (*i.e.,* drug loaded species of 2, 4, 6, and 8). In another embodiment, an ADC mixture contains anti-EGFR ADCs having DARs of less than 4, *e.g.,* a DAR of 2-4. Notably, degradation products may result such that DARs of 1, 3, 5, and 7 may also be included in the mixture. Further, ADCs within the mixture may also have DARs greater than 8. The ADC mixture results from interchain disulfide reduction followed by conjugation.

The term "ADC1-MMAFp", as used herein, refers to an ADC1-MMAF molecule that is found within an ADC mixture that has been purified such that the DAR of the ADC is within a desired range, *e.g.,* (a DAR of 2-4) or the ADC is included in an ADC mixture having a desired average, (*e.g.,* an average DAR of 3).

The term "formulation" as used herein is meant to refer to an aqueous formulation or a lyophilized formulation. In one embodiment, the formulation of the invention is lyophilized. In one embodiment, the formulation of the invention is aqueous. In one embodiment, the formulation of the invention does not include a chelator. In one embodiment, the lyophilized formulation of the invention does not include a chelator.

The term "aqueous formulation" refers to a solution in which the solvent is water. In one embodiment, the term "aqueous formulation" refers to a liquid formulation in which the solvent is water wherein the formulation was not previously lyophilized, (i.e., does not result from reconstitution of a lyophilized formulation). In another embodiment, the term "aqueous formulation" refers to a liquid formulation in which the solvent is water wherein the formulation was previously lyophilized (i.e., a reconstituted formulation).

The term "lyophilized" as used herein in connection with the formulation according to the invention denotes a formulation which is dried by freezing the formulation and subsequently subliming the ice from the frozen content by any freeze-drying methods known in the art, for example commercially available freeze-drying devices.

A "reconstituted" formulation is one which has been prepared by dissolving a lyophilized formulation containing a protein, *e.g.,* an ADC, in a diluent such that the protein is dispersed in the reconstituted formulation. The reconstituted formulation is suitable for administration (*e.g.* intravenous administration) to a subject to be treated with the protein of interest (*e.g*., anti-EGFR antibody drug conjugate).

A "diluent" of interest herein is one which is pharmaceutically acceptable (safe and non-toxic for administration to a human) and is useful for the preparation of a liquid formulation, such as a formulation reconstituted after lyophilization. Exemplary diluents include sterile water, bacteriostatic water for injection (BWFI), a pH buffered solution (*e.g*. phosphate-buffered saline), sterile saline solution, Ringer's solution or dextrose solution. In an alternative embodiment, diluents can include aqueous solutions of salts and/or buffers.

The term "buffer" refers to a compound that resists changes in pH by the action of its acid-base conjugate components when in solution. In one embodiment, a buffer used in this invention has a pH in the range from about 4.5 to about 7.5. Examples of buffers that will control the pH in this range include acetate (*e.g*., sodium acetate), succinate (such as sodium succinate), gluconate, methionine, imidazole, histidine, glycine, arginine, citrate, phosphate, citrate and phosphate, Tris, and other organic acid buffers. In one embodiment, the buffer used in the formulation comprises histidine, succinate, or citrate or phosphate buffer. In one embodiment of the invention, the formulation comprises about 1 -10 mg/mL of a buffer comprising histidine. In another embodiment of the invention, the formulation comprises 1-20 mg of a buffer comprising histidine.

The term "sugar" is meant to refer to as used herein denotes a monosaccharide or an oligosaccharide. A monosaccharide is a monomeric carbohydrate which is not hydrolysable by acids, including simple sugars and their derivatives, *e.g*. aminosugars. Examples of monosaccharides include, but are not limited to, glucose, fructose, galactose, mannose, sorbose, ribose, deoxyribose, neuraminic acid. An oligosaccharide is a carbohydrate consisting of more than one monomeric saccharide unit connected via glycosidic bond(s) either branched or in a chain. The monomeric saccharide units within an oligosaccharide can be identical or different. Depending on the number of monomeric saccharide units the oligosaccharide is a di-, tri-, tetra-, penta- and so forth saccharide. Examples of oligosaccharides include, but are not limited to, maltose, sucrose, lactose, melezitose, trehalose, sorbose, and raffinose. In contrast to polysaccharides, monosaccharides and oligosaccharides are water soluble. Examples of sugars include, but not limited to, a reducing sugar, a nonreducing sugar, a sugar alcohol, and a sugar acid. A "reducing sugar" is a sugar that contains a free aldehyde or ketone group and can reduce metal ions or react covalently with lysine and other amino groups in proteins. A "nonreducing sugar" is a sugar that lacks a free aldehyde or ketonic group and is not oxidized by mild oxidizing agents such as Fehling's or Benedict's solutions. Examples of reducing sugars are fructose, mannose, maltose, lactose, arabinose, xylose, ribose, rhamnose, galactose and glucose. Nonreducing sugars include sucrose, trehalose, sorbose, melezitose and raffinose. Mannitol, xylitol, erythritol, threitol, sorbitol and glycerol are examples of sugar alcohols. In one embodiment of the invention, the sugar is mannitol, sorbitol, sucrose, or trehalose. In another embodiment of the invention, the formulation comprises about 50 -90 mg/ml of a sugar, *e.g*., sucrose. In another further embodiment of the invention, the formulation comprises about 60-80 mg/ml of a sugar, *e.g*., sucrose. In another further embodiment of the invention, the formulation comprises about 70 mg/ml of a sugar, *e.g*., sucrose. In still another further embodiment of the invention, the lyophilized formulation comprises about 320-410 mg of a sugar.

The term "surfactant" generally refers to organic substances having amphipathic structures; namely, they are composed of groups of opposing solubility tendencies, typically an oil-soluble hydrocarbon chain and a water-soluble ionic group. Surfactants can be classified, depending on the charge of the surface-active moiety, into anionic, cationic, and nonionic surfactants. Surfactants are often used as wetting, emulsifying, solubilizing, and dispersing agents for various pharmaceutical compositions and preparations of biological materials. Examples of suitable surfactants include, but are not limited to, sodium lauryl sulfate, polysorbates such as polyoxyethylene sorbitan monooleate, monolaurate, monopalmitate, monstearate or another ester of polyoxyethylene sorbitan (*e.g*., the commercially available TWEENS, such as, TWEEN 20 and TWEEN 80 (ICI Speciality Chemicals)), sodium dioctylsulfosuccinate (DOSS), lecithin, stearylic alcohol, cetostearylic alcohol, cholesterol, polyoxyethylene ricin oil, polyoxyethylene fatty acid glycerides, poloxamers (*e.g*., Pluronics F68™ and F108™, which are block copolymers of ethylene oxide and propylene oxide); polyoxyethylene castor oil derivatives or mixtures thereof.

The term "polysorbate" as used herein refers to oleate esters of sorbitol and its anhydrides, typically copolymerized with ethylene oxide. In one embodiment, the polysorbate has a molecular weight ranging from about 1200 Da (approximate molecular weight of polysorbate 20) to about 1350 Da (approximate molecular weight of polysorbate 80). In one embodiment, a formulation comprises polysorbate 20, polysorbate 40, polysorbate 60, or polysorbate 80. In one embodiment, the formulation comprises about 0.1 to 0.9 mg of a polysorbate. In another embodiment, the formulation comprises about 0.01 - 0.2 mg/ml of a polysorbate. In another embodiment, the formulation comprises 0.05-0.15 mg/ml of a polysorbate.

The term "pharmaceutical formulation" refers to preparations that are in such a form as to permit the biological activity of the active ingredient(s) to be effective and, therefore, may be administered to a subject for therapeutic use.

A "stable" formulation is one in which the anti-EGFR antibody drug conjugate essentially retains its physical stability and/or chemical stability and/or biological activity upon storage. Various analytical techniques for measuring protein stability are available in the art and are reviewed in, *e.g.,* Peptide and Protein Drug Delivery, pp. 247-301, Vincent Lee Ed., Marcel Dekker, Inc., New York, N.Y., Pubs. (1991) and Jones (1993) Adv. Drug Delivery Rev. 10: 29-90. In one embodiment, the stability of the anti-EGFR antibody drug conjugate is determined according to the percentage of monomer protein in the solution, with a low percentage of degraded (*e.g*., fragmented) and/or aggregated protein. For example, a formulation comprising a stable anti-EGFR antibody drug conjugate may include at least 95% monomer anti-EGFR antibody drug conjugate. Alternatively, a formulation may include no more than 5% aggregate and/or degraded anti-EGFR antibody drug conjugate.

An anti-EGFR antibody drug conjugate "retains its physical stability" in a pharmaceutical formulation if it shows substantially no signs of aggregation, precipitation and/or denaturation upon visual examination of color and/or clarity, or as measured by UV light scattering or by size exclusion chromatography. In one embodiment, a stable aqueous formulation is a formulation having less than about 5% anti-EGFR antibody drug conjugate aggregation in the formulation.

An anti-EGFR antibody drug conjugate "retains its chemical stability" in a pharmaceutical formulation if the chemical stability at a given time is such that the anti-EGFR antibody drug conjugate is considered to still retain its biological activity as defined below. Chemical stability can be assessed by detecting and quantifying chemically altered forms of the anti-EGFR antibody drug conjugate. Chemical alteration may involve size modifications (*e.g*., clipping) which can be evaluated using size exclusion chromatography, SDS-PAGE and/or matrix-assisted laser desorption ionization / time of flight mass spectrometry (MALDI/TOF MS), for example. Other types of chemical alternation include charge alteration (*e.g.,* occurring as a result of deamidation), which can be evaluated by, *e.g.,* ion-exchange chromatography.

An anti-EGFR antibody drug conjugate "retains its biological activity" in a pharmaceutical formulation, if the protein in a pharmaceutical formulation is biologically active for its intended purpose. For example, biological activity of an anti-EGFR antibody drug conjugate is retained if the biological activity of the anti-EGFR antibody drug conjugate in the pharmaceutical formulation is within about 30%, about 20%, or about 10% (within the errors of the assay) of the biological activity exhibited at the time the pharmaceutical formulation was prepared (*e.g.,* as determined in an antigen binding assay). In one embodiment, the biological activity is cytotoxicity of epidermoid carcinoma cells.

The term "antibody" broadly refers to an immunoglobulin (Ig) molecule, generally comprised of four polypeptide chains, two heavy (H) chains and two light (L) chains, or any functional fragment, mutant, variant, or derivative thereof, that retains the essential target binding features of an Ig molecule.

In a full-length antibody, each heavy chain is comprised of a heavy chain variable region (abbreviated herein as HCVR or VH) and a heavy chain constant region. The heavy chain constant region is comprised of three domains, CH1, CH2 and CH3. Each light chain is comprised of a light chain variable region (abbreviated herein as LCVR or VL) and a light chain constant region. The light chain constant region is comprised of one domain, CL. The VH and VL regions can be further subdivided into regions of hypervariability, termed complementarity determining regions (CDR), interspersed with regions that are more conserved, termed framework regions (FR). Each VH and VL is composed of three CDRs and four FRs, arranged from amino-terminus to carboxy-terminus in the following order: FR1, CDR1, FR2, CDR2, FR3, CDR3, FR4. Immunoglobulin molecules can be of any type (*e.g.,* IgG, IgE, IgM, IgD, IgA and IgY) and class (*e.g.,* IgG1, IgG2, IgG 3, IgG4, IgA1 and IgA2) or subclass.

The term "antigen-binding portion" of an antibody (or simply "antibody portion"), as used herein, refers to one or more fragments of an antibody that retain the ability to specifically bind to an antigen (*e.g.,* hIL-13). It has been shown that the antigen-binding function of an antibody can be performed by fragments of a full-length antibody. Such antibody embodiments may also be bispecific, dual specific, or multi-specific formats; specifically binding to two or more different antigens. Examples of binding fragments encompassed within the term "antigen-binding portion" of an antibody include (i) a Fab fragment, a monovalent fragment consisting of the VL, VH, CL and CH1 domains; (ii) a F(ab')₂ fragment, a bivalent fragment comprising two Fab fragments linked by a disulfide bridge at the hinge region; (iii) a Fd fragment consisting of the VH and CH1 domains; (iv) a Fv fragment consisting of the VL and VH domains of a single arm of an antibody, (v) a dAb fragment (Ward et al., (1989) Nature 341:544-546, Winter et al., PCT publication WO 90/05144 A1 herein incorporated by reference), which comprises a single variable domain; and (vi) an isolated complementarity determining region (CDR). Furthermore, although the two domains of the Fv fragment, VL and VH, are coded for by separate genes, they can be joined, using recombinant methods, by a synthetic linker that enables them to be made as a single protein chain in which the VL and VH regions pair to form monovalent molecules (known as single chain Fv (scFv); see *e.g.,* Bird et al. (1988) Science 242:423-426; and Huston et al. (1988) Proc. Natl. Acad. Sci. USA 85:5879-5883). Such single chain antibodies are also intended to be encompassed within the term "antigen-binding portion" of an antibody. Other forms of single chain antibodies, such as diabodies are also encompassed. Diabodies are bivalent, bispecific antibodies in which VH and VL domains are expressed on a single polypeptide chain, but using a linker that is too short to allow for pairing between the two domains on the same chain, thereby forcing the domains to pair with complementary domains of another chain and creating two antigen binding sites (see *e.g.,* Holliger, P., et al. (1993) Proc. Natl. Acad. Sci. USA 90:6444-6448; Poljak, R.J., et al. (1994) Structure 2:1121-1123). Such antibody binding portions are known in the art (Kontermann and Dubel eds., Antibody Engineering (2001) Springer-Verlag. New York. 790 pp. (ISBN 3-540-41354-5).

An "isolated antibody", as used herein, is intended to refer to an antibody that is substantially free of other antibodies having different antigenic specificities (*e.g*., an isolated antibody that specifically binds EGFR is substantially free of antibodies that specifically bind antigens other than EGFR). An isolated antibody that specifically binds EGFR may, however, have cross-reactivity to other antigens, such as EGFR molecules from other species. Moreover, an isolated antibody may be substantially free of other cellular material and/or chemicals.

The term "humanized antibody" refers to antibodies which comprise heavy and light chain variable region sequences from a non-human species (*e.g*., a mouse) but in which at least a portion of the VH and/or VL sequence has been altered to be more "human-like", *i.e.,* more similar to human germline variable sequences. In a particular embodiment, the term "humanized antibody" refers to an antibody or antibody variant, derivative or fragment, which specifically binds to an antigen of interest, and comprises a framework (FR) region having substantially the amino acid sequence of a human antibody, and comprises CDRs having substantially the amino acid sequence of a non-human antibody. As used herein, the term "substantially" in the context of a CDR refers to a CDR having an amino acid sequence at least 80%, preferably at least 85%, at least 90%, at least 95%, at least 98% or at least 99% identical to the amino acid sequence of a non-human antibody CDR. In one embodiment, one type of humanized antibody is a CDR-grafted antibody, in which human CDR sequences are introduced into non-human VH and VL sequences to replace the corresponding nonhuman CDR sequences.

As used herein, the term "CDR" refers to the complementarity determining region within antibody variable sequences. There are three CDRs in each of the variable regions of the heavy chain and the light chain, which are designated CDR1, CDR2 and CDR3, for each of the variable regions. The term "CDR set" as used herein refers to a group of three CDRs that occur in a single variable region capable of binding the antigen. The exact boundaries of these CDRs have been defined differently according to different systems. The system described by Kabat (Kabat et al., Sequences of Proteins of Immunological Interest (National Institutes of Health, Bethesda, Md. (1987) and (1991)) not only provides an unambiguous residue numbering system applicable to any variable region of an antibody, but also provides precise residue boundaries defining the three CDRs. These CDRs may be referred to as Kabat CDRs. Chothia and coworkers (Chothia &Lesk, J. Mol. Biol. 196:901-917 (1987) and Chothia et al., Nature 342:877-883 (1989)) found that certain sub- portions within Kabat CDRs adopt nearly identical peptide backbone conformations, despite having great diversity at the level of amino acid sequence. These sub-portions were designated as L1, L2 and L3 or H1, H2 and H3 where the "L" and the "H" designates the light chain and the heavy chains regions, respectively. These regions may be referred to as Chothia CDRs, which have boundaries that overlap with Kabat CDRs. Other boundaries defining CDRs overlapping with the Kabat CDRs have been described by Padlan (FASEB J. 9:133-139 (1995)) and MacCallum (J Mol Biol 262(5):732-45 (1996)). Still other CDR boundary definitions may not strictly follow one of the above systems, but will nonetheless overlap with the Kabat CDRs, although they may be shortened or lengthened in light of prediction or experimental findings that particular residues or groups of residues or even entire CDRs do not significantly impact antigen binding. The methods used herein may utilize CDRs defined according to any of these systems, although preferred embodiments use Kabat or Chothia defined CDRs.

The term "disorder" refers to any condition that would benefit from treatment with the formulations of the invention, *e.g*., a disorder requiring treatment with the anti-EGFR antibody in the formulation. This includes chronic and acute disorders or diseases including those pathological conditions that predispose the subject to the disorder in question.

The term "cancer" is meant to refer to or describe the physiological condition in mammals that is typically characterized by unregulated cell growth. Examples of cancer include, but are not limited to, carcinoma, lymphoma, blastoma, sarcoma, and leukemia or lymphoid malignancies. More particular examples of such cancers include glioblastoma, non-small cell lung cancer, lung cancer, colon cancer, head and neck cancer, breast cancer, squamous cell tumors, anal cancer, skin cancer, and vulvar cancer. In one embodiment, the formulation is administered to a patient having a tumor(s) containing amplifications of the EGFR gene, whereby the tumor expresses the truncated version of the EGFR de2-7. In one embodiment, the formulation comprising ADC1-MMAF may be administered to a subject for the treatment of colorectal cancer, head and neck cancer (including, but not limited to, hypopharyngeal cancer, oropharyngeal cancer, esophageal cancer, laryngeal cancer, and oral cavity cancer), non-small cell lung cancer, pancreatic cancer, gastric cancer, breast cancer, a solid tumor, *e.g.,* a solid tumor likely to over-express the Epidermal Growth Factor Receptor (EGFR), or glioblastoma multiforme.

The term "administering" as used herein is meant to refer to the delivery of a substance (*e.g*., an anti-EGFR antibody drug conjugate) to achieve a therapeutic objective (*e.g.,* the treatment of an EGFR- associated disorder). Modes of administration may be parenteral, enteral and topical. Parenteral administration is usually by injection, and includes, without limitation, intravenous, intramuscular, intraarterial, intrathecal, intracapsular, intraorbital, intracardiac, intradermal, intraperitoneal, transtracheal, subcutaneous, subcuticular, intraarticular, subcapsular, subarachnoid, intraspinal and intrasternal injection and infusion.

The term "therapeutically effective amount" or "effective amount" of an ADC as used herein, refers to an amount effective in the prevention or treatment or alleviation of a symptom of a disorder for the treatment of which the ADC is effective.

The term "treatment" refers to both therapeutic treatment and prophylactic or preventative measures. Those patients in need of treatment include those already with the disorder as well as those in which the disorder is to be prevented.

Various aspects of the invention are described in further detail in the following subsections.

### II. Anti-EGFR Antibody Drug Conjugate Formulations of the Invention

The present invention features formulations comprising an anti-EGFR antibody, or an antigen-binding portion thereof, wherein said EGFR antibody is conjugated to one or more drugs, *e.g.,* an auristatin (*e.g.,* MMAF). The present invention is based, at least in part, on the finding that a formulation comprising an anti-EGFR antibody drug conjugate (ADC), (wherein the anti-EGFR antibody, or antigen-binding portion thereof, is conjugated to one or more molecules of auristatin, such as MMAF), is able to maintain biological activity of the ADC upon reconstitution and maintain a drug to antibody ratio after reconstitution, following storage of the formulation for up to 6 months at 5°C and 25°C.

Formulations described herein may be lyophilized or aqueous. Unless otherwise indicated, the term "formulation" indicates both lyophilized and aqueous.

Certain formulations preferably comprise an anti-Epidermal Growth Factor Receptor (EGFR) antibody drug conjugate (ADC), a sugar, a buffer, and a polysorbate, wherein the formulation has a pH of about 5 - 7. In certain embodiments, the formulation further comprises an anti-EGFR ADC comprising an anti-EGFR antibody, or antigen-binding portion thereof, such as antibody 1, which is conjugated to one or more molecules of an auristatin, such as MMAF.

In one embodiment, the invention provides a lyophilized formulation comprising an anti-EGFR antibody drug conjugate, a sugar, *e.g.,* sucrose, a surfactant, *e.g.,* a polysorbate (such as polysorbate 80), and histidine, wherein the formulation has a pH of about 5 - 7, and wherein the anti-EGFR antibody drug conjugate comprises an anti-EGFR antibody, or antigen-binding portion thereof, conjugated to Monomethylauristatin F (MMAF).

In one embodiment, the invention provides a lyophilized formulation comprising an anti-EGFR antibody drug conjugate, sucrose, a surfactant, and a buffer, wherein the formulation has a pH of about 5 -7, and wherein the anti-EGFR antibody drug conjugate comprises an anti-EGFR antibody, or antigen-binding portion thereof, *e.g*., antibody 1, conjugated via a maleimidocaproyl linker to Monomethylauristatin F (MMAF) (mc-MMAF).

In one embodiment, the invention provides a lyophilized formulation comprising an anti-EGFR antibody drug conjugate, sucrose, a polysorbate, and histidine, wherein the formulation has a pH of about 5 - 7, and wherein the anti-EGFR antibody drug conjugate comprises an anti-EGFR antibody, or antigen-binding portion thereof, *e.g*., antibody 1, conjugated to via a maleimidocaproyl linker Monomethylauristatin F (MMAF) (mc-MMAF).

The formulations described herein preferably have a pH of about 4.0 to about 8.0. In one embodiment, the pH of the formulation ranges from about 5.0 to about 7.0; alternatively the pH may range from about 5 to about 6.5; alternatively the pH of the formulation may range from about 5.5 to about 6.5. The ranges intermediate to the aforementioned pH values, *e.g.,* about 5.6 to about 6.4, are also intended to be part of the invention. Ranges of values using a combination of any of the aforementioned values as upper / lower limits are also intended to be included, *e.g.,* a pH range of about 5.5 to about 6.2.

Formulations of the invention preferably comprise an anti-EGFR ADC, a sugar, a surfactant, and a buffer. Examples of buffers known in the art that may be used in the formulations of the invention include, but are not limited to, acetate, histidine, glycine, arginine, phosphate, Tris, and citrate. In one embodiment, the buffer used in the formulation is histidine.

In one embodiment, the formulation comprises 1-20 mg/ml of a histidine buffer (*e.g.,* about 1 to about 20, about 1 to about 10; about 1 to about 5; or about 1 to about 3 mg/ml) with a pH of about 5.0 to about 7.0; a pH of about 5 to about 6.5; or a pH of about 5.5 to about 6.5, and ranges in between.

In one embodiment, the formulation comprises about 5-25 mM histidine (*e.g*., about 2 to about 25 mM; about 5 to about 25 mM; about 10 to about 25 mM; or about 20 to about 25 mM), and ranges in between.

In one embodiment, the formulation comprises 1-20 mg/ml of succinate buffer (*e.g.,* about 1 to about 20, about 1 to about 10; about 5 to about 20; or about 5 to about 10 mg/ml) with a pH of about 5.0 to about 7.0; a pH of about 5 to about 6.5; or a pH of about 5.5 to about 6.5, and ranges in between. In one embodiment, the formulation comprises about 1-10 mg/ml of a succinate buffer with a pH of about 5.0 to about 7.0; a pH of about 5 to about 6.5; or a pH of about 5.5 to about 6.5, and ranges in between.

In one embodiment, the formulation comprises 1-20 mg/ml of a citrate buffer (*e.g.* about 1 to about 20, about 1 to about 10; about 5 to about 20; or about 5 to about 10 mg/ml) with a pH of about 5.0 to about 7.0; a pH of about 5 to about 6.5; or a pH of about 5.5 to about 6.5, and ranges in between. In one embodiment, the buffer used in the formulation comprises about 1-10 mg/ml of a citrate buffer with a pH of about 5.0 to about 7.0; a pH of about 5 to about 6.5; or a pH of about 5.5 to about 6.5, and ranges in between.

In one embodiment, the formulation comprises 1-20 mg/ml of a phosphate buffer (*e.g.* about 1 to about 20, about 1 to about 10; about 5 to about 20; or about 5 to about 10 mg/ml) with a pH of about 5.0 to about 7.0; a pH of about 5 to about 6.5; or a pH of about 5.5 to about 6.5, and ranges in between. In one embodiment, the buffer used in the formulation comprises about 1-10 mg/ml of a phosphate buffer with a pH of about 5.0 to about 7.0; a pH of about 5 to about 6.5; or a pH of about 5.5 to about 6.5, and ranges in between.

In one embodiment, the formulation comprises 1-20 mg/ml of a citrate / phosphate buffer (*e.g.* about 1 to about 20, about 1 to about 10; about 5 to about 20; or about 5 to about 10 mg/ml) with a pH of about 5.0 to about 7.0; a pH of about 5 to about 6.5; or a pH of about 5.5 to about 6.5. In one embodiment, the buffer used in the formulation comprises about 1-10 mg/ml of a citrate / phosphate buffer with a pH of about 5.0 to about 7.0; a pH of about 5 to about 6.5; or a pH of about 5.5 to about 6.5, and ranges in between.

In addition to the buffer, a sugar is included the formulation. Examples of sugars that may be used in the formulation include, but are not limited to mannitol, sorbitol, sucrose and trehalose. In one embodiment, the sugar used in the formulation is sucrose.

In one embodiment, the formulation comprises a sugar at a concentration of about 50 to about 90 mg/ml, about 50 to about 85 mg/ml, about 50 to about 80 mg/ml, about 50 to about 75 mg/ml, about 50 to about 70 mg/ml, about 50 to about 65 mg/ml, about 50 to about 60 mg/ml, about 60 to about 90 mg/ml, about 60 to about 85 mg/ml, about 60 to about 75 mg/ml, about 60 to about 70 mg/ml, or about 60 to about 75 mg/ml, and ranges in between, *e.g.,* about 55 to about 85 mg/ml of sugar, and ranges in between.

In one embodiment, the formulation comprises sucrose at a concentration of about 50 to about 90 mg/ml, about 50 to about 85 mg/ml, about 50 to about 80 mg/ml, about 50 to about 75 mg/ml, about 50 to about 70 mg/ml, about 50 to about 65 mg/ml, about 50 to about 60 mg/ml, about 60 to about 90 mg/ml, about 60 to about 85 mg/ml, about 60 to about 75 mg/ml, about 60 to about 70 mg/ml, or about 60 to about 75 mg/ml, and ranges in between, *e.g.,* about 55 to about 85 mg/ml of sucrose, and ranges in between.

A surfactant is also be added to the formulation , *e.g.,* for added stability. Exemplary surfactants include, but are not limited to, nonionic detergents such as polysorbates (*e.g*., polysorbates 20, 80) or poloxamers (*e.g*., poloxamer 188).

In one embodiment, the formulation comprises a polysorbate, *e.g*., polysorbate 80, at a concentration of about 0.05-0.20 mg/ml (*e.g*. about 0.05-0.19, 0.05-0.18, 0.05-0.17, 0.05-0.16, 0.05-0.15, 0.05-0.14, 0.05-0.13, 0.05-0.12, 0.05-0.11, or 0.05-0.10 mg/ml, and ranges in between). In one embodiment, the aqueous formulation comprises 0.05-0.15 mg/ml of the polysorbate, *e.g*., polysorbate 80.

In one embodiment, the formulation comprises an anti-EGFR antibody drug conjugate at a concentration of about 1-150 mg/ml (*e.g*. about 1, 5, 10, 15, 20, 25, 30, 35, 40, 45, 50, 55, 60, 65, 70, 75, 80, 85, 90, 95, 100, 105, 110, 120, 125, 130, 135, 140, 145, or 150 mg/ml, and values in between, of the anti-EGFR antibody drug conjugate).

In one embodiment, the formulation comprises an anti-EGFR antibody drug conjugate in an amount of about 1-150 mg, about 10-150 mg, about 20-150 mg, about 30-150 mg, about 40-150 mg, about 50-150 mg, about 60-150 mg, about 70-150 mg, about 80-150 mg, about 90-150 mg, about 100-150 mg, about 10-140 mg, about 20-140 mg, about 30-140 mg, about 40-140 mg, about 50-140 mg, about 60-140 mg. about 70-140 mg, about 80-140 mg, about 90-140 mg, about 100-140 mg, about 10-130 mg, about 20-130 mg, about 30-130 mg, about 40-130 mg, about 50-130 mg, about 60-130 mg about 70-130 mg, about 80-130 mg, about 90-130 mg, about 100-130 mg, about 10-120 mg, about 20-120 mg, about 30-120 mg, about 40-120 mg, about 50-120 mg, about 60-120 mg, about 70-120 mg, about 80-120 mg, about 90-120 mg, about 100-120 mg, about 10-110 mg, about 20-110 mg, about 30-110 mg, about 40-110 mg, about 50-110 mg, about 60-110 mg, about 70-110 mg, about 80-110 mg, about 90-110 mg, and about 100-110 mg, and ranges in between.

In one embodiment, the formulation comprises an anti-EGFR antibody drug conjugate at a concentration of 1 mg/ml, 2 mg/ml, 3 mg/ml, 4 mg/ml, 5 mg/ml, 6 mg/ml, 7 mg/ml, 8 mg/ml, 9 mg/ml, 10 mg/ml, 11 mg/ml, 12 mg/ml, 13 mg/ml, 14 mg/ml, 15 mg/ml, 16 mg/ml, 17 mg/ml, 18 mg/ml, 19 mg/ml, 20 mg/ml, 21 mg/ml, 22 mg/ml, 23 mg/ml, 24 mg/ml, 25 mg/ml, 26 mg/ml, 27 mg/ml, 28 mg/ml, 29 mg/ml, 30 mg/ml, 31 mg/ml, 32 mg/ml, 33 mg/ml, 34 mg/ml, 35 mg/ml, 36 mg/ml, 37 mg/ml, 38 mg/ml, 39 mg/ml, 40 mg/ml, 41 mg/ml, 42 mg/ml, 43 mg/ml, 44 mg/ml, 45 mg/ml, 46 mg/ml, 47 mg/ml, 48 mg/ml, 49 mg/ml, 50 mg/ml, 51 mg/ml, 52 mg/ml, 53 mg/ml, 54 mg/ml, 55 mg/ml, 56 mg/ml, 57 mg/ml, 58 mg/ml, 59 mg/ml, 60 mg/ml, 61 mg/ml, 62 mg/ml, 63 mg/ml, 64 mg/ml, 65 mg/ml, 66 mg/ml, 67 mg/ml, 68 mg/ml, 69 mg/ml, 70 mg/ml, 71 mg/ml, 72 mg/ml, 73 mg/ml, 74 mg/ml, 75 mg/ml, 76 mg/ml, 77 mg/ml, 78 mg/ml, 79 mg/ml, 80 mg/ml, 81 mg/ml, 82 mg/ml, 83 mg/ml, 84 mg/ml, 85 mg/ml, 86 mg/ml, 87 mg/ml, 88 mg/ml, 89 mg/ml, 90 mg/ml, 91 mg/ml, 92 mg/ml, 93 mg/ml, 94 mg/ml, 95 mg/ml, 96 mg/ml, 97 mg/ml, 98 mg/ml, 99 mg/ml, 100 mg/ml, 101 mg/ml, 102 mg/ml, 103 mg/ml, 104 mg/ml, 105 mg/ml, 106 mg/ml, 107 mg/ml, 108 mg/ml, 109 mg/ml, 110 mg/ml, 111 mg/ml, 112 mg/ml, 113 mg/ml, 114 mg/ml, 115 mg/ml, 116 mg/ml, 117 mg/ml, 118 mg/ml, 119 mg/ml, 120 mg/ml, 121 mg/ml, 122 mg/ml, 123 mg/ml, 124 mg/ml, 125 mg/ml, 126 mg/ml, 127 mg/ml, 128 mg/ml, 129 mg/ml, 130 mg/ml, 131 mg/ml, 132 mg/ml, 133 mg/ml, 134 mg/ml, 135 mg/ml, 136 mg/ml, 137 mg/ml, 138 mg/ml, 139 mg/ml, 140 mg/ml, 141 mg/ml, 142 mg/ml, 143 mg/ml, 144 mg/ml, 145 mg/ml, 146 mg/ml, 147 mg/ml, 148 mg/ml, 149 mg/ml or 150 mg/ml of the antibody. Ranges including any of the aforementioned numbers are also included in the invention, *e.g*., 10-150 mg/ml, 10-100 mg/ml, 20-90 mg/ml, 10-70 mg/ml, 10-40 mg.ml, and 1-70 mg/ml.

In one aspect, the invention features an aqueous formulation which is reconstituted from a lyophilized formulation comprising an anti-EGFR antibody drug conjugate, wherein the reconstituted aqueous formulation comprises about 1 - 100 mg/ml of the anti-EGFR ADC, about 1 -10 mg/mL of a buffer (*e.g.,* histidine), about 50 -90 mg/ml of a sugar (*e.g.,* sucrose), and about 0.01 - 0.2 mg/ml of a polysorbate (*e.g*., polysorbate 80), and has a pH of about 5 - 7, and wherein said anti-EGFR antibody drug conjugate comprises an anti-EGFR antibody, or antigen-binding portion thereof, (*e.g*., antibody 1) conjugated to an auristatin (*e.g.,* MMAF).

A lyophilized formulation is initially made as a pre-lyophilized formulation, which is the formulation prior to the lyophilization process. The amount of anti-EGFR antibody drug conjugate present in the pre-lyophilized formulation is determined taking into account the desired dose volumes, mode(s) of administration etc.

In one aspect, the invention features a lyophilized formulation comprising an anti-EGFR antibody drug conjugate, a sugar, (*e.g.,* sucrose), a surfactant, (*e.g.,* polysorbate 80), and histidine, wherein the formulation has a pH of about 5.0 to 7.0, and wherein the anti-EGFR antibody drug conjugate comprises an anti-EGFR antibody, (*e.g*., antibody 1), conjugated to an auristatin, (*e.g*., MMAF).

In one embodiment of the invention, the lyophilized formulation comprises an anti-EGFR ADC, *e.g.,* ADC1-MMAF, sucrose, a polysorbate, *e.g.,* polysorbate 80, and histidine, and does not include, or is substantially free of, mannitol and/or serine. In one embodiment of the invention, the lyophilized formulation comprises an anti-EGFR ADC, *e.g.,* ADC1-MMAF, sucrose, a polysorbate, *e.g*., polysorbate 80, and histidine, and does not include or is substantially free of a divalent cation. In another embodiment of the invention, the lyophilized formulation does not include lactobionic acid. In another embodiment of the invention, the lyophilized formulation does not include a sugar acid.

In certain exemplary aspects, the invention features a lyophilized formulation having a pH ranging from about 5.0 to 7.0 and comprising 1-20 mg of a buffer (*e.g*., histidine), about 320-410 mg of a sugar (*e.g.,* sucrose), about 0.1 to 0.9 mg of a surfactant (*e.g.,* polysorbate 80), and about 50-150 mg of an anti-EGFR antibody drug conjugate, wherein the anti-EGFR antibody drug conjugate comprises an anti-EGFR antibody, (*e.g*., antibody 1), conjugated to an auristatin, (*e.g*., MMAF).

In one embodiment, the lyophilized formulation comprises about 320-410 mg, about 330-400 mg, about 340 to 390 mg, about 350 to 380 mg, or about 360 to 370 mg of a sugar (*e.g.,* sucrose), and ranges (*e.g.,* 355 mg to 375 mg) in between.

In one embodiment, the lyophilized formulation comprises about 0.1-0.9 mg of a surfactant (*e.g*. about 0.1-0.8, 0.1-0.7, 0.01-0.6, 0.01-0.55, 0.2-0.6, 0.3-0.6, 0.4-0.6, and ranges in between).

In one embodiment, the lyophilized formulation comprises an anti-EGFR antibody drug conjugate in an amount of about 1-150 mg, about 10-150 mg, about 20-150 mg, about 30-150 mg, about 40-150 mg, about 50-150 mg, about 60-150 mg, about 70-150 mg, about 80-150 mg, about 90-150 mg, about 100-150 mg, about 10-140 mg, about 20-140 mg, about 30-140 mg, about 40-140 mg, about 50-140 mg, about 60-140 mg, about 70-140 mg, about 80-140 mg, about 90-140 mg, about 100-140 mg, about 10-130 mg, about 20-130 mg, about 30-130 mg, about 40-130 mg, about 50-130 mg, about 60-130 mg about 70-130 mg, about 80-130 mg, about 90-130 mg, about 100-130 mg, about 10-120 mg, about 20-120 mg, about 30-120 mg, about 40-120 mg, about 50-120 mg, about 60-120 mg, about 70-120 mg, about 80-120 mg, about 90-120 mg, about 100-120 mg, about 10-110 mg, about 20-110 mg, about 30-110 mg, about 40-110 mg, about 50-110 mg, about 60-110 mg, about 70-110 mg, about 80-110 mg, about 90-110 mg, and about 100-110 mg, and ranges in between.

In one embodiment, the lyophilized formulation comprises 1-20 mg/ml of histidine, or alternatively, 2-18 mg, 3-17 mg, 4-16 mg, 5-15 mg, 6-14 mg, or 7-13 mg, and ranges in between.

Lyophilization may be performed according to methods known in the art. For example, many different freeze-dryers are available for this purpose such as HULL50 (Hull, USA) or GT20 (Leybold-Heraeus, Germany) freeze-dryers. Freeze-drying is accomplished by freezing the formulation and subsequently subliming ice from the frozen content at a temperature suitable for primary drying. Under this condition, the product temperature is below the eutectic point or the collapse temperature of the formulation. Typically, the shelf temperature for the primary drying will range from about -30 to 25°C. (provided the product remains frozen during primary drying) at a suitable pressure, ranging typically from about 50 to 250 mTorr. The formulation, size and type of the container holding the sample (*e.g*., glass vial) and the volume of liquid will mainly dictate the time required for drying, which can range from a few hours to several days (*e.g.* 40-60 hrs). Optionally, a secondary drying stage may also be performed depending upon the desired residual moisture level in the product. The temperature at which the secondary drying is carried out ranges from about 0-40°C, depending primarily on the type and size of container and the type of protein employed. For example, the shelf temperature throughout the entire water removal phase of lyophilization may be from about 15-30°C (*e.g.,* about 20°C). The time and pressure required for secondary drying will be that which produces a suitable lyophilized cake, dependent, *e.g.,* on the temperature and other parameters. The secondary drying time is dictated by the desired residual moisture level in the product and typically takes at least about 5 hours (*e.g.* 10-15 hours). The pressure may be the same as that employed during the primary drying step. Freeze-drying conditions can be varied depending on the formulation and vial size.

Prior to administration to the patient, the lyophilized formulation is reconstituted with a pharmaceutically acceptable diluent such that the concentration of the anti-EGFR antibody drug conjugate in the reconstituted formulation is, for example, with that necessary for treatment, *e.g*., at least about 1-150 mg/ml. Reconstitution generally takes place at a temperature of about 25° C to ensure complete hydration, although other temperatures may be employed as desired. The time required for reconstitution will depend, *e.g.,* on the type of diluent, amount of excipient(s) and protein. Exemplary diluents include sterile water, bacteriostatic water for injection (BWFI), a pH buffered solution (*e.g*. phosphate-buffered saline), sterile saline solution (*e.g*., 0.9% saline solution), Ringer's solution or dextrose solution. In one embodiment, the lyophilisates are to be dissolved in sterile water for injection and diluted in sterile Normal Saline solution. The diluent optionally contains a preservative. Exemplary preservatives have been described above, with aromatic alcohols such as benzyl or phenol alcohol being the preferred preservatives. The amount of preservative employed is determined by assessing different preservative concentrations for compatibility with the protein and preservative efficacy testing. For example, if the preservative is an aromatic alcohol (such as benzyl alcohol), it can be present in an amount from about 0.1-2.0%, from about 0.5-1.5%, or about 1.0-1.2%.

### III. Anti-EGFR Antibody Drug Conjugates for Use in the Formulations of the Invention

The formulations and methods described herein encompass the use of antibody drug conjugates (ADCs) comprising anti-EGFR antibodies, or antigen-binding portions thereof, that specifically bind to EGFR. The anti-EGFR antibody drug conjugates used in the formulations of the invention are conjugated to a therapeutic agent, whereby the therapeutic agent exerts a cytotoxic, cytostatic or immunosuppressive effect on EGFR-expressing cells.

In particular, the present invention pertains to formulations comprising an anti-EGFR antibody drug conjugate comprising an antibody, or an antigen-binding portion thereof, that recognizes an EGFR epitope which is found in tumorigenic, hyperproliferative or abnormal cells, wherein the epitope is not detectable in normal or wild-type cells. Preferably, the antibody or antigen-binding portion thereof, does not bind to or recognize normal or wild-type cells containing normal or wild-type EGFR epitope in the absence of overexpression and in the presence of normal EGFR post-translational modification.

Anti-EGFR antibodies suitable for use in accordance with the present formulations and methods are typically monoclonal and can include, for example, chimeric (*e.g*., having a human constant region and mouse variable region), humanized, or human antibodies; single chain antibodies; or the like. The immunoglobulin molecules can be of any type (*e.g.,* IgG, IgE, IgM, IgD, IgA and IgY), class (*e.g.,* IgG1, IgG2, IgG3, IgG4, IgA1 and IgA2) or subclass of immunoglobulin molecule. For example, the anti-EGFR antibody used in the anti-EGFR antibody drug conjugate of the invention may be anti-EGFR antibody 1 or 2, or an antigen-binding portion thereof. The sequences and characteristics of antibodies 1 and 2 are described below (see also WO 2011/041319 and US20110076232 (see, *e.g*., antibody sequence of Figure 55), incorporated by reference in their entirety herein). Antibodies 1 and 2 each target the over-expressed form of the epidermal growth factor receptor (EGFR) present in 50% of all cancers of epithelial origin.

In a particular embodiment of the present invention, the anti-EGFR antibody used in the anti-EGFR antibody drug conjugate recognizes amplified wild-type EGFR and the de2-7 EGFR. The anti-EGFR antibody (or anti-EGFR ADC) demonstrates useful specificity, in that it recognizes de2-7 EGFR and amplified EGFR, but does not recognize normal, wild-type EGFR or the unique junctional peptide which is characteristic of de2-7 EGFR. Examples of antibodies having these binding characteristics are antibody 1, antibody 2 and antibody 3. Sequences for antibody 1 and antibody 2 are provided below.

Antibody 2 is a monoclonal murine anti-EGFR antibody. The antibody 2 VH chain comprises nucleic acid sequence (SEQ ID NO: 1) and amino acid sequence, with signal peptide (SEQ ID NO: 2) as shown below (signal peptide underlined in SEQ ID NO: 2).
SEQ ID NO: 1 
ATGAGAGTGCTGATTCTTTTGTGGCTGTTCACAGCCTTTCCTGGTGTCCTGTCTGA
TGTGCAGCTTCAGGAGTCGGGACCTAGCCTGGTGAAACCTTCTCAGTCTCTGTCC
CTCACCTGCACTGTCACTGGCTACTCAATCACCAGTGATTTTGCCTGGAACTGGA
TCCGGCAGTTTCCAGGAAACAAGCTGGAGTGGATGGGCTACATAAGTTATAGTG
GTAACACTAGGTACAACCCATCTCTCAAAAGTCGAATCTCTATCACTCGAGACAC
ATCCAAGAACCAATTCTTCCTGCAGTTGAATTCTGTGACTATTGAGGACACAGCC
ACATATTACTGTGTAACGGCGGGACGCGGGTTTCCTTATTGGGGCCAAGGGACTC
TGGTCACTGTCTCTGCA
SEQ ID NO: 2

MRVLILLWLFTAFPGVLSDVQLQESGPSLVKPSQSLSLTCTVTGYSITSDFAWNWIRQ
FPGNKLEWMGYISYSGNTRYNPSLKSRISITRDTSKNQFFLQLNSVTIEDTATYYCVT
AGRGFPYWGQGTLVTVSA

The antibody 2 VL chain comprises nucleic acid sequence (SEQ ID NO: 3) and amino acid sequence (SEQ ID NO :4) as shown below (signal peptide underlined in SEQ ID NO: 4).
SEQ ID NO: 3 
ATGGTGTCCACAGCTCAGTTCCTTGCATTCTTGTTGCTTTGGTTTCCAGGTGCAAG
ATGTGACATCCTGATGACCCAATCTCCATCCTCCATGTCTGTATCTCTGGGAGAC
ACAGTCAGCATCACTTGCCATTCAAGTCAGGACATTAACAGTAATATAGGGTGGT
TGCAGCAGAGACCAGGGAAATCATTTAAGGGCCTGATCTATCATGGAACCAACT
TGGACGATGAAGTTCCATCAAGGTTCAGTGGCAGTGGATCTGGAGCCGATTATTC
TCTCACCATCAGCAGCCTGGAATCTGAAGATTTTGCAGACTATTACTGTGTACAG
TATGCTCAGTTTCCGTGGACGTTCGGTGGAGGCACCAAGCTGGAAATCAAACGT
SEQ ID NO: 4

MVSTAQFLAFLLLWFPGARCDILMTQSPSSMSVSLGDTVSITCHSSQDINSNIGWLQQ
RPGKSFKGLIYHGTNLDDEVPSRFSGSGSGADYSLTISSLESEDFADYYCVQYAQFPW
TFGGGTKLEIKR

Complementarity determining regions CDR1, CDR2, and CDR3 (SEQ ID NOs: 5, 6, and 7, respectively) of the VH chain sequence SEQ ID NO: 2, without signal peptide (SEQ ID NO: 11) are indicated by underlining below. Key residues of the VH chain sequence (SEQ ID NO: 11) are 24, 37, 48, 67 and 78.
SEQ ID NO: 11

DVQLQESGPSLVKPSQSLSLTCTVTGYSITSDFAWNWIRQFPGNKLEWMGYISYSGNTR
CDR1 CDR2
YNPSLKSRISITRDTSKNQFFLQLNSVTIEDTATYYCVTAGRGFPYWGQGTLVTVSA
CDR3

Complementarity determining regions CDR1, CDR2, and CDR3 (SEQ ID NOS: 8, 9, and 10, respectively) of the VL chain sequence SEQ ID NO :4, without signal peptide (SEQ ID NO: 12) are indicated by underlining below. Key residues of the VH chain sequence (SEQ ID NO: 12) are 36, 46, 57 and 71.
SEQ ID NO: 12

DILMTQSPSSMSVSLGDTVSITCHSSQDINSNIGWLQQRPGKSFKGLIYHGTNLDDEVPSR
CDR1 CDR2
FSGSGSGADYSLTISSLESEDFADYYCVQYAQFPWTFGGGTKLEIKR

CDR3

Antibody 3 is a chimeric antibody comprising the variable domains of the light and heavy chains of antibody 2, and human constant regions.

In one embodiment of the invention, the anti-EGFR antibody used in the formulations and methods of the invention is antibody 1. As described above, antibody 1 is a humanized anti-EGFR antibody which includes the CDRs of antibody 2. The heavy chain variable (VH) and constant (CH) regions of antibody 1 are shown below as SEQ ID NOS: 13 and 14, respectively. The VH region CDR1, CDR2, and CDR3 (SEQ ID NOS: 15, 16, and 17, respectively) are indicated by underlining.
SEQ ID NO: 13 
QVQLQESGPGLVKPSQTLSLTCTVSGYSISSDFAWNWIRQPPGKGLEWMGYISYSGNTR
CDR1 CDR2
YQPSLKSRITISRDTSKNQFFLKLNSVTAADTATYYCVTAGRGFPYWGQGTLVTVSS
CDR3
SEQ ID NO: 14

The light chain variable (VL) and constant (CL) regions of antibody 1 are shown below as SEQ ID NOS: 18 and 19, respectively. The VL region CDR1, CDR2, and CDR3 (SEQ ID NOS: 20, 21, and 22, respectively) are indicated by underlining.
SEQ ID NO: 18 
DIQMTQSPSSMSVSVGDRVTITCHSSQDINSNIGWLQQKPGKSFKGLIYHGTNLDDGVPS
CDR1 CDR2
RFSGSGSGTDYTLTISSLQPEDFATYYCVQYAQFPWTFGGGTKLEIKR
CDR3
SEQ ID NO: 19

TVAAPSVFIFPPSDEQLKSGTASVVCLLNNFYPREAKVQWKVDNALQSGNSQESVTE
QDSKDSTYSLSSTLTLSKADYEKHKVYACEVTHQGLSSPVTKSFNRGEC

In one embodiment, the invention features a lyophilized formulation comprising an anti-EGFR antibody drug conjugate, sucrose, polysorbate 80, and histidine, wherein the formulation has a pH of about 5 -7, wherein the anti-EGFR antibody drug conjugate comprises an anti-EGFR antibody, or antigen binding portion thereof, conjugated to monomethyl auristatin F (MMAF), and wherein said antibody comprises a heavy chain variable region comprising complementarity domain regions (CDRs) comprising the amino acid sequences set forth in SEQ ID NOS: 15, 16, and 17, and comprises a light chain variable region comprising CDRs comprising the amino acid sequences set forth in SEQ ID NOS: 20, 21, and 22.

In one embodiment, the invention provides a lyophilized formulation having a pH of about 5-7 and comprising an anti-EGFR ADC comprising an antibody, or antigen-binding portion thereof, comprising a heavy chain variable region having the amino acid sequence set forth in SEQ ID NO: 13 and a heavy chain constant region having the amino acid sequence as set forth in SEQ ID NO: 14, and a light chain variable region having the amino acid sequence set forth in SEQ ID NO: 18 and a light chain constant region having the amino acid sequence as set forth in SEQ ID NO: 19, histidine, a sugar, *e.g.,* sucrose, and a polysorbate, *e.g.,* polysorbate 80.

In one embodiment, the invention provides a formulation comprising an ADC comprising an anti-EGFR antibody (conjugated to an auristatin, *e.g*., MMAF) having a light chain variable region comprising CDRs as described in the amino acid sequence of SEQ ID NO: 18, a heavy chain variable region comprising CDRs as described in the amino acid sequence of SEQ ID NO: 13.

Anti-EGFR antibodies that may be used make anti-EGFR antibody drug conjugates can be generated by any suitable method known in the art. For example, monoclonal antibodies can be prepared using a wide variety of techniques including, *e.g.,* the use of hybridoma, recombinant, and phage display technologies, or a combination thereof. Hybridoma techniques are generally discussed in, for example, Harlow et al., Antibodies: A Laboratory Manual, (Cold Spring Harbor Laboratory Press, 2nd ed., 1988); and Hammerling, et al., In Monoclonal Antibodies and T-Cell Hybridomas, pp. 563-681 (Elsevier, N.Y., 1981). Examples of phage display methods that can be used to make the anti-CD70 antibodies include, *e.g*., those disclosed in Brinkman et al., 1995, J Immunol Methods 182:41-50; Ames et al., 1995, J Immunol Methods 184:177-186; Kettleborough et al., 1994, Eur J Immunol 24:952-958; Persic et al., 1997, Gene 187:9-18; Burton et al., 1994, Advances in Immunology 57:191-280; PCT Application No. PCT/GB91/01 134; PCT Publications WO 90/02809; WO 91/10737; WO 92/01047; WO 92/18619; WO 93/11236; WO 95/15982; WO 95/20401; and U.S. Pat. Nos. 5,698,426; 5,223,409; 5,403,484; 5,580,717; 5,427,908; 5,750,753; 5,821,047; 5,571,698; 5,427,908; 5,516,637; 5,780,225; 5,658,727; 5,733,743 and 5,969,108 (the disclosures of which are incorporated by reference herein).

Techniques for generating antibody fragments that recognize specific epitopes are also generally known in the art. For example, Fab and F(ab')₂ fragments can be produced by proteolytic cleavage of immunoglobulin molecules, using enzymes such as papain (to produce Fab fragments) or pepsin (to produce F(ab')₂ fragments). F(ab')₂ fragments contain the variable region, the light chain constant region and the CH1 domain of the heavy chain. Techniques to recombinantly produce Fab, Fab' and F(ab')₂ fragments can also be employed using, *e.g*., methods disclosed in PCT publication WO 92/22324; Mullinax et al., 1992, BioTechniques 12(6):864-869; and Sawai et al., 1995, AJRI 34:26-34; and Better et al., 1988, Science 240:1041-1043 (the disclosures of which are incorporated by reference herein).

Examples of techniques that can be used to produce single-chain Fvs and antibodies include those described in U.S. Pat. Nos. 4,946,778 and 5,258,498; Huston et al., 1991, Methods in Enzymology 203:46-88; Shu et al., 1993, Proc Natl Acad Sci USA 90:7995-7999; and Skerra et al., 1988, Science 240:1038-1040.

Antibodies may be produced by any of a number of techniques known in the art. For example, expression from host cells, wherein expression vector(s) encoding the heavy and light chains is (are) transfected into a host cell by standard techniques. The various forms of the term "transfection" are intended to encompass a wide variety of techniques commonly used for the introduction of exogenous DNA into a prokaryotic or eukaryotic host cell, *e.g.,* electroporation, calcium-phosphate precipitation, DEAE-dextran transfection and the like.

Mammalian host cells for expressing the recombinant antibodies of the invention include Chinese Hamster Ovary (CHO cells) (including dhfr-CHO cells, described in Urlaub and Chasin (1980) Proc. Natl. Acad. Sci. USA 77:4216-4220, used with a DHFR selectable marker, *e.g.,* as described in Kaufman and Sharp (1982) J. Mol. Biol. 159:601-621) and DG44 or DUXB11 cells (Urlaub et al. (1986) Som. Cell Molec. Genet. 12:555; Haynes et al. (1983) Nuc. Acid. Res. 11:687-706; Lau et al. (1984) Mol. Cell. Biol. 4:1469-1475), NS0 myeloma cells, monkey kidney line (*e.g.,* CVI and COS, such as a COS 7 cell), SP2 cells, human embryonic kidney (HEK) cells, such as a HEK-293 cell, Chinese hamster fibroblast (*e.g.,* R1610), human cervical carcinoma (*e.g.,* HELA), murine fibroblast (*e.g.,* BALBc/3T3), murine myeloma (P3x63-Ag3.653; NS0; SP2/O), hamster kidney line (*e.g.,* HAK), murine L cell (*e.g.,* L-929), human lymphocyte (*e.g.,* RAJI), human kidney (*e.g.,* 293 and 293T). Host cell lines are typically commercially available (*e.g*., from BD Biosciences, Lexington, Ky.; Promega, Madison, Wis.; Life Technologies, Gaithersburg, Md.) or from the American Type Culture Collection (ATCC, Manassas, Va.).

When recombinant expression vectors encoding the antibody are introduced into mammalian host cells, the antibodies are produced by culturing the host cells for a period of time sufficient to allow for expression of the antibodies in the host cells or secretion of the antibodies into the culture medium in which the host cells are grown. Antibodies can be recovered from the culture medium using standard protein purification methods.

In an exemplary system for recombinant expression of antibodies, a recombinant expression vector encoding both the antibody heavy chain and the antibody light chain is introduced into dhfr-CHO cells by calcium phosphate-mediated transfection. Within the recombinant expression vector, the antibody heavy and light chain cDNAs are each operatively linked to CMV enhancer/AdMLP promoter regulatory elements to drive high levels of transcription of the cDNAs. The recombinant expression vector also carries cDNA encoding DHFR, which allows for selection of CHO cells that have been transfected with the vector using methotrexate selection/amplification. The selected transformant host cells are cultured to allow for expression of the antibody heavy and light chains and intact antibody is recovered from the culture medium. Standard molecular biology techniques are used to prepare the recombinant expression vector, transfect the host cells, select for transformants, culture the host cells and recover the antibody from the culture medium. Still further, the invention provides a method of synthesizing an antibody by culturing a host cell of the invention in a suitable culture medium until the antibody is synthesized. The method can further comprise isolating the antibody from the culture medium.

The anti-EGFR antibody drug conjugate used in the invention comprises an anti-EGFR antibody, or antigen binding portion thereof, conjugated to a cytotoxic or immunosuppressive agent, such that the resulting ADC exerts a cytotoxic or cytostatic effect on an EGFR-expressing cancer cell. Thus, the anti-EGFR antibody drug conjugate exerts a cytotoxic or cytostatic effect on EGFR-expressing cancer cells. In one embodiment, the anti-EGFR ADC is internalized and accumulates within an EGFR-expressing cell, where the ADC exerts a therapeutic effect (*e.g.,* a cytotoxic, cytostatic, or immunosuppressive effect).

Examples of suitable moieties for conjugation to antibodies include chemotherapeutic agents, prodrug converting enzymes, radioactive isotopes or compounds, or toxins. In exemplary embodiments, the anti-EGFR antibody, or an antigen-binding portion thereof is conjugated to an auristatin, *e.g*., MMAF or MMAE. Any agent that exerts a therapeutic effect on cancer cells or activated immune cells can be used as the therapeutic agent for conjugation to an anti-EGFR antibody or derivative thereof. (See, *e.g.,* WO 2004/010957, "Drug Conjugates and Their Use for Treating Cancer, An Autoimmune. Disease or an Infectious Disease" (supra) and U.S. Provisional Application No. 60/400,403 (supra)). Typically, the therapeutic agent is a cytotoxic agent. In some embodiments, an anti-EGFR drug conjugate comprises more than one therapeutic agents per conjugate, for example from about 1 to about 20 therapeutic agents per conjugate.

In a preferred embodiment, the anti-EGFR antibody, or an antigen-binding portion thereof, is conjugated to an auristatin (one or more). Auristatins have been shown to interfere with microtubule dynamics, GTP hydrolysis, and/or nuclear and cellular division and have anticancer and/or antifungal activity.

An anti-EGFR antibody of the invention may be conjugated to at least one auristatin. Auristatins represent a group of dolastatin analogs that have generally been shown to possess anticancer activity by interfering with microtubule dynamics and GTP hydrolysis, thereby inhibiting cellular division. For example, Auristatin E (described in U.S. Patent No. 5,635,483, incorporated by reference herein) is a synthetic analogue of the marine natural product dolastatin 10, a compound that inhibits tubulin polymerization by binding to the same site on tubulin as the anticancer drug vincristine (G. R. Pettit, Prog. Chem. Org. Nat. Prod, 70: 1-79 (1997)). Dolastatin 10, auristatin PE, and auristatin E are linear peptides having four amino acids, three of which are unique to the dolastatin class of compounds. Exemplary embodiments of the auristatin subclass of mitotic inhibitors include, but are not limited to, monomethyl auristatin D (MMAD or auristatin D derivative), monomethyl auristatin E (MMAE or auristatin E derivative), monomethyl auristatin F (MMAF or auristatin F derivative), auristatin F phenylenediamine (AFP), auristatin EB (AEB), auristatin EFP (AEFP), and 5-benzoylvaleric acid-AE ester (AEVB). The synthesis and structure of auristatin derivatives are described in U.S. Patent Application Publication Nos. 2003-0083263, 2005-0238649 and 2005-0009751; International Patent Publication No. WO 04/010957, International Patent Publication No. WO 02/088172, and U.S. Pat. Nos. 6,323,315; 6,239,104; 6,034,065; 5,780,588; 5,665,860; 5,663,149; 5,635,483; 5,599,902; 5,554,725; 5,530,097; 5,521,284; 5,504,191; 5,410,024; 5,138,036; 5,076,973; 4,986,988; 4,978,744; 4,879,278; 4,816,444; and 4,486,414, each of which is incorporated by reference herein.

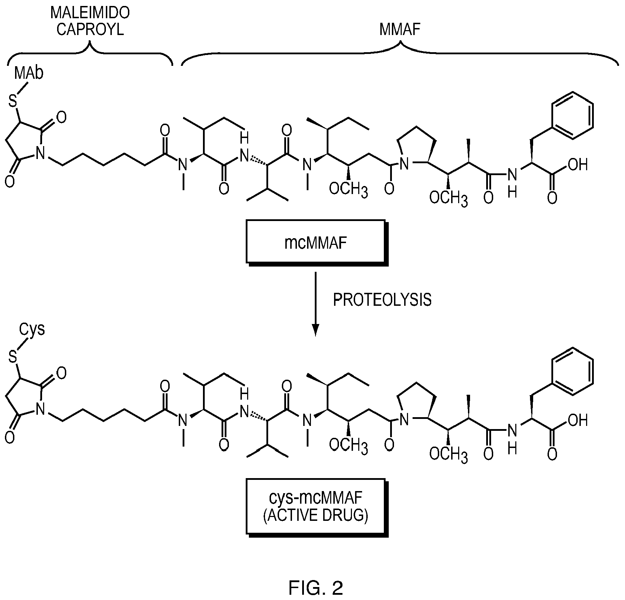

In one embodiment, an anti-EGFR antibody, such as antibody 1, is conjugated to at least one MMAF (monomethyl auristatin F) by a linker such as, but not limited to, maleimidocaproyl (mc-MMAF). The structure of MMAF is provided in Figure 2. An anti-EGFR-antibody ADC may have a drug-to-antibody ratio (DAR) of 2, 4, 6, or 8. Notably, the DAR of an ADC can range from 0 to 8, although higher loads, *e.g.,* 10, are also possible. Monomethyl auristatin F (MMAF) inhibits cell division by blocking the polymerization of tubulin. It has a charged C-terminal phenylalanine residue that attenuates its cytotoxic activity compared to its uncharged counterpart MMAE. Because of its toxicity, it cannot be used as a drug itself, but can be linked to a monoclonal antibody (mAb) that directs it to the cancer cells. In one embodiment, the linker to the anti-EGFR antibody is stable in extracellular fluid, but is cleaved by cathepsin once the conjugate has entered a tumor cell, thus activating the anti-mitotic mechanism.

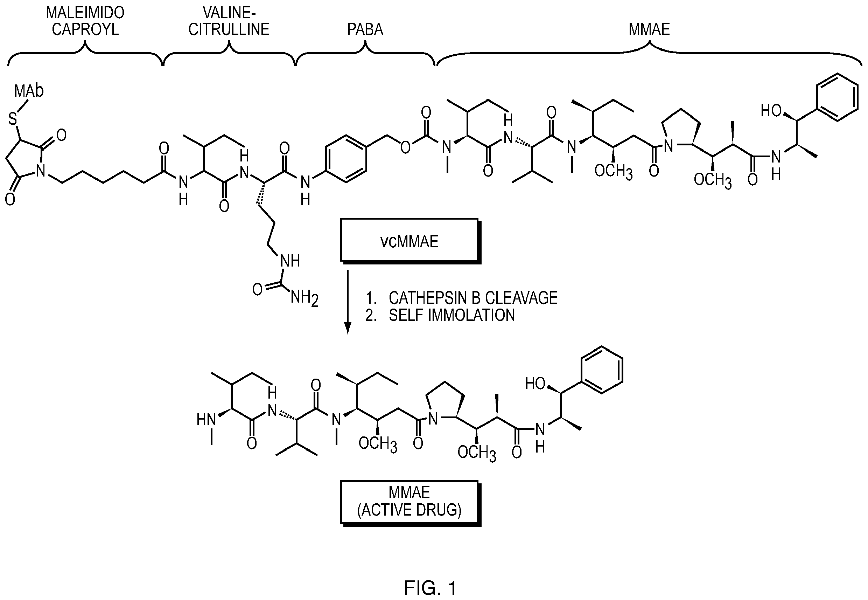

In one embodiment, the anti-EGFR antibody of the invention is conjugated to at least one MMAE (mono-methyl auristatin E). The structure of MMAE is provided in Figure 1, as is an exemplary ADC comprising MMAE. Monomethyl auristatin E (MMAE, vedotin) inhibits cell division by blocking the polymerisation of tubulin. Because of its super toxicity, it also cannot be used as a drug itself. In recent cancer therapy developments, it is linked to a monoclonal antibody (mAb) that recognizes a specific marker expression in cancer cells and directs MMAE to the cancer cells. In one embodiment, the linker linking MMAE to the anti-EGFR antibody is stable in extracellular fluid (i.e., the medium or environment that is external to cells), but is cleaved by cathepsin once the ADC has bound to the specific cancer cell antigen and entered the cancer cell, thus releasing the toxic MMAE and activating the potent anti-mitotic mechanism.

In one embodiment, the anti-EGFR antibody, or antigen-binding portion thereof, is conjugated to an auristatin which is MMAF. In one embodiment, the anti-EGFR ADC is the ADC 1-MMAF. ADC 1-MMAF comprises antibody 1 (described above and in SEQ ID NOs: 13 to 22) covalently linked to one or more molecules of monomethyl auristatin F (MMAF) (see Figure 2 for structure). To generate ADC 1-MMAF, the interchain disulfide bonds of antibody 1 are reduced to sulfhydryl groups. MMAF is then coupled to the antibody via these sulfhydryl groups. ADC 1-MMAF is generated using a noncleavable linker, i.e., a noncleavable maleimidocaproyl (mc) linkage, as shown in Figure 2.

In one particular embodiment, the formulations of the invention comprise anti-EGFR ADCs that are labeled with a detectable or functional label. Detectable labels include, but are not limited to, radiolabels such as the isotopes ²H, ³H, ¹¹C, ¹³C, ¹⁴C, ³²P, ³³S, ³⁴S, ³⁵S, ³⁶S, ³⁶Cl, ⁵¹Cr, ⁵⁷Co, ⁵⁸Co, ⁵⁹Fe, ⁹⁰Y, ¹²¹I, ¹²⁴I, ¹²⁵I, ¹³¹I, ²¹¹At, ¹⁹⁸Au, ⁶⁷Cu, ²²⁵Ac, ²¹³Bi, ⁹⁹Tc and ¹⁸⁶Re, which may be attached to antibodies of the invention using conventional chemistry known in the art of antibody imaging. Labels also include fluorescent labels and labels used conventionally in the art for MRI-CT imagine. They also include enzyme labels such as horseradish peroxidase. Labels further include chemical moieties such as biotin which may be detected via binding to a specific cognate detectable moiety, *e.g.* labeled avidin.

Functional labels may also include substances which are designed to be targeted to the site of a tumor to cause destruction of tumor tissue. Such functional labels include cytotoxic drugs such as 5-fluorouracil or ricin and enzymes such as bacterial carboxypeptidase or nitroreductase, which are capable of converting prodrugs into active drugs at the site of a tumor.

As will be understood by those of skill in the art, the agents set forth above, as well as other suitable agents, may be conjugated or attached to an anti-EGFR antibody, *e.g*., antibody 1, in any suitable manner to produce an anti-EGFR ADC useful in the present invention. For example and without limitation, in various embodiments of the present invention the anti-EGFR antibody and agent(s) may be covalently attached and/or may be conjugated using linker, spacer and/or stretcher compounds, which in various embodiments of the present invention are cleavable or are noncleavable, and result in the therapeutic agent(s) being internalized by the target cell.

In one embodiment, antibody 1 is conjugated to MMAF using a noncleavable maleimidocaproyl linkage (antibody 1-mc-MMAF).

Techniques for conjugating therapeutic agents to proteins, and in particular to antibodies, are well-known. (See, *e.g.,* Arnon et al., "Monoclonal Antibodies For Immunotargeting Of Drugs In Cancer Therapy," in Monoclonal Antibodies And Cancer Therapy (Reisfeld et al. eds., Alan R. Liss, Inc., 1985); Hellstrom et al., "Antibodies For Drug Delivery," in Controlled Drug Delivery (Robinson et al. eds., Marcel Dekker, Inc., 2nd ed. 1987); Thorpe, "Antibody Carriers Of Cytotoxic Agents In Cancer Therapy: A Review," in Monoclonal Antibodies '84: Biological And Clinical Applications (Pinchera et al. eds., 1985); "Analysis, Results, and Future Prospective of the Therapeutic Use of Radiolabeled Antibody In Cancer Therapy," in Monoclonal Antibodies For Cancer Detection And Therapy (Baldwin et al. eds., Academic Press, 1985); and Thorpe et al., 1982, Immunol. Rev. 62:119-58. See also, *e.g.,* PCT publication WO 89/12624.)

In one embodiment, the ADC comprises a linker region between the cytotoxic agent and the antibody. For example, such linker, spacer and/or stretcher compounds include, but are not limited to, the following: amino benzoic acid spacers (see, for example and without limitation, U.S. Patent Nos. 7,091,186 and 7,553,816, each of which is hereby incorporated by reference in its entirety); maleimidocaproyl; p-aminobenzylcarbamoyl (PAB); lysosomal enzyme-cleavable linkers (see, for example and without limitation, U.S. Patent No. 6,214,345, hereby incorporated by reference in its entirety); maleimidocaproyl-polyethylene 20 glycol (MC(PEG)6-OH); N-methyl-valine citrulline; N-succinimidyl 4-(N-maleimidomethyl) cyclohexane-1-carboxylate (SMCC) (see, for example and without limitation, Yoshitake et al. (1979) Eur. J. Biochem., 101, 395-399, hereby incorporated by reference in its entirety); N- succinimidyl 4-(2-pyridyldithio) butanoate (SPDB) (see, for example and without limitation, U.S. Patent No. 4,563,304, hereby incorporated by reference 25 in its entirety); N-Succinimidyl 4-(2-pyridylthio)pentanoate (SPP); valine-citrulline; and other linker, spacer, and/or stretcher compounds (see, for example and without limitation, U.S. Patent Nos. 7,090,843, 7,223,837, and 7,659,241, and U.S. Patent Publication Nos. 2004/0018194, 2004/0121940, 2006/0116422, 2007/0258987, 2008/0213289, 2008/0241128, 2008/0311136, 2008/0317747, and 2009/0010945, each of which is hereby incorporated by reference in its entirety). Generally speaking, techniques for attaching and/or conjugating the agents set forth above, as well as other agents, to specific binding members of the present invention, particularly antibodies and fragments thereof, are known in the art. See, for example and without limitation, Amon et al., "Monoclonal Antibodies For Immunotargeting Of Drugs In Cancer Therapy", in Monoclonal Antibodies And Cancer Therapy, Reisfeld et al. (eds.), pp. 243-56 (Alan R. Liss, Inc. 1985); Hellstrom et al., "Antibodies For Drug Delivery", in Controlled Drug Delivery (2nd Ed.), Robinson et al. (eds.), pp. 623-53 (Marcel Dekker, Inc. 1987); Thorpe, "Antibody Carriers Of Cytotoxic Agents In Cancer Therapy: A Review", In Monoclonal Antibodies '84: Biological And Clinical Applications, Pinchera et al. (eds.), pp. 475-506 (1985); "Analysis, Results, And Future Prospective Of The Therapeutic Use Of Radiolabeled Antibody In Cancer Therapy", in Monoclonal Antibodies For Cancer Detection And Therapy, Baldwin et al. (eds.), pp. 303-16 (Academic Press 1985), and Thorpe et al., "The Preparation And Cytotoxic Properties Of Antibody-Toxin Conjugates", Immunol. Rev., 62:119-58 (1982), each of which is hereby incorporated by reference in its entirety.

A number of different reactions are available for covalent attachment of drugs to antibodies. This is often accomplished by reaction of the amino acid residues of the antibody molecule, including the amine groups of lysine, the free carboxylic acid groups of glutamic and aspartic acid, the sulfhydryl groups of cysteine and the various moieties of the aromatic amino acids. One of the most commonly used non-specific methods of covalent attachment is the carbodiimide reaction to link a carboxy (or amino) group of a compound to amino (or carboxy) groups of the antibody. Additionally, bifunctional agents such as dialdehydes or imidoesters have been used to link the amino group of a compound to amino groups of the antibody molecule. Also available for attachment of drugs to antibodies is the Schiff base reaction. This method involves the periodate oxidation of a drug that contains glycol or hydroxy groups, thus forming an aldehyde which is then reacted with the antibody molecule. Attachment occurs via formation of a Schiff base with amino groups of the antibody molecule. Isothiocyanates can also be used as coupling agents for covalently attaching drugs to antibodies. Other techniques are known to the skilled artisan and within the scope of the present invention. Non-limiting examples of such techniques are described in, *e.g.,* U.S. Pat. Nos. 5,665,358; 5,643,573; and 5,556,623, which are incorporated by reference in their entireties herein.

In certain embodiments, an intermediate, which is the precursor of the linker, is reacted with the drug under appropriate conditions. In certain embodiments, reactive groups are used on the drug and/or the intermediate. The product of the reaction between the drug and the intermediate, or the derivatized drug, is subsequently reacted with the anti-EGFR antibody under appropriate conditions.

Other examples of conjugation methods are described in US Patent No. 7,837,980 (Seattle Genetics), Carter and Senter (2008) Cancer J, 14(3):154, as well as U.S. Published Application Nos. 2004-0157782 A1 and 2005-0238649 and International Patent Application No, PCT/US04/038392.

In certain embodiments, anti-EGFR ADCs may be purified in order to obtain ADCs having a desired drug to antibody ration (DAR). In one embodiment of the invention, the formulation contains an anti-EGFR ADC mixture comprising anti-EGFR ADCs having an average desired drug to antibody ration (DAR), *e.g.,* an average DAR of about 3. In one embodiment of the invention, the formulation comprises an ADC mixture comprising anti-EGFR ADCs having a desired DAR range, *e.g.,* a DAR of about 2-4.

In one embodiment, the formulation contains an ADC mixture where 70% of the ADCs present have a drug loaded species of 4 or less, and wherein the ADC comprises an anti-EGFR antibody and an auristatin. Alternatively, 75% of ADCs present have a drug loaded species of 4 or less; 80% of ADCs present have a drug loaded species of 4 or less; 85% of ADCs present have a drug loaded species of 4 or less; 90% of ADCs present have a drug loaded species of 4 or less; or 95% of ADCs present have a drug loaded species of 4 or less.

In one embodiment of the invention, the formulation comprises an anti-EGFR ADC comprising an anti-EGFR antibody, or antigen-binding portion thereof, conjugated to monomethyl auristatin F (MMAF), wherein said ADC 1-MMAF comprises a heavy chain variable region comprising complementarity domain regions (CDRs) comprising the amino acid sequences set forth in SEQ ID NOS: 15, 16, and 17, and comprises a light chain variable region comprising CDRs comprising the amino acid sequences set forth in SEQ ID NOS: 20, 21, and 22, sucrose, histidine, and polysorbate 80, wherein the formulation comprises an ADC mixture having an average DAR of about 3 or an ADC mixture having a DAR of about 2-4. In a further embodiment of the invention, the formulation is lyophilized. In yet a further embodiment, the anti-EGFR antibody is linked to MMAF via a maleimidocaproyl linker. In still a further embodiment, the anti-EGFR antibody, or antigen-binding portion thereof, comprises a heavy chain variable region comprising the amino acid sequence set forth in SEQ ID NO: 13, and a light chain variable region comprising the amino acid sequence set forth in SEQ ID NO: 18.

In one embodiment, the formulation comprises an anti-EGFR ADC comprising an anti-EGFR antibody, or antigen-binding portion thereof, conjugated to monomethyl auristatin E (MMAE), wherein said ADC 1-MMAE comprises a heavy chain variable region comprising complementarity domain regions (CDRs) comprising the amino acid sequences set forth in SEQ ID NOS: 15, 16, and 17, and comprises a light chain variable region comprising CDRs comprising the amino acid sequences set forth in SEQ ID NOS: 20, 21, and 22, sucrose, histidine, and polysorbate 80, wherein the formulation comprises an ADC mixture having an average DAR of about 3 or an ADC mixture having a DAR of about 2-4. In a further embodiment of the invention, the formulation is lyophilized. In still a further embodiment, the anti-EGFR antibody, or antigen-binding portion thereof, comprises a heavy chain variable region comprising the amino acid sequence set forth in SEQ ID NO: 13, and a light chain variable region comprising the amino acid sequence set forth in SEQ ID NO: 18.

Purification of the ADCs may be achieved in such a way that ADCs having certain DARs are collected. For example, HIC resin may be used to separate high drug loaded ADCs from ADCs having optimal drug to antibody ratios (DARs), *e.g.* a DAR of 4 or less. In one embodiment, a hydrophobic resin is added to an ADC mixture such that undesired ADCs, *i.e.,* higher drug loaded ADCs, bind the resin and can be selectively removed from the mixture. In certain embodiments, separation of the ADCs may be achieved by contacting an ADC mixture (*e.g*., a mixture comprising a drug loaded species of ADC of 4 or less and a drug loaded species of ADC of 6 or more) with a hydrophobic resin, wherein the amount of resin is sufficient to allow binding of the drug loaded species which is being removed from the ADC mixture. The resin and ADC mixture are mixed together, such that the ADC species being removed (*e.g.,* a drug loaded species of 6 or more) binds to the resin and can be separated from the other ADC species in the ADC mixture. The amount of resin used in the method is based on a weight ratio between the species to be removed and the resin, where the amount of resin used does not allow for significant binding of the drug loaded species that is desired. Thus, methods may be used to reduce the average DAR 5.5 to less than 4. Further, the purification methods may be used to isolate ADCs having any desired range of drug loaded species, *e.g*., a drug loaded species of 4 or less, a drug loaded species of 3 or less, a drug loaded species of 2 or less, a drug loaded species of 1 or less.

Certain species of molecule(s) binds to a surface based on hydrophobic interactions between the species and a hydrophobic resin. In one embodiment, method of the invention refers to a purification process that relies upon the intermixing of a hydrophobic resin and a mixture of ADCs, wherein the amount of resin added to the mixture determines which species (*e.g*., ADCs with a DAR of 6 or more) will bind. Following production and purification of an antibody from an expression system (*e.g*., a mammalian expression system), the antibody is reduced and coupled to a drug through a conjugation reaction. The resulting ADC mixture often contains ADCs having a range of DARs, *e.g*., 1 to 8. In one embodiment, the ADC mixture comprises a drug loaded species of 4 or less and a drug loaded species of 6 or more. According to the methods of the invention, the ADC mixture may be purified using a process, such as, but not limited to, a batch process, such that ADCs having a drug loaded species of 4 or less are selected and separated from ADCs having a higher drug load (*e.g*., ADCs having a drug loaded species of 6 or more). Notably, the purification methods described herein may be used to isolate ADCs having any desired range of DAR, *e.g.,* a DAR of 4 or less, a DAR of 3 or less, a DAR of 2 or less.

Thus, in one embodiment, an ADC mixture comprising a drug loaded species of 4 or less and a drug loaded species of 6 or more may be contacted with a hydrophobic resin to form a resin mixture, wherein the amount of hydrophobic resin contacted with the ADC mixture is sufficient to allow binding of the drug loaded species of 6 or more to the resin but does not allow significant binding of the drug load species of 4 or less; and removing the hydrophobic resin from the ADC mixture, such that the composition comprising ADCs is obtained, wherein the composition comprises less than 15% of the drug loaded species of 6 or more, and wherein the ADC comprises an antibody conjugated to an auristatin. In a separate embodiment, the method may include contacting an ADC mixture comprising a drug loaded species of 4 or less and a drug loaded species of 6 or more with a hydrophobic resin to form a resin mixture, wherein the amount of hydrophobic resin contacted with the ADC mixture is sufficient to allow binding of the drug loaded species of 6 or more to the resin but does not allow significant binding of the drug load species of 4 or less; and removing the hydrophobic resin from the ADC mixture, such that the composition comprising ADCs is obtained, wherein the composition comprises less than 15% of the drug loaded species of 6 or more, and wherein the ADC comprises an antibody conjugated to an auristatin, wherein the hydrophobic resin weight is 3 to 12 times the weight of the drug loaded species of 6 or more in the ADC mixture.

The ADC separation method may be performed using a batch purification method. The batch purification process generally includes adding the ADC mixture to the hydrophobic resin in a vessel, mixing, and subsequently separating the resin from the supernatant. For example, in the context of batch purification, a hydrophobic resin may be prepared in or equilibrated to the desired equilibration buffer. A slurry of the hydrophobic resin may thus be obtained. The ADC mixture may then be contacted with the slurry to adsorb the specific species of ADC(s) to be separated by the hydrophobic resin. The solution comprising the desired ADCs that do not bind to the hydrophobic resin material may then be separated from the slurry, e.g., by filtration or by allowing the slurry to settle and removing the supernatant. The resulting slurry can be subjected to one or more washing steps. In order to elute bound ADCs, the salt concentration can be decreased. In one embodiment, the process used in the invention includes no more than 50 g of hydrophobic resin.

Thus, a batch method may be used to contact an ADC mixture comprising a drug loaded species of 4 or less and a drug loaded species of 6 or more with a hydrophobic resin to form a resin mixture, wherein the amount of hydrophobic resin contacted with the ADC mixture is sufficient to allow binding of the drug loaded species of 6 or more to the resin but does not allow significant binding of the drug load species of 4 or less; and removing the hydrophobic resin from the ADC mixture, such that the composition comprising ADCs is obtained, wherein the composition comprises less than 15% of the drug loaded species of 6 or more, and wherein the ADC comprises an antibody conjugated to an auristatin. In a separate embodiment, a batch method is used to contact an ADC mixture comprising a drug loaded species of 4 or less and a drug loaded species of 6 or more with a hydrophobic resin to form a resin mixture, wherein the amount of hydrophobic resin contacted with the ADC mixture is sufficient to allow binding of the drug loaded species of 6 or more to the resin but does not allow significant binding of the drug load species of 4 or less; and removing the hydrophobic resin from the ADC mixture, such that the composition comprising ADCs is obtained, wherein the composition comprises less than 15% of the drug loaded species of 6 or more, and wherein the ADC comprises an antibody conjugated to an auristatin, wherein the hydrophobic resin weight is 3 to 12 times the weight of the drug loaded species of 6 or more in the ADC mixture.

Alternatively, purification may be performed using a circulation process, whereby the resin is packed in a container and the ADC mixture is passed over the hydrophobic resin bed until the specific species of ADC(s) to be separated have been removed. The supernatant (containing the desired ADC species) is then pumped from the container and the resin bed may be subjected to washing steps.

A circulation process may also be used to contact an ADC mixture comprising a drug loaded species of 4 or less and a drug loaded species of 6 or more with a hydrophobic resin to form a resin mixture, wherein the amount of hydrophobic resin contacted with the ADC mixture is sufficient to allow binding of the drug loaded species of 6 or more to the resin but does not allow significant binding of the drug load species of 4 or less; and removing the hydrophobic resin from the ADC mixture, such that the composition comprising ADCs is obtained, wherein the composition comprises less than 15% of the drug loaded species of 6 or more, and wherein the ADC comprises an antibody conjugated to an auristatin. In a separate embodiment, a circulation process is used to contact an ADC mixture comprising a drug loaded species of 4 or less and a drug loaded species of 6 or more with a hydrophobic resin to form a resin mixture, wherein the amount of hydrophobic resin contacted with the ADC mixture is sufficient to allow binding of the drug loaded species of 6 or more to the resin but does not allow significant binding of the drug load species of 4 or less; and removing the hydrophobic resin from the ADC mixture, such that the composition comprising ADCs is obtained, wherein the composition comprises less than 15% of the drug loaded species of 6 or more, and wherein the ADC comprises an antibody conjugated to an auristatin, wherein the hydrophobic resin weight is 3 to 12 times the weight of the drug loaded species of 6 or more in the ADC mixture.

Alternatively, purification may be performed using a flow through process, whereby resin is packed in a container, *e.g*., a column, and the ADC mixture is passed over the packed resin such that the desired ADC species does not substantially bind to the resin and flows through the resin, and the undesired ADC species is bound to the resin. A flow through process may be performed in a single pass mode (where the ADC species of interest are obtained as a result of a single pass through the resin of the container) or in a multi-pass mode (where the ADC species of interest are obtained as a result of multiple passes through the resin of the container). The flow through process is performed such that the weight of resin selected binds to the undesired ADC population, and the desired ADCs (*e.g*., DAR 2-4) flow over the resin and are collected in the flow through after one or multiple passes.

In one embodiment of the invention, a flow through process may be used to contact an ADC mixture comprising a drug loaded species of 4 or less and a drug loaded species of 6 or more with a hydrophobic resin, wherein the amount of hydrophobic resin contacted with the ADC mixture is sufficient to allow binding of the drug loaded species of 6 or more to the resin but does not allow significant binding of the drug load species of 4 or less, where the drug load species of 4 or less passes over the resin and is subsequently collected after one or multiple passes, such that the composition comprising the desired ADCs (e.g. DAR 2-4) is obtained, wherein the composition comprises less than 15% of the drug loaded species of 6 or more, and wherein the ADC comprises an antibody conjugated to an auristatin. In a separate embodiment, a flow through process is used to contact an ADC mixture comprising a drug loaded species of 4 or less and a drug loaded species of 6 or more with a hydrophobic resin by passing the ADC mixture over the resin, wherein the amount of hydrophobic resin contacted with the ADC mixture is sufficient to allow binding of the drug loaded species of 6 or more to the resin but does not allow significant binding of the drug load species of 4 or less, where the drug load species of 4 or less passes over the resin and is subsequently collected, such that the composition comprising ADCs is obtained, wherein the composition comprises less than 15% of the drug loaded species of 6 or more, and wherein the ADC comprises an antibody conjugated to an auristatin, wherein the amount of hydrophobic resin weight is 3 to 12 times the weight of the drug loaded species of 6 or more in the ADC mixture.

Purification methods may be based on the use of a hydrophobic resin to separate high vs. low drug loaded species of ADC. Hydrophobic resin comprises hydrophobic groups which interact with the hydrophobic properties of the ADCs. Hydrophobic groups on the ADC interact with hydrophobic groups within the hydrophobic resin. The more hydrophobic a protein is the stronger it will interact with the hydrophobic resin.

Hydrophobic resin normally comprises a base matrix (e.g., cross-linked agarose or synthetic copolymer material) to which hydrophobic ligands (e.g., alkyl or aryl groups) are coupled. Many hydrophobic resins are available commercially. Examples include, but are not limited to, Phenyl Sepharose™ 6 Fast Flow with low or high substitution (Pharmacia LKB Biotechnology, AB, Sweden); Phenyl Sepharose™ High Performance (Pharmacia LKB Biotechnology, AB, Sweden); Octyl Sepharose™ High Performance (Pharmacia LKB Biotechnology, AB, Sweden); Fractogel™ EMD Propyl or Fractogel™ EMD Phenyl columns (E. Merck, Germany); Macro-Prep™ Methyl or Macro-Prep™. t-Butyl Supports (Bio-Rad, California); WP HI-Propyl (C₃)™ (J. T. Baker, New Jersey); and Toyopearl™ ether, hexyl, phenyl or butyl (TosoHaas, PA). In one embodiment, the hydrophobic resin is a butyl hydrophobic resin. In another embodiment, the hydrophobic resin is a phenyl hydrophobic resin. In another embodiment, the hydrophobic resin is a hexyl hydrophobic resin, an octyl hydrophobic resin, or a decyl hydrophobic resin. In one embodiment, the hydrophobic resin is a methacrylic polymer having n-butyl ligands (*e.g*. TOYOPEARL® Butyl-600M).

Additional methods for purifying separate low and high DAR ADCs are disclosed in U.S. Provisional Application No. 61/792,834 and U.S. Application No. 14/210,602, filed on March 14, 2014 , the disclosures of which are incorporated by reference herein.

### IV. Formulation Uses

In accordance with the present methods, a formulation comprising an anti-EGFR ADC is administered to a subject having (or at risk of having) a disorder requiring treatment with an anti-EGFR antibody or an anti-EGFR-ADC. The formulation comprising the anti-EGFR ADC may be administered either alone or in combination with other compositions in the prevention or treatment of the disorder requiring treatment with the anti-EGFR antibody.

As used herein, the term "a disorder in which EGFR activity is detrimental" is intended to include diseases and other disorders in which the presence of EGFR in a subject suffering from the disorder has been shown to be or is suspected of being either responsible for the pathophysiology of the disorder or a factor that contributes to a worsening of the disorder. Accordingly, a disorder in which EGFR activity is detrimental is a disorder in which inhibition of EGFR activity is expected to alleviate the symptoms and/or progression of the disorder. Such disorders may be evidenced, for example, by an increase in the activity of EGFR or an increase in the amount of EGFR present in a biological sample from a subject suffering from the disorder (*e.g.,* an increase in the concentration of EGFR in a tissue sample, in serum, plasma, synovial fluid, etc. of the subject), which can be detected, for example, using an anti-EGFR antibody.

The formulations of the invention may be used to treat cancer. Examples of cancer that may be treated include, but are not limited to, glioblastoma, non-small cell lung cancer, lung cancer, colon cancer, head and neck cancer, breast cancer, squamous cell tumors, anal cancer, skin cancer, and vulvar cancer.

In one embodiment, a formulation comprising an ADC1-MMAF may be administered to a subject for the treatment of colorectal cancer, head and neck cancer (including, but not limited to, hypopharyngeal cancer, oropharyngeal cancer, esophageal cancer, laryngeal cancer, and oral cavity cancer), pancreatic cancer, and gastric cancer. In one aspect of the invention, a formulation comprising an ADC1-MMAF is administered to a subject to treat a solid tumor likely to over-express the Epidermal Growth Factor Receptor (EGFR), squamous non-small cell lung cancer (NSCLC), or glioblastoma multiforme. Additional examples of such cancers that may be treated with the compositions of the invention include squamous tumors (including, squamous tumors of the lung, head and neck, cervical, etc.), glioblastoma, glioma, non-small cell lung cancer, lung cancer, colon cancer, head and neck cancer, breast cancer, squamous cell tumors, anal cancer, skin cancer, and vulvar cancer.

The unique specificity of anti-EGFR ADCs provides diagnostic and therapeutic uses to identify, characterize, target and treat, reduce or eliminate a number of tumorigenic cell types and tumor types, for example, but not limited to, glioblastoma, non-small cell lung cancer, lung cancer, colon cancer, head and neck cancer, breast cancer, squamous cell tumors, anal cancer, skin cancer and vulvar cancer, without the problems associated with normal tissue uptake that may be seen with previously known EGFR antibodies. Thus, cells overexpressing EGFR (*e.g.* by amplification or expression of a mutant or variant EGFR), and in particular embodiments, those demonstrating aberrant post-translational modification may be recognized, isolated, characterized, targeted and treated or eliminated utilizing anti-EGFR ADCs.

In one aspect of the invention, there is provided a method for treating a subject comprising administering a therapeutically effective amount of an anti-EGFR ADC in any of the formulations as described herein, wherein the subject has a disorder requiring treatment with the anti-EGFR antibody in the formulation (*e.g.* a tumor, a cancerous condition, a precancerous condition, and any condition related to or resulting from hyperproliferative cell growth), such as, but not limited to, a solid tumor likely to over-express the Epidermal Growth Factor Receptor (EGFR) or glioblastoma multiforme.

Methods for detecting expression of EGFR in a tumor are known in the art, *e.g.,* the EGFR pharmDx™ Kit (Dako). In contrast, an "EGFR negative tumor" is defined as a tumor having an absence of EGFR membrane staining above background in a tumor sample as determined by immunohistochemical techniques.

Formulations comprising anti-EGFR ADCs can thus specifically be used to categorize the nature of EGFR tumors or tumorigenic cells, by staining or otherwise recognizing those tumors or cells wherein EGFR overexpression, particularly amplification and/or EGFR mutation, particularly de2-7 EGFR, is present. Further, anti-EGFR ADCs have been shown to demonstrate significant in vivo anti-tumor activity against tumors containing amplified EGFR and against de2-7 EGFR positive xenografts.

Therefore, in a further aspect of the invention, there is provided a method of treatment of a tumor, a cancerous condition, a precancerous condition, and any condition related to or resulting from hyperproliferative cell growth comprising administration of an anti-EGFR ADC in a formulation described herein.

Various delivery systems are known and can be used to administer formulations comprising anti-EGFR ADCs. Methods of introduction include but are not limited to intradermal, intramuscular, intraperitoneal, intravenous, subcutaneous, intranasal, epidural, and oral routes. The ADCs can be administered, for example by infusion or bolus injection, by absorption through epithelial or mucocutaneous linings (*e.g*., oral mucosa, rectal and intestinal mucosa, and the like) and can be administered together with other biologically active agents such as chemotherapeutic agents. Administration can be systemic or local. In one embodiment, the formulation of the invention is delivered to a subject intravenously. In another embodiment, the formulation of the invention is delivered to a subject subcutaneously. In one embodiment, the subject administers the formulation to himself/herself (self-administration).

The amount of the ADC that is effective in the treatment or prevention of a disorder requiring treatment with the anti-EGFR ADC in the formulation, *e.g.* a cancer, can be determined by standard clinical techniques. In addition, *in vitro* assays may optionally be employed to help identify optimal dosage ranges. The precise dose will also depend on the route of administration, and the stage of immunological disorder or EGFR-expressing cancer, and may be decided according to the judgment of the practitioner and each patient's circumstances. In one embodiment, a therapeutically effective amount of the anti-EGFR ADC in the formulation is administered to a subject in need thereof. The term "therapeutically effective amount" or "effective amount" of an anti-EGFR ADC, as used herein, refers to an amount effective in the prevention or treatment or alleviation of a symptom of a disorder for the treatment of which the ADC is effective. An example of a therapeutically effective amount of the anti-EGFR ADC in the formulation is an amount sufficient to inhibit detrimental EGFR activity or treat a disorder in which EGFR activity is detrimental.

A dose of an anti-EGFR ADC can be administered, for example, daily, once per week (weekly), twice per week, thrice per week, four times per week, five times per week, biweekly, every three weeks, monthly or every four weeks, or otherwise as needed.

In certain embodiments, In certain embodiments, the anti-EGFR ADC can be co-administered to a subject with one or more additional therapeutic agents to treat cancer. The term "co-administered" means the administration of two or more different pharmaceutical agents or treatments (*e.g*., radiation treatment) that are administered to a subject by combination in the same pharmaceutical composition or separate pharmaceutical compositions. Thus co-administration involves administration at the same time of a single pharmaceutical composition comprising two or more pharmaceutical agents or administration of two or more different compositions to the same subject at the same or different times.

Anti-EGFR ADCs (or formulations comprising anti-EGFR ADCs) can be co-administered with a therapeutically effective amount of one or more agents to treat a cancer, where examples of the agents include, such as radiation, alkylating agents, angiogenesis inhibitors, antibodies, antimetabolites, antimitotics, antiproliferatives, antivirals, aurora kinase inhibitors, apoptosis promoters (for example, Bcl-xL, Bcl-w and Bfl-1) inhibitors, activators of death receptor pathway, Bcr-Abl kinase inhibitors, BiTE (Bi-Specific T cell Engager) antibodies, antibody drug conjugates, biologic response modifiers, cyclin-dependent kinase inhibitors, cell cycle inhibitors, cyclooxygenase-2 inhibitors, DVDs (dual variable domain antibodies), leukemia viral oncogene homolog (ErbB2) receptor inhibitors, growth factor inhibitors, heat shock protein (HSP)-90 inhibitors, histone deacetylase (HDAC) inhibitors, hormonal therapies, immunologicals, inhibitors of inhibitors of apoptosis proteins (IAPs), intercalating antibiotics, kinase inhibitors, kinesin inhibitors, Jak2 inhibitors, mammalian target of rapamycin inhibitors, microRNA's, mitogen-activated extracellular signal-regulated kinase inhibitors, multivalent binding proteins, non-steroidal anti-inflammatory drugs (NSAIDs), poly ADP (adenosine diphosphate)-ribose polymerase (PARP) inhibitors, platinum chemotherapeutics, polo-like kinase (Plk) inhibitors, phosphoinositide-3 kinase (bromodomain) inhibitors, proteosome inhibitors, purine analogs, pyrimidine analogs, receptor tyrosine kinase inhibitors, etinoids/deltoids plant alkaloids, small inhibitory ribonucleic acids (siRNAs), topoisomerase inhibitors, ubiquitin ligase inhibitors, and the like, and in combination with one or more of these agents.

Anti-EGFR ADCs (or formulations comprising anti-EGFR ADCs) can be co-administered with a therapeutically effective amount of one or more agents to treat a cancer, including BiTE antibodies, which are bi-specific antibodies that direct T-cells to attack cancer cells by simultaneously binding the two cells. The T-cell then attacks the target cancer cell. Examples of BiTE antibodies include adecatumumab (Micromet MT201), blinatumomab (Micromet MT103) and the like. Without being limited by theory, one of the mechanisms by which T-cells elicit apoptosis of the target cancer cell is by exocytosis of cytolytic granule components, which include perforin and granzyme B. In this regard, Bcl-2 has been shown to attenuate the induction of apoptosis by both perforin and granzyme B. These data suggest that inhibition of Bcl-2 could enhance the cytotoxic effects elicited by T-cells when targeted to cancer cells (V.R. Sutton, D.L. Vaux and J.A. Trapani, J. of Immunology 1997, 158 (12), 5783).

Anti-EGFR ADCs (or formulations comprising anti-EGFR ADCs) can be co-administered with a therapeutically effective amount of one or more agents to treat a cancer, including siRNA. SiRNAs are molecules having endogenous RNA bases or chemically modified nucleotides. The modifications do not abolish cellular activity, but rather impart increased stability and/or increased cellular potency. Examples of chemical modifications include phosphorothioate groups, 2'-deoxynucleotide, 2'-OCH₃-containing ribonucleotides, 2'-F-ribonucleotides, 2'-methoxyethyl ribonucleotides, combinations thereof and the like. The siRNA can have varying lengths (*e.g.,* 10-200 bps) and structures (*e.g.,* hairpins, single/double strands, bulges, nicks/gaps, mismatches) and are processed in cells to provide active gene silencing. A double-stranded siRNA (dsRNA) can have the same number of nucleotides on each strand (blunt ends) or asymmetric ends (overhangs). The overhang of 1-2 nucleotides can be present on the sense and/or the antisense strand, as well as present on the 5'- and/ or the 3'-ends of a given strand.

Anti-EGFR ADCs (or formulations comprising anti-EGFR ADCs) can be co-administered with a therapeutically effective amount of one or more agents to treat a cancer, including DVDs and other multivalent binding proteins. Multivalent binding proteins are binding proteins comprising two or more antigen binding sites. Multivalent binding proteins are engineered to have the three or more antigen binding sites and are generally not naturally occurring antibodies. The term "multispecific binding protein" means a binding protein capable of binding two or more related or unrelated targets. Dual variable domain (DVD) binding proteins are tetravalent or multivalent binding proteins binding proteins comprising two or more antigen binding sites. Such DVDs may be monospecific (i.e., capable of binding one antigen) or multispecific (i.e., capable of binding two or more antigens). DVD binding proteins comprising two heavy chain DVD polypeptides and two light chain DVD polypeptides are referred to as DVD Ig's. Each half of a DVD Ig comprises a heavy chain DVD polypeptide, a light chain DVD polypeptide, and two antigen binding sites. Each binding site comprises a heavy chain variable domain and a light chain variable domain with a total of 6 CDRs involved in antigen binding per antigen binding site. Multispecific DVDs include DVD binding proteins that bind DLL4 and VEGF, or C-met and EGFR or ErbB3 and EGFR.

Anti-EGFR ADCs (or formulations comprising anti-EGFR ADCs) can be co-administered with a therapeutically effective amount of one or more agents to treat a cancer, including alkylating agents. Alkylating agents include altretamine, AMD-473, AP-5280, apaziquone, bendamustine, brostallicin, busulfan, carboquone, carmustine (BCNU), chlorambucil, CLORETAZINE® (laromustine, VNP 40101M), cyclophosphamide, decarbazine, estramustine, fotemustine, glufosfamide, ifosfamide, KW-2170, lomustine (CCNU), *mafosfamide,* melphalan, mitobronitol, mitolactol, nimustine, nitrogen mustard N-oxide, ranimustine, temozolomide, thiotepa, TREANDA® (bendamustine), treosulfan, rofosfamide and the like.

Anti-EGFR ADCs (or formulations comprising anti-EGFR ADCs) can be co-administered with a therapeutically effective amount of one or more agents to treat a cancer, including angiogenesis inhibitors. Angiogenesis inhibitors include endothelial-specific receptor tyrosine kinase (Tie-2) inhibitors, epidermal growth factor receptor (EGFR) inhibitors, insulin growth factor-2 receptor (IGFR-2) inhibitors, matrix metalloproteinase-2 (MMP-2) inhibitors, matrix metalloproteinase-9 (MMP-9) inhibitors, platelet-derived growth factor receptor (PDGFR) inhibitors, thrombospondin analogs, vascular endothelial growth factor receptor tyrosine kinase (VEGFR) inhibitors and the like.

Anti-EGFR ADCs (or formulations comprising anti-EGFR ADCs) can be co-administered with a therapeutically effective amount of one or more agents to treat a cancer, including antimetabolites. Antimetabolites include ALIMTA® (pemetrexed disodium, LY231514, MTA), 5-azacitidine, XELODA® (capecitabine), carmofur, LEUSTAT® (cladribine), clofarabine, cytarabine, cytarabine ocfosfate, cytosine arabinoside, decitabine, deferoxamine, doxifluridine, eflornithine, EICAR (5-ethynyl-1-β-D-ribofuranosylimidazole-4-carboxamide), enocitabine, ethnylcytidine, fludarabine, 5-fluorouracil alone or in combination with leucovorin, GEMZAR® (gemcitabine), hydroxyurea, ALKERAN®(melphalan), mercaptopurine, 6-mercaptopurine riboside, methotrexate, mycophenolic acid, nelarabine, nolatrexed, ocfosfate, pelitrexol, pentostatin, raltitrexed, Ribavirin, triapine, trimetrexate, S-1, tiazofurin, tegafur, TS-1, vidarabine, UFT and the like.

Anti-EGFR ADCs (or formulations comprising anti-EGFR ADCs) can be co-administered with a therapeutically effective amount of one or more agents to treat a cancer, including antivirals. Antivirals include ritonavir, hydroxychloroquine and the like.

Anti-EGFR ADCs (or formulations comprising anti-EGFR ADCs) can be co-administered with a therapeutically effective amount of one or more agents to treat a cancer, including aurora kinase inhibitors. Aurora kinase inhibitors include ABT-348, AZD-1152, MLN-8054, VX-680, Aurora A-specific kinase inhibitors, Aurora B-specific kinase inhibitors and pan-Aurora kinase inhibitors and the like.

Anti-EGFR ADCs (or formulations comprising anti-EGFR ADCs) can be co-administered with a therapeutically effective amount of one or more agents to treat a cancer, including Bcl-2 protein inhibitors. Bcl-2 protein inhibitors include AT-101 ((-)gossypol), GENASENSE® (G3139 or oblimersen (Bcl-2-targeting antisense oligonucleotide)), IPI-194, IPI-565, N-(4-(4-((4'-chloro(1,1'-biphenyl)-2-yl)methyl)piperazin-1-yl)benzoyl)-4-(((1R)-3-(dimethylamino)-1-((phenylsulfanyl)methyl)propyl)amino)-3-nitrobenzenesulfonamide) (ABT-737), N-(4-(4-((2-(4-chlorophenyl)-5,5-dimethyl-1-cyclohex-1-en-1-yl)methyl)piperazin-1-yl)benzoyl)-4-(((1R)-3-(morpholin-4-yl)-1-((phenylsulfanyl)methyl)propyl)amino)-3-((trifluoromethyl)sulfonyl)benzenesulfonamide (ABT-263), GX-070 (obatoclax), ABT-199, and the like.

Anti-EGFR ADCs (or formulations comprising anti-EGFR ADCs) can be co-administered with a therapeutically effective amount of one or more agents to treat a cancer, including Bcr-Abl kinase inhibitors, such as DASATINIB® (BMS-354825), GLEEVEC® (imatinib) and the like.

Anti-EGFR ADCs (or formulations comprising anti-EGFR ADCs) can be co-administered with a therapeutically effective amount of one or more agents to treat a cancer, including CDK inhibitors. CDK inhibitors include AZD-5438, BMI-1040, BMS-032, BMS-387, CVT-2584, flavopyridol, GPC-286199, MCS-5A, PD0332991, PHA-690509, seliciclib (CYC-202, R-roscovitine), ZK-304709 and the like.

Anti-EGFR ADCs (or formulations comprising anti-EGFR ADCs) can be co-administered with a therapeutically effective amount of one or more agents to treat a cancer, including COX-2 inhibitors. COX-2 inhibitors include ABT-963, ARCOXIA® (etoricoxib), BEXTRA® (valdecoxib), BMS347070, CELEBREX® (celecoxib), COX-189 (lumiracoxib), CT-3, DERAMAXX® (deracoxib), JTE-522, 4-methyl-2-(3,4-dimethylphenyl)-1-(4-sulfamoylphenyl-1H-pyrrole), MK-663 (etoricoxib), NS-398, parecoxib, RS-57067, SC-58125, SD-8381, SVT-2016, S-2474, T-614, VIOXX® (rofecoxib) and the like.

Anti-EGFR ADCs (or formulations comprising anti-EGFR ADCs) can be co-administered with a therapeutically effective amount of one or more agents to treat a cancer, including other EGFR inhibitors. EGFR inhibitors include EGFR antibodies, ABX-EGF, anti-EGFR immunoliposomes, EGF-vaccine, EMD-7200, ERBITUX® (cetuximab), HR3, IgA antibodies, IRESSA® (gefitinib), TARCEVA® (erlotinib or OSI-774), TP-38, EGFR fusion protein, TYKERB® (lapatinib) and the like.

Anti-EGFR ADCs (or formulations comprising anti-EGFR ADCs) can be co-administered with a therapeutically effective amount of one or more agents to treat a cancer, including HER2 inhibitors. ErbB2 receptor inhibitors include CP-724-714, CI-1033 (canertinib), HERCEPTIN® (trastuzumab), TYKERB® (lapatinib), OMNITARG® (2C4, petuzumab), TAK-165, GW-572016 (ionafarnib), GW-282974, EKB-569, PI-166, dHER2 (HER2 vaccine), APC-8024 (HER-2 vaccine), anti-HER/2neu bispecific antibody, B7.her2IgG3, AS HER2 trifunctional bispecfic antibodies, mAB AR-209, mAB 2B-1 and the like.

Anti-EGFR ADCs (or formulations comprising anti-EGFR ADCs) can be co-administered with a therapeutically effective amount of one or more agents to treat a cancer, including histone deacetylase inhibitors, such as depsipeptide, LAQ-824, MS-275, trapoxin, suberoylanilide hydroxamic acid (SAHA), TSA, valproic acid and the like.

HSP-90 inhibitors include 17-AAG-nab, 17-AAG, CNF-101, CNF-1010, CNF-2024, 17-DMAG, geldanamycin, IPI-504, KOS-953, MYCOGRAB® (human recombinant antibody to HSP-90), NCS-683664, PU24FCl, PU-3, radicicol, SNX-2112, STA-9090 VER49009 and the like.

Anti-EGFR ADCs (or formulations comprising anti-EGFR ADCs) can be co-administered with a therapeutically effective amount of one or more agents to treat a cancer, including inhibitors of inhibitors of apoptosis proteins, such as HGS1029, GDC-0145, GDC-0152, LCL-161, LBW-242 and the like.

Anti-EGFR ADCs (or formulations comprising anti-EGFR ADCs) can be co-administered with a therapeutically effective amount of one or more agents to treat a cancer, including other ADCs, such as anti-CD22-MC-MMAF, anti-CD22-MC-MMAE, anti-CD22-MCC-DM1, CR-011-vcMMAE, PSMA-ADC, MEDI-547, SGN-19Am SGN-35, SGN-75 ADCs and the like

Anti-EGFR ADCs (or formulations comprising anti-EGFR ADCs) can be co-administered with a therapeutically effective amount of one or more agents to treat a cancer, including activators of death receptor pathway, such as TRAIL, antibodies or other agents that target TRAIL or death receptors (*e.g.,* DR4 and DR5) such as Apomab, conatumumab, ETR2-ST01, GDC0145, (*lexatumumab*), HGS-1029, LBY-135, PRO-1762 and trastuzumab.

Anti-EGFR ADCs (or formulations comprising anti-EGFR ADCs) can be co-administered with a therapeutically effective amount of one or more agents to treat a cancer, including kinesin inhibitors, such as Eg5 inhibitors such as AZD4877, ARRY-520; CENPE inhibitors such as GSK923295A and the like.

Anti-EGFR ADCs (or formulations comprising anti-EGFR ADCs) can be co-administered with a therapeutically effective amount of one or more agents to treat a cancer, including JAK-2 inhibitors, such as CEP-701 (lesaurtinib), XL019 and INCB018424 and the like.

Anti-EGFR ADCs (or formulations comprising anti-EGFR ADCs) can be co-administered with a therapeutically effective amount of one or more agents to treat a cancer, including MEK inhibitors, such as ARRY-142886, ARRY-438162 PD-325901, PD-98059 and the like.

Anti-EGFR ADCs (or formulations comprising anti-EGFR ADCs) can be co-administered with a therapeutically effective amount of one or more agents to treat a cancer, including mTOR inhibitors, such as AP-23573, CCI-779, everolimus, RAD-001, rapamycin, temsirolimus, ATP-competitive TORC1/TORC2 inhibitors, including PI-103, PP242, PP30, Torin 1 and the like.

Anti-EGFR ADCs (or formulations comprising anti-EGFR ADCs) can be co-administered with a therapeutically effective amount of one or more agents to treat a cancer, including non-steroidal anti-inflammatory drugs (NSAIDs), such as AMIGESIC® (salsalate), DOLOBID® (diflunisal), MOTRIN® (ibuprofen), ORUDIS® (ketoprofen), RELAFEN® (nabumetone), FELDENE® (piroxicam), ibuprofen cream, ALEVE® (naproxen) and NAPROSYN® (naproxen), VOLTAREN® (diclofenac), INDOCIN® (indomethacin), CLINORIL® (sulindac), TOLECTIN® (tolmetin), LODINE® (etodolac), TORADOL® (ketorolac), DAYPRO® (oxaprozin) and the like.

Anti-EGFR ADCs (or formulations comprising anti-EGFR ADCs) can be co-administered with a therapeutically effective amount of one or more agents to treat a cancer, including PDGFR inhibitors, such as C-451, CP-673, CP-868596 and the like.

Anti-EGFR ADCs (or formulations comprising anti-EGFR ADCs) can be co-administered with a therapeutically effective amount of one or more agents to treat a cancer, including platinum chemotherapeutics, such as cisplatin, ELOXATIN® (oxaliplatin) eptaplatin, lobaplatin, nedaplatin, PARAPLATIN® (carboplatin), satraplatin, picoplatin and the like.

Anti-EGFR ADCs (or formulations comprising anti-EGFR ADCs) can be co-administered with a therapeutically effective amount of one or more agents to treat a cancer, including polo-like kinase inhibitors, *e.g.,* BI-2536 and the like.

Anti-EGFR ADCs (or formulations comprising anti-EGFR ADCs) can be co-administered with a therapeutically effective amount of one or more agents to treat a cancer, including phosphoinositide-3 kinase (PI3K) inhibitors, such as wortmannin, LY294002, XL-147, CAL-120, ONC-21, AEZS-127, ETP-45658, PX-866, GDC-0941, BGT226, BEZ235, XL765 and the like.

Anti-EGFR ADCs (or formulations comprising anti-EGFR ADCs) can be co-administered with a therapeutically effective amount of one or more agents to treat a cancer, including thrombospondin analogs, such as ABT-510 (thrombospondin mimetic), ABT-567, ABT-898 (thrombospondin-1 mimetic peptide), TSP-1 and the like.

Anti-EGFR ADCs (or formulations comprising anti-EGFR ADCs) can be co-administered with a therapeutically effective amount of one or more agents to treat a cancer, including VEGFR inhibitors, such as AVASTIN® (bevacizumab), ABT-869, AEE-788, ANGIOZYME™ (a ribozyme that inhibits angiogenesis (Ribozyme Pharmaceuticals (Boulder, CO.) and Chiron, (Emeryville, CA)), axitinib (AG-13736), AZD-2171, CP-547,632, IM-862, MACUGEN (pegaptamib), NEXAVAR® (sorafenib, BAY43-9006), pazopanib (GW-786034), vatalanib (PTK-787, ZK-222584), SUTENT® (sunitinib, SU-11248), VEGF trap, ZACTIMA™ (vandetanib, ZD-6474), GA101, ofatumumab, ABT-806 (mAb-806), ErbB3 specific antibodies, BSG2 specific antibodies, DLL4 specific antibodies and C-met specific antibodies, and the like. Anti-EGFR ADCs (or formulations comprising anti-EGFR ADCs) can be co-administered with a therapeutically effective amount of one or more agents to treat a cancer, including antibiotics, such as intercalating antibiotics aclarubicin, actinomycin D, amrubicin, annamycin, adriamycin, BLENOXANE® (bleomycin), daunorubicin, CAELYX® or MYOCET® (liposomal doxorubicin), elsamitrucin, epirbucin, glarbuicin, ZAVEDOS® (idarubicin), mitomycin C, nemorubicin, neocarzinostatin, peplomycin, pirarubicin, rebeccamycin, stimalamer, streptozocin, VALSTAR® (valrubicin), zinostatin and the like.

Anti-EGFR ADCs (or formulations comprising anti-EGFR ADCs) can be co-administered with a therapeutically effective amount of one or more agents to treat a cancer, including topoisomerase inhibitors, such as aclarubicin, 9-aminocamptothecin, amonafide, amsacrine, becatecarin, belotecan, BN-80915, CAMPTOSAR® (irinotecan hydrochloride), camptothecin, CARDIOXANE® (dexrazoxine), diflomotecan, edotecarin, ELLENCE® or PHARMORUBICIN® (epirubicin), etoposide, exatecan, 10-hydroxycamptothecin, gimatecan, lurtotecan, mitoxantrone, orathecin, pirarbucin, pixantrone, rubitecan, sobuzoxane, SN-38, tafluposide, topotecan and the like.

Anti-EGFR ADCs (or formulations comprising anti-EGFR ADCs) can be co-administered with a therapeutically effective amount of one or more agents to treat a cancer, including therapeutic antibodies, such as AVASTIN® (bevacizumab), CD40-specific antibodies, chTNT-1/B, denosumab, ERBITUX® (cetuximab), HUMAX-CD4® (zanolimumab), IGF1R-specific antibodies, lintuzumab, PANOREX® (edrecolomab), RENCAREX® (WX G250), RITUXAN® (rituximab), ticilimumab, trastuzimab, CD20 antibodies types I and II and the like.

Anti-EGFR ADCs (or formulations comprising anti-EGFR ADCs) can be co-administered with a therapeutically effective amount of one or more agents to treat a cancer, including hormonal therapies, such as ARIMIDEX® (anastrozole), AROMASIN® (exemestane), arzoxifene, CASODEX® (bicalutamide), CETROTIDE® (cetrorelix), degarelix, deslorelin, DESOPAN® (trilostane), dexamethasone, DROGENIL® (flutamide), EVISTA® (raloxifene), AFEMA™ (fadrozole), FARESTON® (toremifene), FASLODEX® (fulvestrant), FEMARA® (letrozole), formestane, glucocorticoids, HECTOROL® (doxercalciferol), RENAGEL® (sevelamer carbonate), lasofoxifene, leuprolide acetate, MEGACE® (megesterol), MIFEPREX® (mifepristone), NILANDRON™ (nilutamide), NOLVADEX® (tamoxifen citrate), PLENAXIS™ (abarelix), prednisone, PROPECIA® (finasteride), rilostane, SUPREFACT® (buserelin), TRELSTAR® (luteinizing hormone releasing hormone (LHRH)), VANTAS® (Histrelin implant), VETORYL® (trilostane or modrastane), ZOLADEX® (fosrelin, goserelin) and the like.

Anti-EGFR ADCs (or formulations comprising anti-EGFR ADCs) can be co-administered with a therapeutically effective amount of one or more agents to treat a cancer, including deltoids and retinoids, such as seocalcitol (EB1089, CB1093), lexacalcitrol (KH1060), fenretinide, PANRETIN® (aliretinoin), ATRAGEN® (liposomal tretinoin), TARGRETIN® (bexarotene), LGD-1550 and the like.

Anti-EGFR ADCs (or formulations comprising anti-EGFR ADCs) can be co-administered with a therapeutically effective amount of one or more agents to treat a cancer, including PARP inhibitors, such as veliparib, olaparib, KU-59436, AZD-2281, AG-014699, BSI-201, BGP-15, INO-1001, ONO-2231 and the like.

Anti-EGFR ADCs (or formulations comprising anti-EGFR ADCs) can be co-administered with a therapeutically effective amount of one or more agents to treat a cancer, including plant alkaloids, such as, but are not limited to, vincristine, vinblastine, vindesine, vinorelbine and the like.

Anti-EGFR ADCs (or formulations comprising anti-EGFR ADCs) can be co-administered with a therapeutically effective amount of one or more agents to treat a cancer, including proteasome inhibitors, such as VELCADE® (bortezomib), MG132, NPI-0052, PR-171 and the like.

Anti-EGFR ADCs (or formulations comprising anti-EGFR ADCs) can be co-administered with a therapeutically effective amount of one or more agents to treat a cancer, including immunologicals. Examples of immunologicals include interferons and other immune-enhancing agents. Interferons include interferon alpha, interferon alpha-2a, interferon alpha-2b, interferon beta, interferon gamma-la, ACTIMMUNE® (interferon gamma-lb) or interferon gamma-nl, combinations thereof and the like. Other agents include ALFAFERONE®,(IFN-α), BAM-002 (oxidized glutathione), BEROMUN® (tasonermin), BEXXAR® (tositumomab), CAMPATH® (alemtuzumab), CTLA4 (cytotoxic lymphocyte antigen 4), decarbazine, denileukin, epratuzumab, GRANOCYTE® (lenograstim), lentinan, leukocyte alpha interferon, imiquimod, MDX-010 (anti-CTLA-4), melanoma vaccine, mitumomab, molgramostim, MYLOTARG™ (gemtuzumab ozogamicin), NEUPOGEN® (filgrastim), OncoVAC-CL, OVAREX® (oregovomab), pemtumomab (Y-muHMFG1), PROVENGE® (sipuleucel-T), sargaramostim, sizofilan, teceleukin, THERACYS® (Bacillus Calmette-Guerin), ubenimex, VIRULIZIN® (immunotherapeutic, Lorus Pharmaceuticals), Z-100 (Specific Substance of Maruyama (SSM)), WF-10 (Tetrachlorodecaoxide (TCDO)), PROLEUKIN® (aldesleukin), ZADAXIN® (thymalfasin), ZENAPAX® (daclizumab), ZEVALIN® (90Y-Ibritumomab tiuxetan) and the like.

Anti-EGFR ADCs (or formulations comprising anti-EGFR ADCs) can be co-administered with a therapeutically effective amount of one or more agents to treat a cancer, including biological response modifiers, such as agents that modify defense mechanisms of living organisms or biological responses, such as survival, growth or differentiation of tissue cells to direct them to have anti-tumor activity and include krestin, lentinan, sizofiran, picibanil PF-3512676 (CpG-8954), ubenimex and the like.

Anti-EGFR ADCs (or formulations comprising anti-EGFR ADCs) can be co-administered with a therapeutically effective amount of one or more agents to treat a cancer, including pyrimidine analogs, such as cytarabine (ara C or Arabinoside C), cytosine arabinoside, doxifluridine, FLUDARA® (fludarabine), 5-FU (5-fluorouracil), floxuridine, GEMZAR® (gemcitabine), TOMUDEX® (ratitrexed), TROXATYL™ (triacetyluridine troxacitabine) and the like.

Anti-EGFR ADCs (or formulations comprising anti-EGFR ADCs) can be co-administered with a therapeutically effective amount of one or more agents to treat a cancer, including purine analogs, such as LANVIS® (thioguanine) and PURI-NETHOL® (mercaptopurine).

Anti-EGFR ADCs (or formulations comprising anti-EGFR ADCs) can be co-administered with a therapeutically effective amount of one or more agents to treat a cancer, including antimitotic agents, such as batabulin, epothilone D (KOS-862), N-(2-((4-hydroxyphenyl)amino)pyridin-3-yl)-4-methoxybenzenesulfonamide, ixabepilone (BMS 247550), paclitaxel, TAXOTERE® (docetaxel), PNU100940 (109881), patupilone, XRP-9881 (larotaxel), vinflunine, ZK-EPO (synthetic epothilone) and the like.

Anti-EGFR ADCs (or formulations comprising anti-EGFR ADCs) can be co-administered with a therapeutically effective amount of one or more agents to treat a cancer, including ubiquitin ligase inhibitors, such as MDM2 inhibitors, such as nutlins, NEDD8 inhibitors such as MLN4924 and the like.

Compounds of this invention can also be used as radiosensitizers that enhance the efficacy of radiotherapy. Examples of radiotherapy include external beam radiotherapy, teletherapy, brachytherapy and sealed, unsealed source radiotherapy and the like.

Anti-EGFR ADCs (or formulations comprising anti-EGFR ADCs) can be co-administered with a therapeutically effective amount of one or more agents to treat a cancer, including chemotherapeutic agents such as ABRAXANE™ (ABI-007), ABT-100 (farnesyl transferase inhibitor), ADVEXIN® (Ad5CMV-p53 vaccine), ALTOCOR® or MEVACOR® (lovastatin), AMPLIGEN® (poly I:poly C12U, a synthetic RNA), APTOSYN® (exisulind), AREDIA® (pamidronic acid), arglabin, L-asparaginase, atamestane (1-methyl-3,17-dione-androsta-1,4-diene), AVAGE® (tazarotene), AVE-8062 (combreastatin derivative) BEC2 (mitumomab), cachectin or cachexin (tumor necrosis factor), canvaxin (vaccine), CEAVAC® (cancer vaccine), CELEUK® (celmoleukin), CEPLENE® (histamine dihydrochloride), CERVARIX® (human papillomavirus vaccine), CHOP® (C: CYTOXAN® (cyclophosphamide); H: ADRIAMYCIN® (hydroxydoxorubicin); O: Vincristine (ONCOVIN®); P: prednisone), CYPAT™ (cyproterone acetate), combrestatin A4P, DAB(389)EGF (catalytic and translocation domains of diphtheria toxin fused via a His-Ala linker to human epidermal growth factor) or TransMID-107R™ (diphtheria toxins), dacarbazine, dactinomycin, 5,6-dimethylxanthenone-4-acetic acid (DMXAA), eniluracil, EVIZON™ (squalamine lactate), DIMERICINE® (T4N5 liposome lotion), discodermolide, DX-8951f (exatecan mesylate), enzastaurin, EPO906 (epithilone B), GARDASIL® (quadrivalent human papillomavirus (Types 6, 11, 16, 18) recombinant vaccine), GASTRIMMUNE®, GENASENSE®, GMK (ganglioside conjugate vaccine), GVAX® (prostate cancer vaccine), halofuginone, histerelin, hydroxycarbamide, ibandronic acid, IGN-101, IL-13-PE38, IL-13-PE38QQR (cintredekin besudotox), IL-13-pseudomonas exotoxin, interferon-a, interferon-y, JUNOVAN™ or MEPACT™ (mifamurtide), lonafarnib, 5,10-methylenetetrahydrofolate, miltefosine (hexadecylphosphocholine), NEOVASTAT®(AE-941), NEUTREXIN® (trimetrexate glucuronate), NIPENT® (pentostatin), ONCONASE® (a ribonuclease enzyme), ONCOPHAGE® (melanoma vaccine treatment), ONCOVAX® (IL-2 Vaccine), ORATHECIN™ (rubitecan), OSIDEM® (antibody-based cell drug), OVAREX® MAb (murine monoclonal antibody), paclitaxel, PANDIMEX™ (aglycone saponins from ginseng comprising 20(S)protopanaxadiol (aPPD) and 20(S)protopanaxatriol (aPPT)), panitumumab, PANVAC®-VF (investigational cancer vaccine), pegaspargase, PEG Interferon A, phenoxodiol, procarbazine, rebimastat, REMOVAB® (catumaxomab), REVLIMID® (lenalidomide), RSR13 (efaproxiral), SOMATULINE® LA (lanreotide), SORIATANE® (acitretin), staurosporine (Streptomyces staurospores), talabostat (PT100), TARGRETIN® (bexarotene), TAXOPREXIN® (DHA-paclitaxel), *TELCYTA*® (canfosfamide, TLK286), temilifene, TEMODAR® (temozolomide), tesmilifene, thalidomide, THERATOPE® (STn-KLH), thymitaq (2-amino-3,4-dihydro-6-methyl-4-oxo-5-(4-pyridylthio)quinazoline dihydrochloride), TNFERADE™ (adenovector: DNA carrier containing the gene for tumor necrosis factor-α), TRACLEER® or ZAVESCA® (bosentan), tretinoin (Retin-A), tetrandrine, TRISENOX® (arsenic trioxide), VIRULIZIN®, ukrain (derivative of alkaloids from the greater celandine plant), vitaxin (anti-alphavbeta3 antibody), XCYTRIN® (motexafin gadolinium), XINLAY™ (atrasentan), XYOTAX™ (paclitaxel poliglumex), YONDELIS® (trabectedin), ZD-6126, ZINECARD® (dexrazoxane), ZOMETA® (zolendronic acid), zorubicin and the like.

In one embodiment, the formulation comprising the anti-EGFR-ADC is intravenously administered to a subject having glioblastoma in combination with radiation and/or TEMODAR® (temozolomide).

Further, in one embodiment, the formulation can be provided as a pharmaceutical kit comprising (a) a container containing an anti-EGFR ADC in lyophilized form and (b) a second container containing a pharmaceutically acceptable diluent (e.g., sterile water) for injection. The pharmaceutically acceptable diluent can be used for reconstitution or dilution of the lyophilized ADC. Optionally associated with such container(s) can be a notice in the form prescribed by a governmental agency regulating the manufacture, use or sale of pharmaceuticals or biological products, which notice reflects approval by the agency of manufacture, use or sale for human administration.

The invention is further described in the following examples, which are in not intended to limit the scope of the invention.

### EXAMPLES

### Example 1: Stability Testing of Antibody Drug Conjugates (ADCs)

The following example describes tests used to assay the stability of certain ADCs (in liquid form) in comparison to unconjugated antibodies. Antibodies conjugated to either MMAF (see Figure 2) (referred to below as "ADC 1- MMAF" which is humanized anti-EGFR antibody 1- MMAF conjugate) or MMAE (see Figure 1) (referred to below as "ADC 1- MMAE" which is humanized anti-EGFR antibody 1 -MMAE conjugate) were tested and compared to humanized anti-EGFR antibody 1 alone. Among the properties examined were the onset of unfolding temperature using dynamic scanning fluorescence and DSC, secondary and tertiary structure analysis by FTIR and near UV-CD, respectively, accelerated stability at low and high concentrations, serum stability, freeze/thaw stability at low and high concentrations and solubility. The formulations described in this example were liquid formulations.

### Analysis of onset of unfolding using Dynamic Scanning Fluorescence (DSF) and Differential Scanning Calorimetry (DSC)

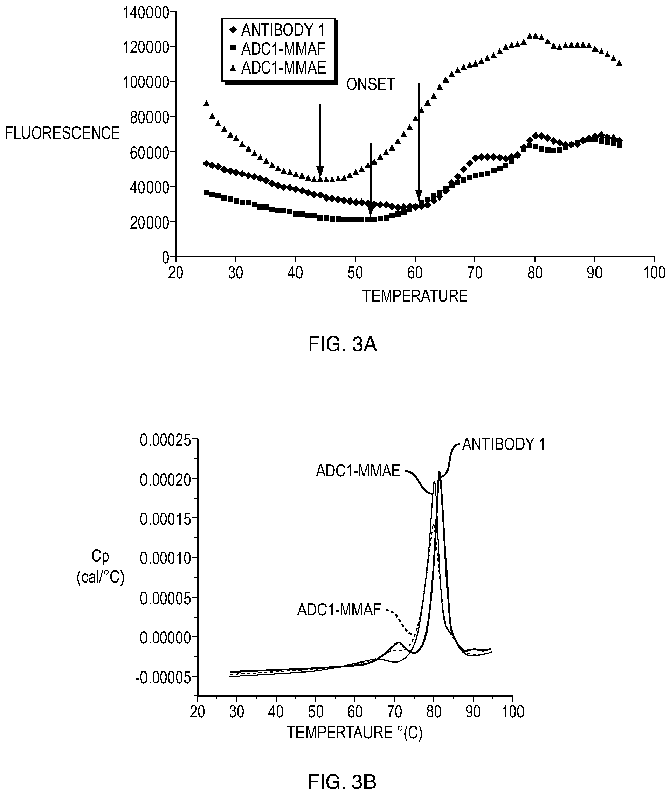

Two different techniques, dynamic scanning fluorescence (DSF) and differential scanning calorimetry (DSC), were used to determine the onset of unfolding and the conformational stability of antibody 1, ADC 1- MMAF and ADC 1- MMAE during thermal denaturation. As shown in Figure 3A, the change in fluorescence intensity was related to protein unfolding levels and temperatures. The results from thermostability measurements were compared with the data acquired using DSC (Figure 3B). Unfolding of the protein at a temperature greater than 55° C was used as a measure of stability. As shown in Figure 3A and B, the onset of unfolding occurred at a lower temperature in ADC 1- MMAE (46° C) compared to ADC 1- MMAF (55° C), and was highest for antibody 1 (61° C), indicating antibody 1 was the most stable of the three molecules. Thus, the ADCs showed thermodynamic stability decrease, as was reflected in the lower unfolding temperatures for ADC 1- MMAF and ADC-1 MMAE vs. antibody 1 alone.

### Secondary and tertiary structure analyses of antibody 1, ADC 1- MMAF and ADC 1-MMAE

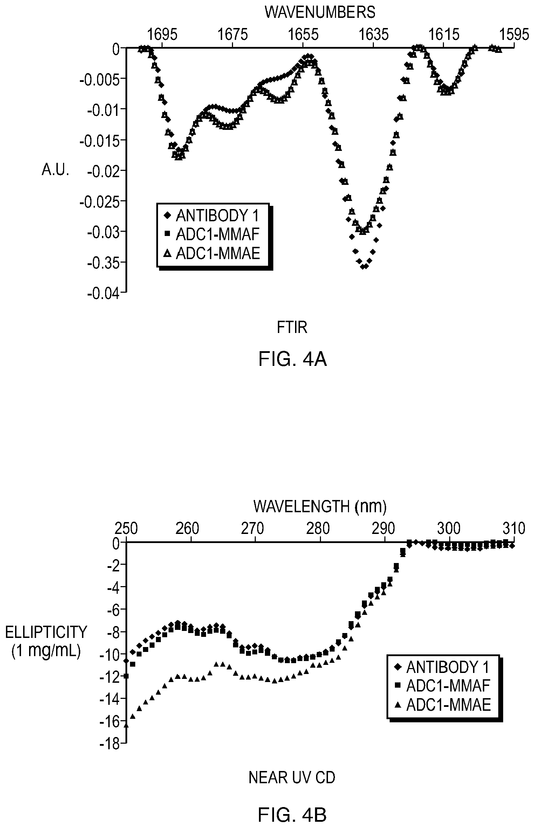

The stabilities of antibody 1 , ADC 1- MMAF, and ADC 1-MMAE were determined using both Fourier Transform Infrared Spectroscopy (FTIR) and near UV- Circular Dichroism (CD). It has been shown that CD spectra between 260 and approximately 180 nm can be analyzed for the different secondary structural types: alpha helix, parallel and antiparallel beta sheet, turn, and other. Changes in secondary structure of antibody 1, the ADC 1- MMAF and ADC 1-MMAE in citrate / phosphate buffer alone at pH 5/6/7 were monitored by FTIR (Figure 4A) and CD (Figure 4B). As shown in Figures 4A and 4B, physical characteristics of ADC 1- MMAE and ADC 1- MMAF were slightly altered in unstructured elements compared to antibody 1. There were also slightly altered unstructured elements in ADC 1- MMAE and ADC 1- MMAF compared to antibody 1 alone, as evidenced by the FTIR data in Figure 4A. In general, however, each of the three molecules showed the presence of > 40% β sheet band at 1638 cm⁻¹. CD results showed a different profile for ADC 1- MMAE as compared to ADC 1- MMAF and antibody 1 (Figure 4B). Generally, however, each of the molecules had an S-shaped profile with negative ellipticity at 280 nm.

### Accelerated stability studies of antibody 1, ADC 1- MMAF and ADC 1-MMAE

Accelerated stability studies can help provide information on the effect of short term exposure to conditions that otherwise typically occur over a longer period. Antibody 1, ADC 1- MMAF and ADC 1- MMAE were formulated at both low concentrations (1 mg/ml) in 10 mM citrate/ phosphate buffer, pH 6, and higher concentrations (60 mg/ml) in 15 mM histidine, pH 5.75. Stability was defined as less than 5% monomer loss at 40°C after 7 days at most stable pH

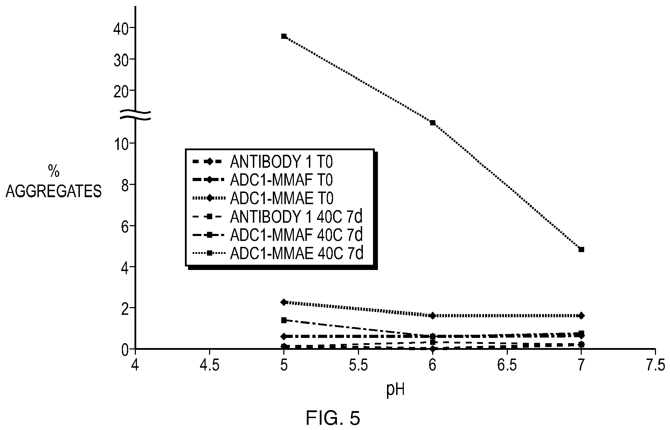

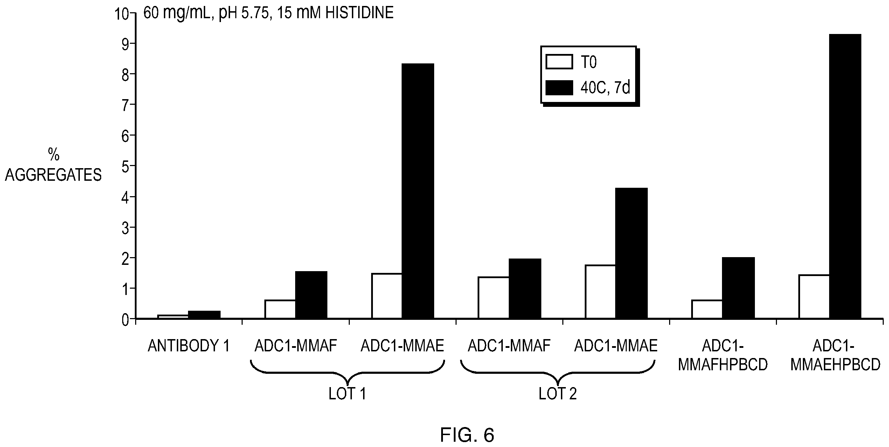

The results of these studies are shown in Figure 5 and Figure 6. Figure 5 shows aggregation (as % aggregates; determined by SEC analysis) at an initial time point (T0), and after storage at 40°C for 7 days. Figure 5 shows that at a concentration of 1 mg/ml, there was an increased aggregation propensity of ADC 1- MMAE at low concentrations, compared to ADC 1- MMAF and antibody 1. Figure 6 shows aggregation (as % aggregates) at initial time point (T0), and after storage at 40 C for 7 days. Figure 6 shows that at a concentration of 60 mg/ml, there is a significantly increased aggregation propensity of ADC 1-MMAE at high concentrations, as compared to ADC 1- MMAF and antibody 1. Further, there was no impact of known stabilizing agents like hydroxy propyl beta cyclodextrin (labeled "HPBCD" in "Figure 6) on aggregation.

### Plasma stability of antibody 1, ADC 1-MMAF and ADC 1-MMAE

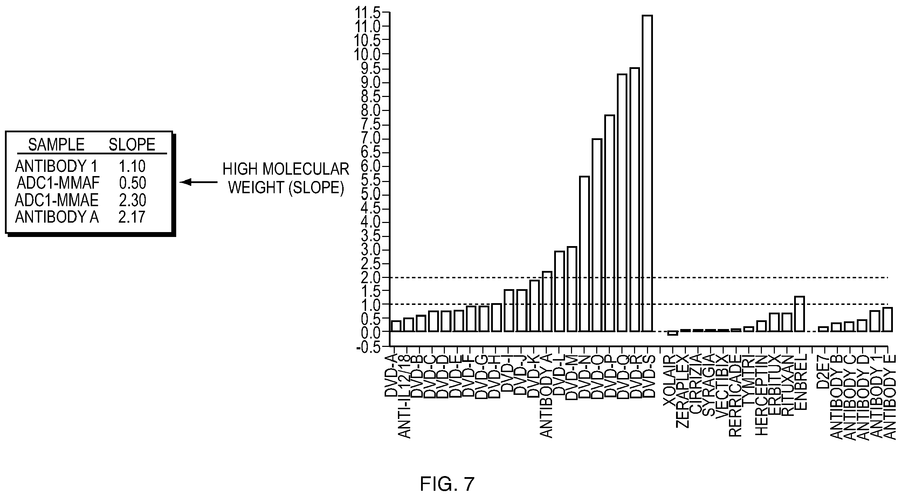

Plasma stability plays an important role in drug discovery and development, as unstable compounds tend to have rapid clearance and short half-life, resulting in poor *in vivo* performance. The *in vitro* serum stability of antibody 1, ADC 1- MMAF and ADC 1-MMAE was evaluated in a serum stability assay. Briefly, the antibodies were labeled with Aexa Flur® (Life Technologies). Labeled antibody 1 and ADC-1-MMAF and ADC-1-MMAE were then incubated in filtered serum. Samples were collected on days 0, 1, 3, 5, 7, and analyzed by size-exclusion chromatography (SEC). The slope of the percent of high molecular weight (HMW) aggregates was calculated between days 0-7. Stability was defined as less than 1% HMW species per day. Thus, the lower the slope, the less aggregation was present. More specifically, the graph in Figure 7 shows that the slope of the ADC 1-MMAF (0.5%) was lower than antibody 1 (1.10%) and ADC 1-MMAE (2.30), thus, indicating less aggregation and better plasma stability. Figure 7 also describes the slopes of the percent of HMW aggregates for a range of other molecules, *e.g.,* antibodies and DVD-Igs, for comparison purposes.

### Freeze/thaw stability of high and low concentrations of antibody 1, ADC 1-MMAF and ADC 1--MMAE

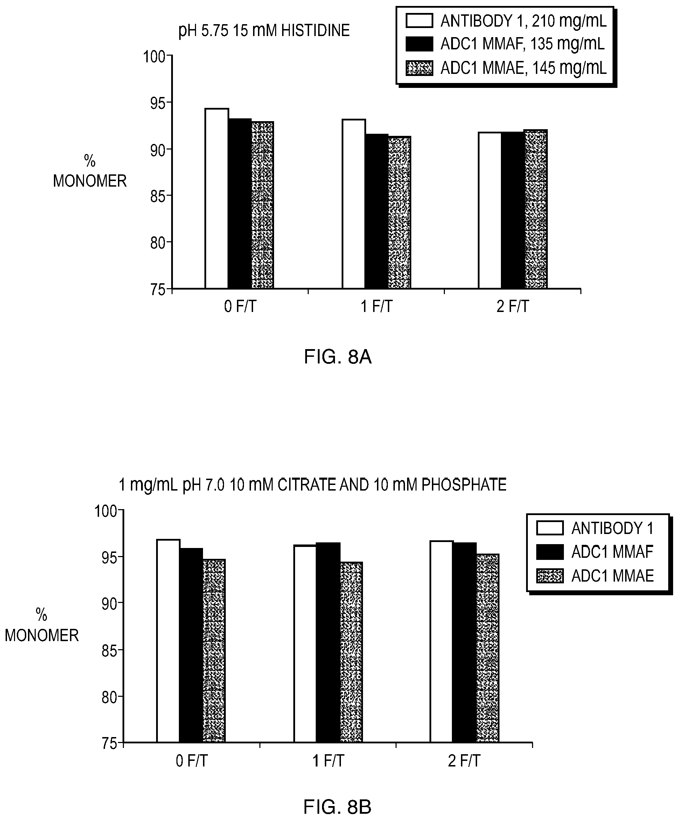

Aggregation of antibodies can be induced by freeze-thawing and elevated temperature, typical stress factors during development, production and storage. Antibody 1, ADC 1- MMAF and ADC 1- MMAE were subjected to 0, 1 or 2 freeze-thaw cycles, and characterized by spectroscopic techniques (SEC). High concentrations of antibody 1 (210 mg/ml), ADC 1- MMAF (135 mg/ml) and ADC 1-MMAE (145 mg/ml) were formulated in 15 mM histidine buffer at a pH of 5.75. The results showing the % monomer (determined by SEC) are described in Figure 8A, and suggest that there was no change in aggregation (defined as < 2% HMW species increase after 2 F/T cycles) among the three molecules In the experiments shown in Figure 8B, antibody 1, ADC 1-MMAF and ADC 1-MMAE at the same concentrations were formulated at a concentration of 1 mg/ml in 10 mM citrate and 10 mM phosphate buffer at a pH of 7.0. As shown in Figures 8A and 8B, there was no significant change in freeze/thaw stability after one or two cycles of freeze/ thaw among antibody 1, ADC 1-MMAF and ADC 1-MMAE at either high or low concentrations.

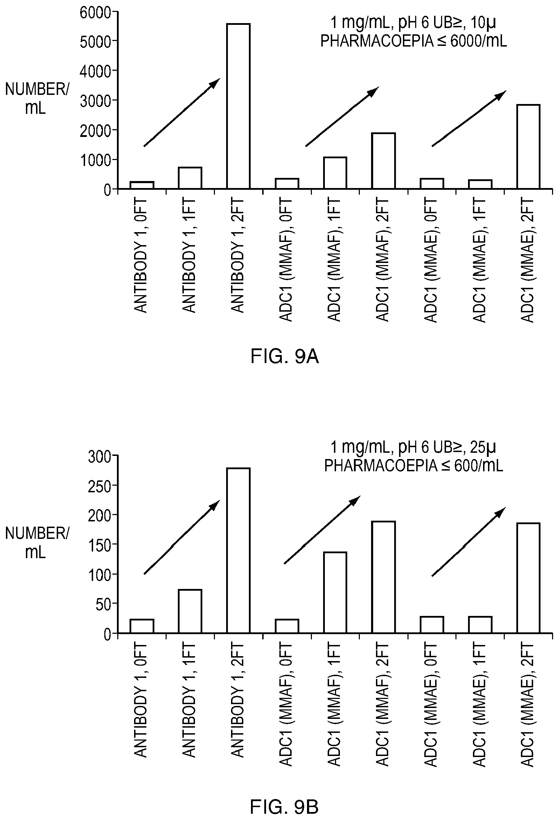

Particle formation was also tested for the three molecules at a concentration of 1 mg/ml in citrate / phosphate buffer alone at pH 6, during freeze-thawing (0, 1 or 2 freeze thaw cycles). Subvisible particles (>= 10 µm and >= 25 µm, respectively) were determined to be below pharmacopeial limits (>= 10 µm and less than or equal to 600/ Ml (>= 25 µm), respectively (Figure 9A). Microflow imaging (MFI) was also used to detect and quantify subvisible particles (less than 10 microns) (Figure 9B). Figures 9A and 9B describe an overall increase in subvisible particles for antibody 1, ADC 1-MMAF and ADC 1-MMAE over time.

### Solubility of antibody 1 versus ADC 1 with MMAF or MMAE

The solubility of antibody 1, ADC 1-MMAF and ADC 1-MMAE in a formulation containing 10 mM citrate and 10 mM phosphate buffer system at pH 6 was examined at 5 degrees C. Solubility was defined as a solution having no precipitate when formulated at at least a concentration of 50 mg/ml. The solubility of each of antibody 1, ADC 1- MMAF and ADC 1-MMAE was determined to be as follows: antibody 1 > 210 mg/mL; ADC 1-MMAF : > 135 mg/mL (Source 1); ADC 1-MMAF : > 92 mg/mL A(Source 2); ADC 1-MMAE: > 145 mg/mL (Source 1); and ADC 1-MMAE: > 116 mg/mL (Source 2). It is noted that solubility may have exceeded the above mentioned concentrations, as limited material prevented further concentration.

### Conclusion

Overall, ADC 1-MMAF and ADC 1-MMAE showed lower stability than unconjugated antibody 1, as described, for example, in the accelerated studies and the unfolding assay described herein.

### Example 2: ADC 1-MMAF Stable Lyophilized Formulation

ADC 1-MMAF is an anti-EGFR antibody drug conjugate comprising antibody 1 covalently linked to MMAF. ADC 1-MMAF was formulated as a lyophilized powder for injection upon reconstitution and packaged in glass vials. The lyophilized powder was reconstituted with 5 mL sterile water for injection (SWFI) and provided a 20 mg/mL of ADC 1-MMAF solution for injection. The drug product formulation was for single use and contained no preservative. The composition of the ADC 1-MMAF per vial (lyophilized powder) and per mL (reconstituted solution) is described in Table 1, below. The reconstituted drug product was diluted with 0.9% saline solution (Sodium Chloride Injection, USP) for dose administration by infusion.

**Table 1. Composition of ADC 1-MMAF Powder for Injection Solution, 20 mg/mL**

| **Name of Ingredients** | **Function** | **Amount (mg) per vial** | **Amount (mg/mL) reconstituted** |
|---|---|---|---|
| ADC 1-MMAF | Drug substance | 105 | 20 |
| Histidine | Buffering agent | 12 | 2.3 |
| Sucrose | Bulking agent | 368 | 70 |
| Polysorbate 80 | Surfactant | 0.53 | 0.10 |
| Hydrochloric acid ^{a} | pH adjustment | q.s. | q.s. |
| Water for Injection/ Water for injections | Vehicle | Not applicable^{b} | To total weight of 1032 mg |

| | | | |
|---|---|---|---|
| a. Used as 10% solution b. Not present after freeze drying | | | |

The formulation described in Table 1 is representative of a lyophilized formulation for an anti-EGFR ADC where the conjugate is an auristatin derivative, such as MMAF. The formulation comprises a buffer, a sugar, a surfactant and an anti-EGFR antibody drug conjugate.

Examples 3-5 describe stability studies testing the lyophilized ADC 1-MMAF formulation described in Example 2. Examples 6-8 describe stability studies testing the purified lyophilized ADC 1-MMAF formulation described in Example 2. For stability studies, ADC 1-MMAF (or ADC1-MMAFp) was stored as a lyophilizate in 20 ml colorless glass vials, closed with grey rubber stopper and a grey plastic cap. ADC 1-MMAF (or ADC1-MMAFp) was stored under the following three separate conditions: 5° C; 25° C (60% relative humidity); 40° C (75% relative humidity) for stability testing.

### Example 3: Stability of ADC 1-MMAF in a Lyophilized Formulation at 5° C

The following experiments were performed after reconstitution with SWFI at the initial time point, and after reconstitution following storage of the lyophilizate for up to eighteen months at 5° C.

### Appearance of lyophilizate and reconstituted solution not impacted by long term storage at 5° C

Appearance of the lyophilizate and of the reconstituted solution was visually assessed to confirm that the lyophilizate was practically free from visible foreign particulate matter and free from moisture in packaging material. At the initial time point, and after reconstitution following storage at 5° C for up to twelve months, the appearance of the lyophilizate complied with the foregoing criteria. After reconstitution following storage at 5° C for up to twelve months, the reconstituted solution was a colorless to slightly yellow solution, and was practically free from visible particulate matter.

### Color of solution not impacted by long term storage at 5° C

Color of the reconstituted solution (visual) was assessed using a Blue / Yellow scale (BY-scale), where the samples were tested against a reference solution and the values reported. At the initial time point, and after reconstitution following storage at 5° C for up to twelve months, all reconstituted solutions had a BY-scale measure less than or equal to 7. Thus, storage did not affect the color of the solution.

### Clarity and opalescence of solution not affected by long term storage at 5° C

Clarity and opalescence of the reconstituted solution was measured by a standard nephelometer using the light scattering principle and followed the respective Ph. Eur. or USP methods (as was also done for Examples 4 to 8). At the initial time point, and after reconstitution following storage at 5° C for up to twelve months, the appearance of the reconstituted solution was not more opalescent than reference suspension IV (per European Pharmacopeia, ph. Eur) (< = RSII).

### Protein content of solution not affected by long term storage at 5° C

Protein content of the reconstituted solution was determined. The protein concentration of the ADC was determined spectrophotometrically using a wavelength of 280 nm and the ADC1-MMAF extinction coefficient (1.43) (as was also done for Examples 4 to 8). At the initial time point, protein content of the reconstituted solution was 18.8 mg/ml. After reconstitution following storage at 5° C for three months, the protein content of the reconstituted solution was 20.2 mg/ml.

### Biological activity of solution not affected by long term storage at 5° C

A cytotoxicity assay using a human epidermoid carcinoma cell line was used to test the biological activity (%) of the reconstituted solution in comparison to an ADC1-MMAF control which had been previously confirmed to have cytotoxic activity (the control having 100% activity). At the initial time point, relative biological activity was 109%. After reconstitution following storage at 5° C for one month, relative biological activity was 105%. After reconstitution following storage at 5° C for three months, relative biological activity was 97%. After reconstitution following storage at 5° C for six months, relative biological activity was 108%. After reconstitution following storage at 5° C for nine months, relative biological activity was 105%. After reconstitution following storage at 5° C for 12 months, relative biological activity was 99%. After reconstitution following storage at 5° C for 18 months, relative biological activity was 112%.

### Size of molecules and/or molecular complexes not affected by long term storage at 5° C

Size exclusion chromatography of the reconstituted solution was performed using Size Exclusion-High Performance Liquid Chromatography (SE-HPLC). SE-HPLC determined the purity of ADC1-MMAF using size-exclusion HPLC. The macromolecules are isocratically separated during the gel filtration HPLC according to the decreasing molecular size. Purity was determined by comparing the area of ADC1-MMAF main peak to the total area of the sample chromatogram, excluding buffer-related peaks. The method is capable of resolving high molecular weight aggregates and truncated antibody species from the ADC1-MMAF main peak. This method was also used in Examples 4 to 8.

The main peak (%) was measured in addition to high molecular weight species (%) and low molecular weight species (%). At the initial time point, the main peak was 98.9%. High molecular weight species were 0.9% and low molecular weight species were 0.2%.

After reconstitution following storage at 5° C for one month, the main peak was 98.8%. High molecular weight species were 0.9% and low molecular weight species were 0.2%.

After reconstitution following storage at 5° C for three months, the main peak was 98.8%. High molecular weight species were 1.0% and low molecular weight species were 0.2%.

After reconstitution following storage at 5° C for six months, the main peak was 99.0%. High molecular weight species were 0.9% and low molecular weight species were 0.2%. \ After reconstitution following storage at 5° C for nine months, the main peak was 98.9%. High molecular weight species were 0.9% and low molecular weight species were 0.3%.

After reconstitution following storage at 5° C for 12 months, the main peak was 98.8%. High molecular weight species were 0.8% and low molecular weight species were 0.4%.

After reconstitution following storage at 5° C for 18 months, the main peak was 98.8%. High molecular weight species were 0.9% and low molecular weight species were 0.3%.

Thus, all time points showed little to no change in the main peak % and high and low molecular weight species among the initial, one, three, six, nine, twelve and eighteen month time point measurements.

### Chromatographic peak of the solution unchanged by long term storage at 5° C

Cation exchange chromatography (CEX) was performed on the reconstituted solution using High Performance Liquid Chromatography (CEX-HPLC). In cation exchange chromatography, positively charged molecules are attracted to a negatively charged solid support. The cation exchange (CEX-HPLC) chromatography assay is a charged based separation method which was used to monitor stability of ADC1-MMAF in test samples through comparison with a reference standard (ADC1-MMAF which had been previously characterized). The method is capable of resolving the main peak from acidic and basic species.

At the initial time point, and after reconstitution following storage at 5° C for up to eighteen months the appearance of the reconstituted solution was characterized by a predominant chromatographic peak pattern of the sample that conformed to that of a control which was the ADC1-MMAF which had been previously characterized. Thus, the chromatographic peak remained relatively unchanged over the course of eighteen months at 5° C.

### Purity of the solution not affected by long term storage at 5° C

Capillary gel electrophoresis (CE-SDS-R) was performed on the reconstituted solution at the initial time point, and after reconstitution following storage at 5° C for up to 18 months to determine the purity of the solution. At the initial time point, the percent purity was 97.4%. After reconstitution following storage at 5° C for one month, the percent purity was 97.6%. After reconstitution following storage at 5° C for three months, the percent purity was 96.9%. After reconstitution following storage at 5° C for six months, the percent purity was 97.1%. After reconstitution following storage at 5° C for nine months, the percent purity was 96.7%. After reconstitution following storage at 5° C for twelve months, the percent purity was 97.2%. After reconstitution following storage at 5° C for eighteen months, the percent purity was 97.3%. Thus, the purity of the reconstituted solution did not significantly change between the initial time point, and up to eighteen months after reconstitution.

### Drug to antibody ratio of the solution unchanged after long term storage at 5° C

Hydrophobic interaction chromatography (HIC) was performed on the reconstituted solution in order to determine the drug to antibody ratio (DAR) over the eighteen month time period. HIC is used to separate proteins on the basis of relative hydrophobicity. The HIC method is a measure of the hydrophobicity of ADC1-MMAF. The bound components are eluted off the ligand in order of increasing hydrophobicity by using a gradient to decrease the salt concentration of the mobile phase. For ADC1-MMAF, the first peak eluted from the column was unconjugated antibody. The remaining peaks represented an increasing number of drug-linker molecules per antibody. Thus, the number of drug-linkers per antibody was determined by peak retention time and relative peak area. This method was also used in Examples 4 to 8. At the initial time point, and after reconstitution following storage at 5° C for up to eighteen months, the drug to antibody ratio (DAR) was measured as 4.1-4.2 for all reconstituted solutions.

### Percent unconjugated antibody in the solution unchanged after long term storage at 5° C

The reconstituted solution was also analyzed to determine the percent unconjugated antibody for ADC1-MMAF using the HIC method described above for determining the DAR (and also used for Examples 4 to 8). At the initial time point, the percent was not more than 4.1. After reconstitution following storage at 5° C for one month, the percent was not more than 4.1. After reconstitution following storage at 5° C for three months, the percent was not more than 4.1. After reconstitution following storage at 5° C for six months, the percent was not more than 3.9. After reconstitution following storage at 5° C for nine months, the percent was not more than 4.0. After reconstitution following storage at 5° C for twelve months, the percent was not more than 4.0. After reconstitution following storage at 5° C for eighteen months, the percent was not more than 4.0. Thus, the percent at all times points was similar.

### Percent quenched drug linker in the solution not changed by long-term storage at 5° C

Reversed phase chromatography (RP-HPLC) was performed on the reconstituted solution to determine the percent quenched drug linker. RP-HPLC is the separation of molecules based upon their interaction with a hydrophobic matrix which is largely based on their polarity. This method determines the quantity of the quenched drug-linker, and total impurities in the solution. ADC1-MMAF was removed from the sample by precipitation with methanol followed by centrifugation. The analysis of the supernatant for the quenched drug-linker and total impurities was performed by RP-HPLC using a C-18 column with UV detection at 214 nm. This method was also used in Examples 4 to 8.

At the initial time point, the percent was not more than 0.007. After reconstitution following storage at 5° C for one month, the percent was not more than 0.005. After reconstitution following storage at 5° C for three months, the percent was not more than 0.006. After reconstitution following storage at 5° C for six months, the percent was not more than 0.005. After reconstitution following storage at 5° C for nine months, the percent was not more than 0.005. After reconstitution following storage at 5° C for twelve months, the percent was not more than 0.007. After reconstitution following storage at 5° C for eighteen months, the percent was not more than 0.005. Thus, the percent at all times points was similar for percent quenched drug linker.

### Total impurities in the solution did not change after long term storage at 5° C

The reconstituted solution was also analyzed to determine the percent of total impurities using RP-HPLC, using the method described above with respect to the percent quenched linker (also used for Examples 4 to 8). At the initial time point, the percent was not more than 0.007. After reconstitution following storage at 5° C for one month, the percent was not more than 0.005. After reconstitution following storage at 5° C for three months, the percent was not more than 0.006. After reconstitution following storage at 5° C for six months, the percent was not more than 0.005. After reconstitution following storage at 5° C for nine months, the percent was not more than 0.005. After reconstitution following storage at 5° C for twelve months, the percent was not more than 0.007. After reconstitution following storage at 5° C for eighteen months, the percent was not more than 0.005. Thus, the percent at all times points was similar for total impurities.

### Particulate contamination of the solution not affected by long term storage at 5° C

Particulate contamination was assessed by determining sub-visible particles. Acceptance criteria was met by determining the number of particles greater than 10 µm per container, with not more than 6000 (USP (US Pharmacopeia) and European Pharmacopoeia standard). At the initial time point, the number of particles per container greater than 10 µm was 33. After reconstitution following storage at 5° C for one month, the number of particles per container greater than 10 µm was 22. After reconstitution following storage at 5° C for three months, the number of particles per container greater than 10 µm was 25. After reconstitution following storage at 5° C for six months, the number of particles per container greater than 10 µm was 73. After reconstitution following storage at 5° C for twelve months, the number of particles per container greater than 10 µm was 20. Thus, all time points met with the acceptance criteria for the number of particles per container greater than 10 µm.

Acceptance criteria was also met by determining the number of particles greater than 25 µm per container, with NMT of 600 (USP (US Pharmacopeia) and European Pharmacopoeia standard). At the initial time point, the number of particles per container greater than 25 µm was 0. After reconstitution following storage at 5° C for one month, the number of particles per container greater than 25 µm was 2. After reconstitution following storage at 5° C for three months, the number of particles per container greater than 25 µm was 2. After reconstitution following storage at 5° C for six months, the number of particles per container greater than 25 µm was 10. After reconstitution following storage at 5° C for twelve months, the number of particles per container greater than 25 µm was 3. Thus, all time points met with the acceptance criteria for the number of particles per container greater than 25 µm.

### Water content of the solution not affected by long term storage at 5° C

Water content was assessed by Karl-Fischer-Titration. Karl Fischer titration uses coulometric or volumetric titration to determine trace amounts of water in a sample, reported as a percent. At the initial time point, the percent water in the reconstituted solution was 2.0%. After reconstitution following storage at 5° C for three months, the percent water in the reconstituted solution was 0.7%. After reconstitution following storage at 5° C for six months, the percent water in the reconstituted solution was 0.7%. After reconstitution following storage at 5° C for twelve months, the percent water in the reconstituted solution was 0.8%. Thus, at the initial time point, and after reconstitution following storage at 5° C for one, three, six and twelve months, the water content of the reconstituted solution was negligible.

### pH of the solution not affected by long term storage at 5° C

pH of the reconstituted solution was determined at the initial time point, and after reconstitution following storage at 5° C for one, three, six and twelve months. At the initial time point, the pH value of the reconstituted solution was 6.0. After storage at 5° C for three months, the pH value of the reconstituted solution was 5.9. After storage at 5° C for six months, the pH value of the reconstituted solution was 5.9. After storage at 5° C for twelve months, the pH value of the reconstituted solution was 5.9.

### Osmolality of the solution was not affected by long term storage at 5° C

Osmolality of the reconstituted solution was determined at the initial time point, and after reconstitution following storage at 5° C for one month by measuring the mean value of milliosmoles of solute per kilogram of solvent (mOsmol/kg). At the initial time point, osmolality was 235 mOsmol/kg. At one month, osmolality was 251 mOsmol/kg.

### Example 4: Stability of ADC 1-MMAF in a Lyophilized Formulation at 25° C

The following tests were performed after reconstitution with SWFI at the initial time point, and after reconstitution following storage of the lyophilizate for one, three and six months at 25° C (60% relative humidity):

### Appearance of lyophilizate and reconstituted solution not impacted by long term storage at 25° C

Appearance of the lyophilizate and of the reconstituted solution was visually assessed to confirm lyophilizate was practically free from visible foreign particulate matter and free from moisture in packaging material. At the initial time point, and after reconstitution following storage for one, three and six months at 25° C (60% relative humidity), the appearance of the lyophilizate complied with the foregoing criteria. After reconstitution, the reconstituted solution was a colorless to slightly yellow solution, practically free from visible particulate matter. Thus, at the initial time point, and after reconstitution following storage for one, three and six months at 25° C (60% relative humidity), the appearance of the reconstituted solution was that the solution was colorless to slightly yellow solution, practically free from visible particulate matter.

### Color of solution not impacted by long term storage at 25° C

Color of solution (visual) was assessed using a Blue/Yellow (BY)-scale where the samples were tested against a reference solution and the values were reported. At the initial time point, and after reconstitution following storage for one, three and six months at 25° C (60% relative humidity), all reconstituted solutions had a BY-scale measure less than or equal to 7. Thus, storage did not affect the color of the solution.

### Clarity and opalescence of solution not affected by long term storage at 25° C

Clarity and opalescence of the reconstituted solution was assessed. Acceptance criteria of the reconstituted solution was met if the reconstituted solution was not more opalescent than reference suspension IV (per European Pharmacopeia, ph. Eur.). At the initial time point, and after reconstitution following storage for one, three and six months at 25° C (60% relative humidity), the appearance of the reconstituted solution was not more opalescent than reference suspension IV (per European Pharmacopeia, ph. Eur.) (< = RSII).

### Biological activity of solution not affected by long term storage at 25° C

A cytotoxicity assay using a human epidermoid carcinoma cell line was used to test the biological activity (%) of the reconstituted solution in comparison to an ADC1-MMAF control which had been previously confirmed to have cytotoxic activity (the control having 100% activity. At the initial time point, relative biological activity was 109%. After reconstitution following storage at 25° C (60% relative humidity) for one month, relative biological activity was 113%. After reconstitution following storage at 25° C/ 60% relative humidity for three months, relative biological activity was 103%. After reconstitution following storage at 25° C/ 60% relative humidity for six months, relative biological activity was 109%.

### Size of molecules and/or molecular complexes not affected by long term storage at 25° C

Size exclusion chromatograph was performed by Size Exclusion-High Performance Liquid Chromatography (SE-HPLC). The main peak (%) was measured, in addition to high molecular weight species (%) and low molecular weight species (%). At the initial time point, the main peak was 98.9%. High molecular weight species were 0.9% and low molecular weight species were 0.2%. After reconstitution following storage for at 25° C (60% relative humidity) one month, the main peak was 98.8%. High molecular weight species were 1.0% and low molecular weight species were 0.2%. After reconstitution following storage at 25° C (60% relative humidity) for three months, the main peak was 98.8%. High molecular weight species were 1.0% and low molecular weight species were 0.2%. After reconstitution following storage at 25° C (60% relative humidity) for six months, the main peak was 98.9%. High molecular weight species were 0.9% and low molecular weight species were 0.2%. Thus, there was little to no change in main peak % and high and low molecular weight species among initial, one, three and six month measurements.

### Chromatographic peak of the solution unchanged by long term storage at 25° C

Cation exchange chromatography (CEX) was performed by High Performance Liquid Chromatography (CEX-HPLC). At the initial time point, and after reconstitution following storage at 25° C/ 60% relative humidity for one month, three months and six months the appearance of the reconstituted solution was characterized by a predominant chromatographic peak pattern of the sample that conformed to that of a reference standard.

### Purity of the solution not affected by long term storage at 25° C

Capillary gel electrophoresis (CE-SDS-R) was performed on the reconstituted solution at the initial time point, and after reconstitution following storage for one, three and six months at 25° C (60% relative humidity). At the initial time point, the percent purity was 97.4%. After reconstitution following storage for one month, the percent purity was 97.4%. After reconstitution following storage for three months, the percent purity was 96.9%. After reconstitution following storage for six months, the percent purity was 97.1%. Thus, the purity of the reconstituted solution did not significantly change between the initial time point, and at and after reconstitution following storage for one, three and six months at 25° C (60% relative humidity).

### Drug to antibody ratio of the solution unchanged after long term storage at 25° C

Hydrophobic interaction chromatography (HIC) was performed on the reconstituted solution. At the initial time point, and after reconstitution following storage for one, three and six months at 25° C (60% relative humidity), DAR was measured as 4.2 for all reconstituted solutions.

### Percent unconjugated antibody in the solution unchanged after long term storage

The reconstituted solution was analyzed to determine the percent unconjugated antibody. At the initial time point, the percent was not more than 4.1. After reconstitution following storage for one month at 25° C (60% relative humidity), the percent was not more than 4.1. After reconstitution following storage for three months at 25° C (60% relative humidity), the percent was not more than 4.1. After reconstitution following storage for six months at 25° C (60% relative humidity), the percent was not more than 3.9.

### Percent quenched drug linker in the solution not changed by long-term storage at 25° C

Reversed phase chromatography (RP-HPLC) was performed on the reconstituted solution to determine the percent quenched linker. At the initial time point, the percent was not more than 0.007. After reconstitution following storage for one month at 25° C (60% relative humidity), the percent was not more than 0.005. After reconstitution following storage for three months at 25° C (60% relative humidity), the percent was not more than 0.006. After reconstitution following storage for six months at 25° C (60% relative humidity), the percent was not more than 0.005. Thus, the percent at all times points was similar for percent quenched drug linker.

### Total impurities in the solution did not change after long term storage at 25° C

The reconstituted solution was analyzed to determine the percent of total impurities. At the initial time point, the percent was not more than 0.007. After reconstitution following storage for one month at 25° C (60% relative humidity), the percent was not more than 0.005. After reconstitution following storage for three months at 25° C (60% relative humidity), the percent was not more than 0.006. After reconstitution following storage for six months at 25° C (60% relative humidity), the percent was not more than 0.005. Thus, the percent at all times points was similar for total impurities.

### Particulate contamination of the solution not affected by long term storage at 25° C

Particulate contamination was assessed by determining sub-visible particles. Acceptance criteria was met by determining the number of particles greater than 10 µm per container, with no more than 6000 (USP (US Pharmacopeia) and European Pharmacopoeia standard). At the initial time point, the number of particles per container greater than 10 µm was 33. After reconstitution following storage for one month at 25° C (60% relative humidity), the number of particles per container greater than 10 µm was 18. After reconstitution following storage for three months at 25° C (60% relative humidity), the number of particles per container greater than 10 µm was 25. After reconstitution following storage for six months at 25° C (60% relative humidity), the number of particles per container greater than 10 µm was 50. Thus, all time points met with the acceptance criteria for the number of particles per container greater than 10 µm. Acceptance criteria was also met by determining the number of particles greater than 25 µm per container, with not more than 600. At the initial time point, the number of particles per container greater than 25 µm was 0. After reconstitution following storage for one month at 25° C (60% relative humidity), the number of particles per container greater than 25 µm was 2. After reconstitution following storage for three month at 25° C (60% relative humidity), the number of particles per container greater than 25 µm was 2. After reconstitution following storage for six months at 25° C (60% relative humidity), the number of particles per container greater than 25 µm was 2. Thus, all time points met with the acceptance criteria for the number of particles per container greater than 25 µm.

### Water content of the solution not affected by long term storage at 25° C

Water content was assessed by Karl-Fischer-Titration. At the initial time point, the percent water in the reconstituted solution was 2.0%. After reconstitution following storage for one month at 25° C (60% relative humidity), the percent water in the reconstituted solution was undetectable. After reconstitution following storage for three months at 25° C (60% relative humidity), the percent water in the reconstituted solution was 0.8%. After reconstitution following storage for six months at 25° C (60% relative humidity), the percent water in the reconstituted solution was 0.9%. Thus, at the initial time point, and after reconstitution following storage for one, three and six months, the water content of the reconstituted solution was negligible.

### pH of the solution not affected by long term storage at 25° C

pH of the reconstituted solution was determined at the initial time point, and after reconstitution following storage for three and six months at 25° C (60% relative humidity). At the initial time point, the pH value of the reconstituted solution was 6.0. After reconstitution following storage for three months at 25° C (60% relative humidity), the pH value of the reconstituted solution was 5.9. After reconstitution following storage for six months at 25° C (60% relative humidity), the pH value of the reconstituted solution was 5.9.

### Example 5: Stability of the ADC 1-MMAF Lyophilized Formulation at 40 °C

The following tests were performed after reconstitution of the lyophilized ADC1-MMAF formulation describe in Example 2, with SWFI at the initial time point, and after reconstitution following storage of the lyophilizate for one, three and six months at 40° C/(75% relative humidity):

### Appearance of lyophilizate and reconstituted solution not impacted by long term storage at 40° C

Appearance of the lyophilizate and of the reconstituted solution was visually assessed to confirm lyophilizate was practically free from visible foreign particulate matter and free from moisture in packaging material. At the initial time point, and after reconstitution following storage for one, three and six months at 40° C (75% relative humidity), the appearance of the lyophilizate complied with the foregoing criteria. After reconstitution, the reconstituted solution was a colorless to slightly yellow solution, practically free from visible particulate matter. At the initial time point, and after reconstitution following storage for one, three and six months at 40° C (75% relative humidity), the appearance of the reconstituted solution a colorless to slightly yellow solution, practically free from visible particulate matter.

### Color of solution not impacted by long term storage at 40° C

Color of solution (visual) was assessed using a BY-scale, where the samples were tested against a reference solution and the values were reported. At the initial time point, and after reconstitution following storage for one, three and six months at 40° C (75% relative humidity), all reconstituted solutions had a BY-scale measure less than or equal to 7. Thus, storage did not affect the color of the solution.

### Clarity and Opalescence of solution not affected by long term storage at 40° C

Clarity and Opalescence of the reconstituted solution was assessed. Acceptance criteria of the reconstituted solution was met if the reconstituted solution was not more opalescent than reference suspension IV (per European Pharmacopeia, Ph. Eur. At the initial time point, and after reconstitution following storage for one, three and six months at 40° C (75% relative humidity), the appearance of the reconstituted solution was not more opalescent than reference suspension IV (per European Pharmacopeia, Ph. Eur.) (< = RSII).

### Biological activity of solution not affected by long term storage at 40° C

A cytotoxicity assay using a human epidermoid carcinoma cell line was used to test the biological activity (%) of the reconstituted solution in comparison to an ADC1-MMAF control which had been previously confirmed to have cytotoxic activity (the control having 100% activity). At the initial time point, biological activity was 109%. After reconstitution following storage at 40° C (75% relative humidity) for one month, relative biological activity was 117%. After reconstitution following storage at 40° C (75% relative humidity) for three months, relative biological activity was 113%. After reconstitution following storage at 40° C (75% relative humidity) for six months, relative biological activity was 110%. Thus, the percent biological activity at all times points had biological activity over 100%.

### Size of molecules and/or molecular complexes not affected by long term storage at 40° C

Size exclusion chromatograph was performed by Size Exclusion-High Performance Liquid Chromatograph (SE-HPLC). The main peak (%) was measured in addition to high molecular weight species (%) and low molecular weight species (%). At the initial time point, the main peak was 98.9%. High molecular weight species were 0.9% and low molecular weight species were 0.2%. After reconstitution following storage for at 40° C (75% relative humidity) one month, the main peak was 98.8%. High molecular weight species were 1.0% and low molecular weight species were 0.2%. After reconstitution following storage at 40° C (75% relative humidity) for three months, the main peak was 98.7%. High molecular weight species were 1.1% and low molecular weight species were 0.2%. After reconstitution following storage at 40° C (75% relative humidity) for six months, the main peak was 98.8%. High molecular weight species were 1.0% and low molecular weight species were 0.2%. Thus, there was little to no change in main peak % and high and low molecular weight species among initial, one, three and six month measurements.

### Chromatographic peak of the solution unchanged by long term storage at 40° C

Cation exchange chromatography (CEX) was performed by High Performance Liquid Chromatography (CEX-HPLC). At the initial time point, and after reconstitution following storage at 40° C (75% relative humidity) for one month, three months and six months the appearance of the reconstituted solution was characterized by a predominant chromatographic peak pattern of the sample conforms to that of the reference standard.

### Purity of the solution not affected by long term storage

Capillary gel electrophoresis (CE-SDS-R) was performed on the reconstituted solution at the initial time point, and after reconstitution following storage for one, three and six months at 40° C (75% relative humidity) and was used to determine the percent purity of the solution. At the initial time point, the percent purity was 97.4%. After reconstitution following storage for one month, the percent purity was 97.5%. After reconstitution following storage for three months, the percent purity was 96.9%. After reconstitution following storage for six months, the percent purity was 97.2%. Thus, the purity of the reconstituted solution did not significantly change between the initial time point, and at and after reconstitution following storage for one, three and six months at 40° C (75% relative humidity).

### Drug to antibody ratio of the solution unchanged after long term storage at 40° C

Hydrophobic interaction chromatography (HIC) was performed on the reconstituted solution. At the initial time point, and after reconstitution following storage for one, three and six months at 40° C (75% relative humidity), DAR was measured as 4.2 for all reconstituted solutions.

### Percent unconjugated antibody in the solution unchanged after long term storage at 40° C

At the initial time point, the percent was not more than 4.1. After reconstitution following storage for one month at 40° C (75% relative humidity), the percent was not more than 4.1. After reconstitution following storage for three months at 40° C (75% relative humidity), the percent was not more than 4.1. After reconstitution following storage for six months at 40° C (75% relative humidity), the percent was not more than 3.9. Thus, the percent at all times points was similar.

### Percent quenched drug linker in the solution not changed by long term storage at 40° C

Reversed phase chromatography (RP-HPLC) was performed on the reconstituted solution to determine the percent quenched drug linker. At the initial time point, the percent was not more than 0.007. After reconstitution following storage for one month at 40° C (75% relative humidity), the percent was not more than 0.005. After reconstitution following storage for three months at 40° C (75% relative humidity), the percent was not more than 0.006. After reconstitution following storage for six months at 40° C (75% relative humidity), the percent was not more than 0.005. Thus, the percent at all times points was similar for percent quenched drug linker.

### Total impurities in the solution did not change after long term storage at 40° C

The reconstituted solution was analyzed to determine the percent of total impurities using RP-HPLC. At the initial time point, the percent was not more than 0.007. After reconstitution following storage for one month at 40° C (75% relative humidity), the percent was not more than 0.005. After reconstitution following storage for three months at 40° C (75% relative humidity), the percent was not more than 0.006. After reconstitution following storage for six months at 40° C (75% relative humidity), the percent was not more than 0.005. Thus, the percent at all times points was similar for total impurities.

### Particulate contamination of the solution not affected by long term storage at 40° C

Particulate contamination was assessed by determining sub-visible particles. Acceptance criteria was met by determining the number of particles greater than 10 µm per container, with not more than 6000 (USP (US Pharmacopeia) and European Pharmacopoeia standard). At the initial time point, the number of particles per container greater than 10 µm was 33. After reconstitution following storage for one month at 40° C (75% relative humidity), the number of particles per container greater than 10 µm was 5. After reconstitution following storage for three months at 40° C (75% relative humidity), the number of particles per container greater than 10 µm was 12. After reconstitution following storage for six months at 40° C (75% relative humidity), the number of particles per container greater than 10 µm was 32. Thus, all time points met with the acceptance criteria for the number of particles per container greater than 10 µm. Acceptance criteria was also met by determining the number of particles greater than 25 µm per container, with not more than 600. At the initial time point, the number of particles per container greater than 25 µm was 0. After reconstitution following storage for one month at 40° C (75% relative humidity), the number of particles per container greater than 25 µm was 0. After reconstitution following storage for three month at 40° C (75% relative humidity), the number of particles per container greater than 25 µm was 0. After reconstitution following storage for six months at 40° C (75% relative humidity), the number of particles per container greater than 25 µm was 2. Thus, all time points met with the acceptance criteria for the number of particles per container greater than 25 µm.

### Water content of the solution not affected by long term storage at 40° C

Water content was assessed by Karl-Fischer-Titration. Karl Fischer titration uses coulometric or volumetric titration to determine trace amounts of water in a sample, reported as a percent. At the initial time point, the percent water in the reconstituted solution was 2.0%. After reconstitution following storage for one month at 40° C (75% relative humidity), the percent water in the reconstituted solution was undetectable. After reconstitution following storage for three months at 40° C (75% relative humidity), the percent water in the reconstituted solution was 0.9%. After reconstitution following storage for six months at 40° C (75% relative humidity), the percent water in the reconstituted solution was 1.0%. Thus, at the initial time point, and after reconstitution following storage for one, three and six months, the water content of the reconstituted solution was negligible.

### pH of the solution not affected by long term storage at 40° C

pH of the reconstituted solution was determined at the initial time point, and after reconstitution following storage for one, three and six months at 40° C (75% relative humidity). At the initial time point, the pH value of the reconstituted solution was 6.0. After reconstitution following storage for three months at 40° C (75% relative humidity), the pH value of the reconstituted solution was 5.9. After reconstitution following storage for six months at 40° C (75% relative humidity), the pH value of the reconstituted solution was 5.9.

Purification of ADC 1-MMAF was performed in accordance with the batch purification method described herein, and disclosed in U.S. Provisional Application No. 61/792834 and U.S. Application No. 14/210,602, filed on March 14, 2014, the contents of which are incorporated by reference herein. The average DAR for ADC mixtures comprising ADC1-MMAFp described in Examples 6 to 8 was about 3.0.

Stability experiments described in Examples 6 to 8 were performed after reconstitution with SWFI at an initial time point, and after reconstitution following storage of the lyophilizate comprising ADC1-MMAFp for three months at 5° C, and for one and three months at both 25° C (60% relative humidity) and 40° C/(75% relative humidity).

### Example 6: Stability of Purified ADC 1-MMAF (ADCI-MMAFp) in a Lyophilized Formulation at 5° C

The following tests were performed after reconstitution of the lyophilized ADC1-MMAFp formulation described in Example 2, with SWFI at the initial time point, and after reconstitution of the formulation following storage of the lyophilizate for up to three months at 5° C.

### Appearance of lyophilizate and reconstituted solution not impacted by long term storage at 5° C

Appearance of the lyophilizate and of the reconstituted solution was visually assessed to confirm that the lyophilizate was practically free from visible foreign particulate matter and free from moisture in packaging material. At the initial time point, and after reconstitution following storage at 5° C for three months, the appearance of the lyophilizate complied with the foregoing criteria. After reconstitution, the reconstituted solution was a colorless to slightly yellow solution, and was practically free from visible particulate matter.

### Color of solution not impacted by long term storage at 5° C

Color of the reconstituted solution (visual) was assessed using a Blue / Yellow scale (BY-scale), where the samples were tested against a reference solution and the values reported. At the initial time point, and after reconstitution following storage at 5° C for three months, all reconstituted solutions had a BY-scale measure less than or equal to 7. Thus, storage of the ADC1-MMAFp formulation did not affect the color of the solution.

### Clarity and opalescence of solution not affected by long term storage at 5° C

Clarity and opalescence of the reconstituted solution was assessed. At the initial time point, and after reconstitution following storage at 5° C for three months, the appearance of the reconstituted solution was not more opalescent than reference suspension IV (per European Pharmacopeia, ph. Eur) (< = RSII).

### Biological activity of solution not affected by long term storage at 5° C

A cytotoxicity assay using a human epidermoid carcinoma cell line was used to test the biological activity (%) of the reconstituted solution in comparison to an ADC1-MMAF control which had been previously confirmed to have cytotoxic activity (the control having 100% activity). At the initial time point, relative biological activity was 102%. After reconstitution following storage at 5° C in a lyophilized form for three months, relative biological activity was 98%.

### Size of molecules and/or molecular complexes not affected by long term storage at 5° C

Size exclusion chromatography of the reconstituted solution was performed using Size Exclusion-High Performance Liquid Chromatography (SE-HPLC). The main peak (%) was measured in addition to high molecular weight species (%) and low molecular weight species (%). At the initial time point, the main peak was 99.5%. High molecular weight species were 0.2% and low molecular weight species were 0.2%. After reconstitution following storage at 5° C in a lyophilized form for three months, the main peak was 99.6%. High molecular weight species were 0.2% and low molecular weight species were 0.2% at both initially and when reconstituted following three months of storage in lyophilized form.

Thus, all time points showed little to no change in the main peak % and high and low molecular weight species among the initial and three month time point measurements.

### Chromatographic peak of the solution unchanged by long term storage at 5° C

Cation exchange chromatography (CEX) was performed on the reconstituted solution using High Performance Liquid Chromatography (CEX-HPLC). In cation exchange chromatography, positively charged molecules are attracted to a negatively charged solid support. At the initial time point, and after reconstitution following storage at 5° C in lyophilized form for three months the appearance of the reconstituted solution was characterized by a predominant chromatographic peak pattern of the sample that conformed to that of a control which was the ADC1-MMAF which had been previously characterized. Thus, the chromatographic peak remained relatively unchanged over the course of three months at 5° C.

### Purity of the solution not affected by long term storage at 5° C

Capillary gel electrophoresis (CE-SDS-R) was performed on the reconstituted solution at the initial time point, and after reconstitution following storage at 5° C for three months to determine the purity of the solution. At the initial time point, the percent purity was 97.4%. After reconstitution following storage at 5° C in lyophilized form for three months, the percent purity was 97.6%. Thus, the purity of the reconstituted solution did not significantly change between the initial time point and three months of storage.

### Drug to antibody ratio (DAR) of ADC1-MMAF remained unchanged after storage at 5° C

Hydrophobic interaction chromatography (HIC) was performed on the reconstituted solution in order to determine the drug to antibody ratio (DAR) over a three month storage period. HIC is used to separate proteins on the basis of relative hydrophobicity. At the initial time point, and after reconstitution following storage in lyophilized form at 5° C for three months, the average drug to antibody ratio (DAR) for ADC1-MMAF was measured as 3.0 and 2.9, respectively.

### Percent unconjugated antibody 1 in the solution unchanged after storage at 5° C

The reconstituted solution was also analyzed to determine the percent unconjugated antibody for ADC1-MMAF. At the initial time point, the percent was not more than 7.6. After reconstitution following storage at 5° C in lyophilized form for three months, the percent was not more than 7.6. Thus, the percent of unconjugated antibody 1 remained the same over the three month period.

### Percent quenched drug linker in the solution unchanged by storage at 5° C

Reversed phase chromatography (RP-HPLC) was performed on the reconstituted solution to determine the percent quenched drug linker. The percent of quenched drug linker was not detected both initially and after reconstitution following storage at 5° C for three months in lyophilized form (the practical limit of detection was 0.001%).

### Total impurities in the solution unchanged after storage at 5° C

Reconstituted solution was also analyzed to determine the percent of total impurities using RP-HPLC. The percent of total impurities was not detectable both initially and after reconstitution following storage at 5° C for three months in lyophilized form (the practical limit of detection was 0.001%; practical limit of quantitation was 0.003%).

### Particulate contamination of the solution not affected by storage at 5° C

Particulate contamination was assessed by determining sub-visible particles using two different acceptance criteria.

Acceptance criteria was met by determining the number of particles greater than 10 µm per container, with not more than 6000 (USP (US Pharmacopeia) and European Pharmacopoeia standard). At the initial time point, the number of particles per container greater than 10 µm was 13. After reconstitution following storage at 5° C for three months in lyophilized form, the number of particles per container greater than 10 µm was 20. Thus, both time points met with the acceptance criteria for the number of particles per container greater than 10 µm.

Acceptance criteria was also met by determining the number of particles greater than 25 µm per container, with NMT of 600 (USP (US Pharmacopeia) and European Pharmacopoeia standard). At the initial time point, the number of particles per container greater than 25 µm was 0. After reconstitution following storage at 5° C for three months, the number of particles per container greater than 25 µm was 0. Thus, both time points met with the acceptance criteria for the number of particles per container greater than 25 µm.

### Water content of the solution not affected by storage at 5° C

Water content was assessed by Karl-Fischer-Titration. Karl Fischer titration uses coulometric or volumetric titration to determine trace amounts of water in a sample, reported as a percent. At the initial time point, the percent water in the reconstituted solution was 0.8%. After reconstitution following storage at 5° C for three months in lyophilized form, the percent water in the reconstituted solution was 0.8%. Thus, at the initial time point and after reconstitution following storage at 5° C for three months, the water content of the reconstituted solution was negligible.

### pH of the solution not affected by storage at 5° C

pH of the reconstituted solution was determined at the initial time point, and after reconstitution following storage at 5° C for three months. At the initial time point, the pH value of the reconstituted solution was 5.9. After storage at 5° C for three months, the pH value of the reconstituted solution was 5.9. Thus, storage of the ADC1-MMAFp composition for three months at 5° C did not affect pH.

### Example 7: Stability of ADC 1-MMAFp in a Lyophilized Formulation at 25° C

The following experiments were performed after reconstitution of the lyophilized ADC1-MMAFp formulation (see Example 2) with SWFI at the initial time point, and after reconstitution following storage of the lyophilizate for one and three months at 25° C (60% relative humidity).

### Appearance of lyophilizate and reconstituted solution not impacted by storage at 25° C

Appearance of the lyophilizate and of the reconstituted solution was visually assessed to confirm lyophilizate was practically free from visible foreign particulate matter and free from moisture in packaging material. At the initial time point, and after reconstitution following storage for one and three months at 25° C, the appearance of the lyophilizate complied with the foregoing criteria. After reconstitution, the reconstituted solution was a colorless to slightly yellow solution, practically free from visible particulate matter. Thus, at the initial time point, and after reconstitution following storage for one and three months at 25° C, the appearance of the reconstituted solution was that the solution was colorless to slightly yellow solution, and practically free from visible particulate matter.

### Color of solution not impacted by storage at 25° C

Color of solution (visual) was assessed using a Blue/Yellow (BY)-scale where the samples were tested against a reference solution and the values were reported. At the initial time point, and after reconstitution following storage for one and three months at 25° C, all reconstituted solutions had a BY-scale measure less than or equal to 7. Thus, storage did not affect the color of the solution.

### Clarity and opalescence of solution not affected by storage at 25° C

Clarity and opalescence of the reconstituted solution was assessed. Acceptance criteria of the reconstituted solution was met if the reconstituted solution was not more opalescent than reference suspension IV (per European Pharmacopeia, ph. Eur.).At the initial time point, and after reconstitution following storage for one and three months at 25° C, the appearance of the reconstituted solution was not more opalescent than reference suspension IV (per European Pharmacopeia, ph. Eur.) (< = RSII).

### Biological activity of solution not affected by storage at 25° C

A cytotoxicity assay using a human epidermoid carcinoma cell line was used to test the biological activity (%) of the reconstituted solution in comparison to an ADC1-MMAF control which had been previously confirmed to have cytotoxic activity (the control having 100% activity. At the initial time point, relative biological activity was 102%. After reconstitution following storage at 25° C for one month, relative biological activity was 101%. After reconstitution following storage at 25° C for three months, relative biological activity was 97%.

### Size of molecules and/or molecular complexes not affected by storage at 25° C

Size exclusion chromatograph was performed by Size Exclusion-High Performance Liquid Chromatography (SE-HPLC). The main peak (%) was measured, in addition to high molecular weight species (%) and low molecular weight species (%). At the initial time point, the main peak was 99.5%. High molecular weight species were 0.2% and low molecular weight species were 0.2%. After reconstitution following storage for at 25° C one month, the main peak was 99.5%. High molecular weight species were 0.3% and low molecular weight species were 0.2%. After reconstitution following storage at 25° C for three months, the main peak was 99.6%. High molecular weight species were 0.3% and low molecular weight species were 0.2%. Thus, there was little to no change in main peak % and high and low molecular weight species among initial, one and three month measurements.

### Chromatographic peak of the solution unchanged by storage at 25° C

Cation exchange chromatography (CEX) was performed by High Performance Liquid Chromatography (CEX-HPLC). At the initial time point, and after reconstitution following storage at 25° C for one month and three months the appearance of the reconstituted solution was characterized by a predominant chromatographic peak pattern of the sample that conformed to that of a reference standard.

### Purity of the solution not affected by storage at 25° C

Capillary gel electrophoresis (CE-SDS-R) was performed on the reconstituted solution at the initial time point, and after reconstitution following storage for one and three at 25° C. At the initial time point, the percent purity was 97.4%. After reconstitution following storage for one month, the percent purity was 97.5%. After reconstitution following storage for three months, the percent purity was 97.6%. Thus, the purity of the reconstituted solution did not significantly change between the initial time point, and after reconstitution following storage for one and three months at 25° C.

### Drug to antibody ratio (DAR) of ADC1-MMAFp in the solution remained unchanged after storage at 25° C

Hydrophobic interaction chromatography (HIC) was performed on the reconstituted solution. At the initial time point, DAR was measured as 3.0. After reconstitution following storage for one month at 25° C, DAR was measured as 3.0. After reconstitution following storage for three months at 25° C, DAR was measured as 2.9. Thus, DAR of ADC1-MMAFp did not significantly change between the initial time point, and after reconstitution following storage for one and three months at 25° C.

### Percent unconjugated antibody 1 in the solution unchanged after storage at 25° C

The reconstituted solution was analyzed to determine the percent unconjugated antibody 1. At the initial time point, the percent was not more than 7.6. After reconstitution following storage for one month at 25° C, the percent was not more than 7.6. After reconstitution following storage for three months at 25° C, the percent was not more than 7.6.

### Percent quenched drug linker in the solution not changed by storage at 25° C

Reversed phase chromatography (RP-HPLC) was performed on the reconstituted solution to determine the percent quenched linker. At the initial time point, the one month time point, and the three month time point, the percent of quenched linked was not detectable (practical limit of detection was 0.001%; practical limit of quantitation was 0.003%).

### Total impurities in the solution did not change after storage at 25° C

The reconstituted solution was analyzed to determine the percent of total impurities. At the initial time point, the percent was not detected. After reconstitution following storage for one month at 25° C, the percent of impurities was less than 0.003. After reconstitution following storage for three months at 25° C, the percent was not detected (practical limit of detection was 0.001%; practical limit of quantitation was 0.003%). Thus, the percent at all times points was similar for total impurities.

### Particulate contamination of the solution not affected by storage at 25° C

Particulate contamination was assessed by determining sub-visible particles according to two acceptance criteria.

Acceptance criteria was met by determining the number of particles greater than 10 µm per container, with no more than 6000 (USP (US Pharmacopeia) and European Pharmacopoeia standard). At the initial time point, the number of particles per container greater than 10 µm was 13. After reconstitution following storage for one month at 25° C, the number of particles per container greater than 10 µm was 12. After reconstitution following storage for three months at 25° C, the number of particles per container greater than 10 µm was 17. Thus, all time points met with the acceptance criteria for the number of particles per container greater than 10 µm. Acceptance criteria was also met by determining the number of particles greater than 25 µm per container, with not more than 600. At the initial time point, the number of particles per container greater than 25 µm was 0. After reconstitution following storage for one month at 25° C, the number of particles per container greater than 25 µm was 0. After reconstitution following storage for three month at 25° C, the number of particles per container greater than 25 µm was 7. Thus, all time points met with the acceptance criteria for the number of particles per container greater than 25 µm.

### Water content of the solution not affected by storage at 25° C

Water content was assessed by Karl-Fischer-Titration. At the initial time point, the percent water in the reconstituted solution was 0.8%. After reconstitution following storage for one month at 25° C, the percent water in the reconstituted solution was 0.8%. After reconstitution following storage for three months at 25° C, the percent water in the reconstituted solution was 0.8%. Thus, at the initial time point, and after reconstitution following storage for one and three months, the water content of the reconstituted solution was negligible.

### pH of the solution not affected by storage at 25° C

pH of the reconstituted solution was determined at the initial time point, and after reconstitution following storage for one and three months at 25° C. At the initial time point, the pH value of the reconstituted solution was 5.9. After reconstitution following storage for three months at 25° C, the pH value of the reconstituted solution was 5.9. After reconstitution following storage for six months at 25° C, the pH value of the reconstituted solution was 5.9.

### Example 8: Stability of ADC 1-MMAFp Lyophilized Formulation at 40 °C

The following tests were performed after reconstitution of the lyophilized ADC1-MMAFp formulation describe in Example 2, with SWFI at the initial time point, and after reconstitution following storage of the lyophilizate for one and three months at 40° C/(75% relative humidity).

### Appearance of lyophilizate and reconstituted solution not impacted by storage at 40° C

Appearance of the lyophilizate and of the reconstituted solution was visually assessed to confirm lyophilizate was practically free from visible foreign particulate matter and free from moisture in packaging material. At the initial time point, and after reconstitution following storage for one and three months at 40° C, the appearance of the lyophilizate complied with the foregoing criteria. After reconstitution, the reconstituted solution was a colorless to slightly yellow solution, practically free from visible particulate matter. At the initial time point, and after reconstitution following storage for one and three months at 40° C, the appearance of the reconstituted solution was that the solution was a colorless to slightly yellow solution, practically free from visible particulate matter.

### Color of solution not impacted by storage at 40° C

Color of solution (visual) was assessed using a BY-scale, where the samples were tested against a reference solution and the values were reported. At the initial time point, and after reconstitution following storage for one and three months at 40° C, all reconstituted solutions had a BY-scale measure less than or equal to 7. Thus, storage did not affect the color of the solution.

### Clarity and Opalescence of solution not affected by storage at 40° C

Clarity and opalescence of the reconstituted solution was assessed. Acceptance criteria of the reconstituted solution was met if the reconstituted solution was not more opalescent than reference suspension IV (per European Pharmacopeia, Ph. Eur. At the initial time point, and after reconstitution following storage for one and three months at 40° C, the appearance of the reconstituted solution was not more opalescent than reference suspension IV (per European Pharmacopeia, Ph. Eur.) (< = RSII).

### Biological activity of solution not affected by storage at 40° C

A cytotoxicity assay using a human epidermoid carcinoma cell line was used to test the biological activity (%) of the reconstituted solution in comparison to an ADC1-MMAF control which had been previously confirmed to have cytotoxic activity (the control having 100% activity). At the initial time point, biological activity was 102%. After reconstitution following storage at 40° C for one month, relative biological activity was 101%. After reconstitution following storage at 40° C for three months, relative biological activity was 105%. Thus, the percent biological activity at all times points had biological activity over 100% in the formulation of Example 2.

### Size of molecules and molecular complexes not affected by storage at 40° C

Size exclusion chromatograph was performed by Size Exclusion-High Performance Liquid Chromatograph (SE-HPLC). The main peak (%) was measured in addition to high molecular weight species (%) and low molecular weight species (%). At the initial time point, the main peak was 99.5%. High molecular weight species were 0.2% and low molecular weight species were 0.2%. After reconstitution following storage for at 40° C one month, the main peak was 99.5%. High molecular weight species were 0.3% and low molecular weight species were 0.2%. After reconstitution following storage at 40° C for three months, the main peak was 99.5%. High molecular weight species were 0.3% and low molecular weight species were 0.2%. Thus, there was little to no change in main peak % and high and low molecular weight species among initial, one and three month measurements.

### Chromatographic peak of the solution unchanged by storage at 40° C

Cation exchange chromatography (CEX) was performed by High Performance Liquid Chromatography (CEX-HPLC). At the initial time point, and after reconstitution following storage at 40° C for one month and three months the appearance of the reconstituted solution was characterized by a predominant chromatographic peak pattern of the sample conforms to that of the reference standard.

### Purity of the solution not affected by storage at 40° C

Capillary gel electrophoresis (CE-SDS-R) was performed on the reconstituted solution at the initial time point, and after reconstitution following storage for one, three and six months at 40° C and was used to determine the percent purity of the solution. At the initial time point, the percent purity was 97.4%. After reconstitution following storage for one month, the percent purity was 97.5%. After reconstitution following storage for three months, the percent purity was 97.6%. Thus, the purity of the reconstituted solution did not significantly change between the initial time point, and at and after reconstitution following storage for one and three months at 40° C.

### Drug to antibody ratio (DAR) of ADC1-MMAFp unchanged after storage at 40° C

Hydrophobic interaction chromatography (HIC) was performed on the reconstituted solution. At the initial time point, DAR was measured as 3.0. After reconstitution following storage for one month at 40° C, DAR was measured as 3.0. After reconstitution following storage for three months at 40° C, DAR was measured as 2.9. Thus, DAR did not significantly change between the initial time point, and after reconstitution following storage for one and three months at 40° C.

### Percent unconjugated antibody 1 in the solution unchanged after storage at 40° C

At the initial time point, the percent of unconjugated antibody 1 was not more than 7.6. After reconstitution following storage for one month at 40° C, the percent of unconjugated antibody 1 was not more than 7.6. After reconstitution following storage for three months at 40° C, the percent of unconjugated antibody 1 was not more than 7.6. Thus, the percent at all times points was similar.

### Percent quenched drug linker in the solution not changed by storage at 40° C

Reversed phase chromatography (RP-HPLC) was performed on the reconstituted solution to determine the percent quenched drug linker. At the initial time point, the percent was not detected. After reconstitution following storage for one month at 40° C, the percent was not detected. After reconstitution following storage for three months at 40° C, the percent was not detected. Thus, the percent quenched drug linker at all times points was not detected.

### Total impurities in the solution did not change after storage at 40° C

The reconstituted solution was analyzed to determine the percent of total impurities using RP-HPLC. At the initial time point, the percent was not detected. After reconstitution following storage for one month at 40° C, the percent was less than 0.003. After reconstitution following storage for three months at 40° C, the percent was not detected. Thus, the percent at all times points was similar for total impurities.

### Particulate contamination of the solution not affected by storage at 40° C

Particulate contamination was assessed by determining sub-visible particles according to two acceptance criteria.

Acceptance criteria was met by determining the number of particles greater than 10 µm per container, with not more than 6000 (USP (US Pharmacopeia) and European Pharmacopoeia standard). At the initial time point, the number of particles per container greater than 10 µm was 13. After reconstitution following storage for one month at 40° C, the number of particles per container greater than 10 µm was 5. After reconstitution following storage for three months at 40° C, the number of particles per container greater than 10 µm was 8. Thus, all time points met with the acceptance criteria for the number of particles per container greater than 10 µm.

Acceptance criteria was also met by determining the number of particles greater than 25 µm per container, with not more than 600. At the initial time point, the number of particles per container greater than 25 µm was 0. After reconstitution following storage for one month at 40° C, the number of particles per container greater than 25 µm was 0. After reconstitution following storage for three month at 40° C, the number of particles per container greater than 25 µm was 2. Thus, all time points met with the acceptance criteria for the number of particles per container greater than 25 µm.

### Water content of the solution not affected by storage at 40° C

Water content was assessed by Karl-Fischer-Titration. Karl Fischer titration uses coulometric or volumetric titration to determine trace amounts of water in a sample, reported as a percent. At the initial time point, the percent water in the reconstituted solution was 0.8%. After reconstitution following storage for one month at 40° C, the percent water in the reconstituted solution was 0.8%. After reconstitution following storage for three months at 40° C, the percent water in the reconstituted solution was 0.8%. Thus, at the initial time point, and after reconstitution following storage for one and three months, the water content of the reconstituted solution was negligible.

### pH of the solution not affected by storage at 40° C

pH of the reconstituted solution was determined at the initial time point, and after reconstitution following storage for one and three months at 40° C. At the initial time point, the pH value of the reconstituted solution was 5.9. After reconstitution following storage for three months at 40° C, the pH value of the reconstituted solution was 5.9. After reconstitution following storage for six months at 40° C, the pH value of the reconstituted solution was 5.9.

### Example 9: Large Scale Manufacturing of Lyophilized ADC 1-MMAF Formulation

For the preparation of the bulk solution, histidine was dissolved in water for injection, pH was adjusted with hydrochloric acid 10% w/w, and water for injection was added to the final weight. The formulation buffer excipients (sucrose and polysorbate 80) were weighed and dissolved in 15 mM histidine solution. The resulting solution was then examined for appearance.

ADC 1-MMAF was thawed at 30°C in a water bath, examined for appearance, pooled and weighed. 15 mM histidine buffer, polysorbate 80, and sucrose were added to ADC 1-MMAF. The appearance of the solution was examined and its pH and density determined, followed by sterile filtration. The solution was then lyophilized under controlled conditions.

The critical in-process tests that were carried out during the manufacture of ADC 1-MMAF Powder for Injection Solution, 20 mg/mL are listed in Table 2, below.

**Table 2. In-Process Control Tests and Acceptance Limits**

| **Operation** | **Test** | **Acceptance Limit** |
|---|---|---|
| First filtration | Filter Integrity | Forward flow test, diffusion rate or bubble point must comply with the filter specification |
| Sterilization by filtration | Bioburden prior to second filtration | < 10 CFU/100 mL |
| | Filter Integrity | Forward flow test, diffusion rate or bubble point must comply with the filter specification |

The amount of ADC 1-MMAF and buffer solution used for a typical batch of ADC 1-MMAF solution for lyophilization are shown in Table 3, below. A list of components and the amounts used to manufacture the formulation buffer are described in Table 4 and Table 5.

**Table 3. Batch Formula for ADC 1-MMAF Bulk Drug Product Solution**

| | |
|---|---|
| **Dosage Strength** | 20 mg/mL |
| **Batch Size** | 27 L^{a} |

| **Component** | **Amount (kg) per Batch** |
|---|---|
| ADC 1-MMAF Drug Substance | 15.57 ^{b, c, d} |
| Formulation Buffer ^{e} | 12.29 ^{d} |

| | |
|---|---|
| a. Density of bulk solution: 1.032 g/mL b. Equivalent to 0.540 kg of protein at 35 g protein/L drug substance concentrate; density of drug substance concentrate: 1.0089 g/mL c. Drug substance concentrate contains 15 mM histidine d. Amounts vary depending on actual protein concentration of drug substance concentrate e. The batch formula for this material is presented in Table 4 | |

**Table 4. Batch Formula for Formulation Buffer Solution**

| | |
|---|---|
| **Batch Size** | 11.58 L^{a} |

| **Component ^{b}** | **Amount (g) per Batch** |
|---|---|
| Sucrose | 1890^{b} |
| Polysorbate 80 | 2.7^{c} |
| 15 mM Histidine Solution ^{d} | ad 12290 |

| | |
|---|---|
| Abbreviation: ad = to total weight of a. Density of solution: 1.0616 g/mL b. Amount corresponds to a final concentration in the bulk product solution of 70 g/L c. Amount corresponds to a final concentration in the bulk product solution of 0.10 g/L d. The batch formula for this material is presented in Table 6 . | |

**Table 5. Batch Formula for 15 mM Histidine Solution**

| | |
|---|---|
| **Batch Size** | 15 L^{a} |

| **Component** | **Amount (g) per Batch** |
|---|---|
| Histidine | 34.95 |
| Hydrochloric Acid 10% (w/w)^{b} | q.s |
| Water for Injection | ad 14999 |

| | |
|---|---|
| Abbreviation: ad = to total weight of a. Density of solution: 0.9993 g/mL b. Used for pH adjustment | |

It is understood that the examples and embodiments described herein are for illustrative purposes only and that various modifications or changes in light thereof will be suggested to persons skilled in the art and are to be included within the spirit and purview of the appended claims. All publications, patents, and patent applications cited herein are hereby incorporated by reference in their entirety for all purposes.

**SUMMARY OF SEQUENCES**

| | | |
|---|---|---|
| SEQ ID NO: 1 | Nucleic acid | Variable heavy chain sequence of antibody 2 (murine anti-EGFR Ab) |
| SEQ ID NO: 2 | Protein | Variable heavy chain sequence of antibody 2 (murine anti-EGFR Ab) |
| SEQ ID NO: 3 | Nucleic acid | Variable light chain sequence of antibody 2 (murine anti-EGFR Ab) |
| SEQ ID NO: 4 | Protein | Variable light chain sequence of antibody 2 (murine anti-EGFR Ab). |
| SEQ ID NO: 5 | Protein | CDR1 of the variable heavy chain sequence SEQ ID NO:2 |
| SEQ ID NO: 6 | Protein | CDR2 of the variable heavy chain sequence SEQ ID NO:2 |
| SEQ ID NO: 7 | Protein | CDR3 of the variable heavy chain sequence SEQ ID NO:2 |
| SEQ ID NO: 8 | Protein | CDR1 of the variable light chain sequence SEQ ID NO:2 |
| SEQ ID NO: 9 | Protein | CDR2 of the variable light chain sequence SEQ ID NO:2 |
| SEQ ID NO: 10 | Protein | CDR3 of the variable light chain sequence SEQ ID NO:2 |
| SEQ ID NO: 11 | Protein | Variable heavy chain sequence of antibody 2 (murine anti-EGFR Ab) without signal peptide |
| SEQ ID NO: 12 | Protein | Variable light chain sequence of antibody 2 (murine anti-EGFR Ab) without signal peptide |
| SEQ ID NO: 13 | Protein | Variable heavy chain sequence of antibody 1 (humanized anti-EGFR Ab) |
| SEQ ID NO: 14 | Protein | Constant heavy chain sequence of antibody 1 (humanized anti-EGFR Ab) |
| SEQ ID NO: 15 | Protein | CDR1 of the variable heavy chain sequence SEQ ID NO:13 |
| SEQ ID NO: 16 | Protein | CDR2 of the variable heavy chain sequence SEQ ID NO:13 |
| SEQ ID NO: 17 | Protein | CDR3 of the variable heavy chain sequence SEQ ID NO:13 |
| SEQ ID NO: 18 | Protein | Variable light chain sequence of antibody 1 (humanized anti-EGFR Ab) |
| SEQ ID NO: 19 | Protein | Constant light chain sequence of antibody 1 (humanized anti-EGFR Ab) |
| SEQ ID NO: 20 | Protein | CDR1 of the variable light chain sequence SEQ ID NO:18 |
| SEQ ID NO: 21 | Protein | CDR2 of the variable light chain sequence SEQ ID NO:18 |
| SEQ ID NO: 22 | Protein | CDR3 of the variable light chain sequence SEQ ID NO:18 |

Preferred embodiments of the present invention are described below and are referred to as embodiments E1 to E40.
E1. A formulation comprising an anti-Epidermal Growth Factor Receptor (EGFR) antibody drug conjugate (ADC), a sugar, histidine, and a surfactant, wherein said formulation has a pH of about 5 - 7, and wherein said anti-EGFR ADC comprises an anti-EGFR antibody, or antigen-binding portion thereof, conjugated to an auristatin.
E2. The formulation of E 1, wherein the auristatin is Monomethylauristatin F (MMAF).
E3. The formulation of E2, wherein the MMAF is conjugated to the antibody with a malcimidocaproyl linker.
E4. The formulation of any one of E1-3, wherein the surfactant is a polysorbate or a poloxamer.
E5. The formulation of E4, wherein the formulation comprises 0.05-0.15 mg/ml of the polysorbate.
E6. The formulation of E4 or 5, wherein the polysorbate is polysorbate 80.
E7. The formulation of any one of E1-6, wherein the formulation comprises about 1-40 mg/ml of the anti-EGFR ADC.
E8. The formulation of any one of E1-7, wherein the formulation comprises about 60-80 mg/ml of the sugar.
E9. The formulation of any one of E1-8, wherein the sugar is selected from the group consisting of mannitol, sorbitol, sucrose, and trehalose.
E10. The formulation of any one of E 1-9, comprising about 5-25 mM histidine.
E11. The formulation of any one of E1-10, wherein the formulation has a pH of about 5.5 to 6.5.
E12. The formulation of any one of E1-11, wherein the formulation is lyophilized.
E13. The formulation of any one of E1-3, wherein the formulation is lyophilized and the sugar is sucrose.
E14. The formulation of E13, wherein the surfactant is a polysorbate.
E15. The formulation of E14, wherein the polysorbate is polysorbate 80.
E16. The formulation of any one of E1-3, wherein when said formulation is an aqueous formulation comprising about 1 - 100 mg/ml of the anti-EGFR ADC, about 1 -10 mg/mL histidine, about 50 -90 mg/ml of the sugar, and about 0.01 - 0.2 mg/ml of the surfactant.
E17. The formulation of E16, wherein the sugar is sucrose.
E18. The formulation of E16 or 17, wherein the surfactant is polysorbate 80.
E19. The formulation of any one of E16-18, wherein the formulation has a pH of about 5.5 to 6.5.
E20. The formulation of any one of E 16-19, comprising 1-150 mg/ml of the anti-EGFR ADC.
E21. The formulation of E20, comprising 1-40 mg/ml of the anti-EGFR ADC.
E22. The formulation of any one of E1-21, wherein the antibody is humanized.
E 23. The formulation of any one of E 1-22, wherein the antibody, or antigen-binding portion thereof, comprises a heavy chain variable region comprising the amino acid sequence set forth in SEQ ID NO: 13, and a light chain variable region comprising the amino acid sequence set forth in SEQ ID NO: 18.
E 24. The formulation of any one of E 1-22, wherein said antibody, or antigen-binding portion thereof, comprises a heavy chain variable region comprising complementarity domain regions (CDRs) comprising the amino acid sequences set forth in SEQ ID NOS: 15, 16, and 17, and comprises a light chain variable region comprising CDRs comprising the amino acid sequences set forth in SEQ ID NOS: 20, 21, and 22.
E 25. The formulation of any one of E1-3, wherein in the formulation is a lyophilized formulation comprising sucrose, polysorbate 80, and histidine, and wherein the antibody, or antigen-binding portion thereof, comprises a heavy chain variable region comprising complementarity domain regions (CDRs) comprising the amino acid sequences set forth in SEQ ID NOS: 15, 16, and 17, and comprises a light chain variable region comprising CDRs comprising the amino acid sequences set forth in SEQ ID NOS: 20, 21, and 22.
E 26. The formulation of E25, comprising 1-120 mg of the anti-EGFR ADC.
E 27. The formulation of any one of E1-26, wherein the formulation comprises an ADC mixture having an average DAR of about 3 or an ADC mixture having a DAR of about 2-4.
E28. A formulation comprising
   an anti-EGFR ADC comprising an anti-EGFR antibody, or antigen-binding portion thereof, conjugated to monomethylauristatin F (MMAF), wherein said ADC 1-MMAF comprises a heavy chain variable region comprising complementarity domain regions (CDRs) comprising the amino acid sequences set forth in SEQ ID NOS: 15, 16, and 17, and comprises a light chain variable region comprising CDRs comprising the amino acid sequences set forth in SEQ ID NOS: 20, 21, and 22,
   sucrose,
   histidine, and
   polysorbate 80,
   wherein the formulation comprises an ADC mixture having an average DAR of about 3 or an ADC mixture having a DAR of about 2-4.
E29. The formulation of E28, which is lyophilized.
E30. The formulation of E28 or 29, wherein the anti-EGFR antibody, or antigen-binding portion thereof, comprises a heavy chain variable region comprising the amino acid sequence set forth in SEQ ID NO: 13, and a light chain variable region comprising the amino acid sequence set forth in SEQ ID NO: 18.
E 31. The formulation of any one of E1-30, which is a pharmaceutical formulation.
E 32. A method of preparing the formulation of any one of E1-3 or 29, said method comprising lyophilizing an aqueous formulation having a pH ranging from about 5 to 7 and comprising 1-20 mg of histidine, about 320-410 mg of the sugar, about 0.1 to 0.9 mg of the surfactant, and about 1-150 mg of the anti-EGFR ADC.
E 33. A method for treating a subject comprising administering a therapeutically effective amount of the formulation of any one of E1-31 to a subject, wherein the subject has a disorder requiring treatment with the anti-EGFR ADC.
E 34. The method of E33, wherein the disorder requiring treatment with the anti-EGFR ADC is cancer.
E 35. The method of E34, wherein the cancer is selected from the group consisting of: glioblastoma, non-small cell lung cancer, lung cancer, colon cancer, head and neck cancer, breast cancer, squamous cell tumors, anal cancer, skin cancer, and vulvar cancer.
E 36. The method of E34, wherein the cancer is selected from the group consisting of squamous tumors, glioblastoma, glioma, non-small cell lung cancer (NSCLC), lung cancer, colon cancer, head and neck cancer, breast cancer, anal cancer, skin cancer, and vulvar cancer.
E 37. The method of E36, wherein the squamous tumor is selected from the group consisting of squamous lung tumor, a squamous tumor of the head or neck, and a squamous cervical tumor.
E 38. The method of E 34, wherein the cancer is a solid tumor having overexpression of EGFR.
E 39. The method of any one of E33-38, wherein the formulation is administered intravenously.
E40. A kit comprising the formulation of any one of E1-29.

## Claims

1. A lyophilized formulation comprising an anti-Epidermal Growth Factor Receptor (EGFR) antibody drug conjugate (ADC), a sugar, histidine, and a surfactant, and wherein said anti-EGFR ADC comprises an anti-EGFR antibody conjugated to an auristatin.

2. The formulation of claim 1, wherein the auristatin is Monomethylauristatin F (MMAF), optionally wherein the MMAF is conjugated to the antibody with a maleimidocaproyl linker.

3. The formulation of any one of claims 1-2, wherein the surfactant is a polysorbate, optionally wherein the formulation is reconstituted and comprises 0.01-0.2 mg/ml of the polysorbate, e.g. polysorbate 80.

4. The formulation of any one of claims 1-3, wherein the formulation comprises about 120 mg of the anti-EGFR ADC.

5. The formulation of any one of claims 1-3, wherein the formulation is reconstituted and comprises:
(a) about 1-100 mg/ml of the anti-EGFR ADC;
(b) about 50-90 mg/ml of the sugar, optionally wherein the sugar is selected from the group consisting of mannitol, sorbitol, sucrose, and trehalose; and/or
(c) about 1-10 mg/mL histidine,
optionally wherein the formulation has a pH of about 5.5 to 6.5 upon reconstitution.

6. The formulation of claim 1 or claim 2, wherein the sugar is sucrose, optionally wherein the surfactant is a polysorbate, e.g. polysorbate 80.

7. The formulation of claim 1 or claim 2, wherein said formulation is reconstituted and comprises about 1 - 100 mg/ml of the anti-EGFR ADC, about 1-10 mg/mL histidine, about 50 - 90 mg/ml of the sugar, and about 0.01 - 0.2 mg/ml of the surfactant.

8. The formulation of claim 7, wherein the sugar is sucrose, optionally wherein the surfactant is polysorbate 80 and/or the formulation has a pH of about 5.5 to 6.5.

9. The formulation of claim 7 or claim 8, comprising 10-100 mg/ml of the anti-EGFR ADC.

10. The formulation of claim 9, comprising 1-40 mg/ml of the anti-EGFR ADC.

11. The formulation of any one of claims 1-10, wherein the antibody:
(a) comprises a heavy chain variable region comprising the amino acid sequence set forth in SEQ ID NO: 13, and a light chain variable region comprising the amino acid sequence set forth in SEQ ID NO: 18; and/or
(b) comprises a heavy chain constant region comprising the amino acid sequence as set forth in SEQ ID NO: 14 and/or a light chain constant region comprising the amino acid sequence as set forth in SEQ ID NO: 19.

12. The formulation of any one of claims 1-11, wherein the formulation comprises an ADC mixture having an average DAR of about 3 or an ADC mixture having a DAR of about 2-4.

13. A lyophilized formulation comprising
an anti-EGFR ADC comprising an anti-EGFR antibody conjugated to monomethylauristatin F (MMAF), wherein said ADC comprises a heavy chain variable region comprising complementarity domain regions (CDRs) comprising the amino acid sequences set forth in SEQ ID NOS: 15, 16, and 17, and comprises a light chain variable region comprising CDRs comprising the amino acid sequences set forth in SEQ ID NOS: 20, 21, and 22,
sucrose,
histidine, and
polysorbate 80,
wherein the formulation comprises an ADC mixture having an average DAR of about 3 or an ADC mixture having a DAR of about 2-4, wherein
(a) the anti-EGFR antibody comprises a heavy chain constant region comprising the amino acid sequence as set forth in SEQ ID NO: 14 and/or a light chain constant region comprising the amino acid sequence as set forth in SEQ ID NO: 19; or
(b) the anti-EGFR antibody is an IgGl isotype.

14. The formulation of any one of claims 1-13, which is a pharmaceutical formulation.

15. The formulation of any one of claims 1-14, for use in a method of treating a disorder requiring treatment with the anti-EGFR ADC, optionally wherein:
(a) the disorder requiring treatment with the anti-EGFR ADC is cancer; optionally wherein
(i) the cancer is selected from the group consisting of: glioblastoma, non-small cell lung cancer, lung cancer, colon cancer, head and neck cancer, breast cancer, squamous cell tumors, anal cancer, skin cancer, and vulvar cancer; or the cancer is selected from the group consisting of squamous tumors, glioblastoma, glioma, non-small cell lung cancer (NSCLC)squamous lung tumor, a squamous tumor of the head or neck, and a squamous cervical tumor:
(ii) the cancer is a solid tumor having overexpression of EGFR.

16. The formulation for use according to claim 15, wherein the formulation is administered intravenously.

17. A kit comprising the formulation of any one of claims 1-13.


SEQUENCE LISTING

<110> ABBVIE DEUTSCHLAND GMBH & CO. KG
      ABBVIE INC.

<120> ANTI-EGFR ANTIBODY DRUG CONJUGATE FORMULATIONS

<130> P45932EP-D1-PCT

<140> To be assigned
<141> 2014-03-14

<150> US 61/790,490
<151> 2013-03-15

<160> 22

<170> PatentIn version 3.5

<210> 1
<211> 402
<212> DNA
<213> Artificial Sequence

<220>
<223> Description of Artificial Sequence: Synthetic
      polynucleotide

<400> 1
atgagagtgc tgattctttt gtggctgttc acagcctttc ctggtgtcct gtctgatgtg      60

cagcttcagg agtcgggacc tagcctggtg aaaccttctc agtctctgtc cctcacctgc     120

actgtcactg gctactcaat caccagtgat tttgcctgga actggatccg gcagtttcca     180

ggaaacaagc tggagtggat gggctacata agttatagtg gtaacactag gtacaaccca     240

tctctcaaaa gtcgaatctc tatcactcga gacacatcca agaaccaatt cttcctgcag     300

ttgaattctg tgactattga ggacacagcc acatattact gtgtaacggc gggacgcggg     360

tttccttatt ggggccaagg gactctggtc actgtctctg ca                        402


<210> 2
<211> 134
<212> PRT
<213> Artificial Sequence

<220>
<223> Description of Artificial Sequence: Synthetic
      polypeptide

<400> 2
Met Arg Val Leu Ile Leu Leu Trp Leu Phe Thr Ala Phe Pro Gly Val
1               5                   10                  15


Leu Ser Asp Val Gln Leu Gln Glu Ser Gly Pro Ser Leu Val Lys Pro
            20                  25                  30


Ser Gln Ser Leu Ser Leu Thr Cys Thr Val Thr Gly Tyr Ser Ile Thr

35                  40                  45

Ser Asp Phe Ala Trp Asn Trp Ile Arg Gln Phe Pro Gly Asn Lys Leu
    50                  55                  60

Glu Trp Met Gly Tyr Ile Ser Tyr Ser Gly Asn Thr Arg Tyr Asn Pro
65                  70                  75                  80

Ser Leu Lys Ser Arg Ile Ser Ile Thr Arg Asp Thr Ser Lys Asn Gln
                85                  90                  95

Phe Phe Leu Gln Leu Asn Ser Val Thr Ile Glu Asp Thr Ala Thr Tyr
            100                 105                 110

Tyr Cys Val Thr Ala Gly Arg Gly Phe Pro Tyr Trp Gly Gln Gly Thr
        115                 120                 125

Leu Val Thr Val Ser Ala
    130

<210> 3
<211> 384
<212> DNA
<213> Artificial Sequence

<220>
<223> Description of Artificial Sequence: Synthetic
      polynucleotide

<400> 3
atggtgtcca cagctcagtt ccttgcattc ttgttgcttt ggtttccagg tgcaagatgt      60

gacatcctga tgacccaatc tccatcctcc atgtctgtat ctctgggaga cacagtcagc     120

atcacttgcc attcaagtca ggacattaac agtaatatag ggtggttgca gcagagacca     180

gggaaatcat ttaagggcct gatctatcat ggaaccaact tggacgatga agttccatca     240

aggttcagtg gcagtggatc tggagccgat tattctctca ccatcagcag cctggaatct     300

gaagattttg cagactatta ctgtgtacag tatgctcagt ttccgtggac gttcggtgga     360

ggcaccaagc tggaaatcaa acgt                                            384

<210> 4
<211> 128
<212> PRT
<213> Artificial Sequence

<220>
<223> Description of Artificial Sequence: Synthetic
      polypeptide

<400> 4
Met Val Ser Thr Ala Gln Phe Leu Ala Phe Leu Leu Leu Trp Phe Pro

1               5                   10                  15

Gly Ala Arg Cys Asp Ile Leu Met Thr Gln Ser Pro Ser Ser Met Ser
            20                  25                  30

Val Ser Leu Gly Asp Thr Val Ser Ile Thr Cys His Ser Ser Gln Asp
        35                  40                  45

Ile Asn Ser Asn Ile Gly Trp Leu Gln Gln Arg Pro Gly Lys Ser Phe
    50                  55                  60

Lys Gly Leu Ile Tyr His Gly Thr Asn Leu Asp Asp Glu Val Pro Ser
65                  70                  75                  80

Arg Phe Ser Gly Ser Gly Ser Gly Ala Asp Tyr Ser Leu Thr Ile Ser
                85                  90                  95

Ser Leu Glu Ser Glu Asp Phe Ala Asp Tyr Tyr Cys Val Gln Tyr Ala
            100                 105                 110

Gln Phe Pro Trp Thr Phe Gly Gly Gly Thr Lys Leu Glu Ile Lys Arg
        115                 120                 125

<210> 5
<211> 6
<212> PRT
<213> Artificial Sequence

<220>
<223> Description of Artificial Sequence: Synthetic
      peptide

<400> 5
Ser Asp Phe Ala Trp Asn
1               5

<210> 6
<211> 16
<212> PRT
<213> Artificial Sequence

<220>
<223> Description of Artificial Sequence: Synthetic
      peptide

<400> 6
Tyr Ile Ser Tyr Ser Gly Asn Thr Arg Tyr Asn Pro Ser Leu Lys Ser
1               5                   10                  15

<210> 7
<211> 9
<212> PRT
<213> Artificial Sequence

<220>
<223> Description of Artificial Sequence: Synthetic
      peptide

<400> 7
Val Thr Ala Gly Arg Gly Phe Pro Tyr
1               5


<210> 8
<211> 11
<212> PRT
<213> Artificial Sequence

<220>
<223> Description of Artificial Sequence: Synthetic
      peptide

<400> 8
His Ser Ser Gln Asp Ile Asn Ser Asn Ile Gly
1               5                   10


<210> 9
<211> 7
<212> PRT
<213> Artificial Sequence

<220>
<223> Description of Artificial Sequence: Synthetic
      peptide

<400> 9
His Gly Thr Asn Leu Asp Asp
1               5


<210> 10
<211> 9
<212> PRT
<213> Artificial Sequence

<220>
<223> Description of Artificial Sequence: Synthetic
      peptide

<400> 10
Val Gln Tyr Ala Gln Phe Pro Trp Thr
1               5


<210> 11
<211> 116
<212> PRT
<213> Artificial Sequence

<220>
<223> Description of Artificial Sequence: Synthetic
      polypeptide

<400> 11
Asp Val Gln Leu Gln Glu Ser Gly Pro Ser Leu Val Lys Pro Ser Gln

1               5                   10                  15

Ser Leu Ser Leu Thr Cys Thr Val Thr Gly Tyr Ser Ile Thr Ser Asp
            20                  25                  30

Phe Ala Trp Asn Trp Ile Arg Gln Phe Pro Gly Asn Lys Leu Glu Trp
        35                  40                  45

Met Gly Tyr Ile Ser Tyr Ser Gly Asn Thr Arg Tyr Asn Pro Ser Leu
    50                  55                  60

Lys Ser Arg Ile Ser Ile Thr Arg Asp Thr Ser Lys Asn Gln Phe Phe
65                  70                  75                  80

Leu Gln Leu Asn Ser Val Thr Ile Glu Asp Thr Ala Thr Tyr Tyr Cys
                85                  90                  95

Val Thr Ala Gly Arg Gly Phe Pro Tyr Trp Gly Gln Gly Thr Leu Val
            100                 105                 110

Thr Val Ser Ala
        115

<210> 12
<211> 108
<212> PRT
<213> Artificial Sequence

<220>
<223> Description of Artificial Sequence: Synthetic
      polypeptide

<400> 12
Asp Ile Leu Met Thr Gln Ser Pro Ser Ser Met Ser Val Ser Leu Gly
1               5                   10                  15

Asp Thr Val Ser Ile Thr Cys His Ser Ser Gln Asp Ile Asn Ser Asn
            20                  25                  30

Ile Gly Trp Leu Gln Gln Arg Pro Gly Lys Ser Phe Lys Gly Leu Ile
        35                  40                  45

Tyr His Gly Thr Asn Leu Asp Asp Glu Val Pro Ser Arg Phe Ser Gly
    50                  55                  60

Ser Gly Ser Gly Ala Asp Tyr Ser Leu Thr Ile Ser Ser Leu Glu Ser
65                  70                  75                  80

Glu Asp Phe Ala Asp Tyr Tyr Cys Val Gln Tyr Ala Gln Phe Pro Trp
                85                  90                  95

Thr Phe Gly Gly Gly Thr Lys Leu Glu Ile Lys Arg
            100                 105

<210> 13
<211> 116
<212> PRT
<213> Artificial Sequence

<220>
<223> Description of Artificial Sequence: Synthetic
      polypeptide

<400> 13
Gln Val Gln Leu Gln Glu Ser Gly Pro Gly Leu Val Lys Pro Ser Gln
1               5                   10                  15

Thr Leu Ser Leu Thr Cys Thr Val Ser Gly Tyr Ser Ile Ser Ser Asp
            20                  25                  30

Phe Ala Trp Asn Trp Ile Arg Gln Pro Pro Gly Lys Gly Leu Glu Trp
        35                  40                  45

Met Gly Tyr Ile Ser Tyr Ser Gly Asn Thr Arg Tyr Gln Pro Ser Leu
    50                  55                  60

Lys Ser Arg Ile Thr Ile Ser Arg Asp Thr Ser Lys Asn Gln Phe Phe
65                  70                  75                  80

Leu Lys Leu Asn Ser Val Thr Ala Ala Asp Thr Ala Thr Tyr Tyr Cys
                85                  90                  95

Val Thr Ala Gly Arg Gly Phe Pro Tyr Trp Gly Gln Gly Thr Leu Val
            100                 105                 110

Thr Val Ser Ser
        115

<210> 14
<211> 330
<212> PRT
<213> Artificial Sequence

<220>
<223> Description of Artificial Sequence: Synthetic
      polypeptide

<400> 14

<210> 15
<211> 6
<212> PRT
<213> Artificial Sequence

<220>
<223> Description of Artificial Sequence: Synthetic
      peptide

<400> 15
Ser Asp Phe Ala Trp Asn
1               5

<210> 16
<211> 16
<212> PRT
<213> Artificial Sequence

<220>
<223> Description of Artificial Sequence: Synthetic
      peptide

<400> 16
Tyr Ile Ser Tyr Ser Gly Asn Thr Arg Tyr Gln Pro Ser Leu Lys Ser
1               5                   10                  15

<210> 17
<211> 9
<212> PRT
<213> Artificial Sequence

<220>
<223> Description of Artificial Sequence: Synthetic
      peptide

<400> 17
Val Thr Ala Gly Arg Gly Phe Pro Tyr
1               5

<210> 18
<211> 108
<212> PRT
<213> Artificial Sequence

<220>
<223> Description of Artificial Sequence: Synthetic
      polypeptide

<400> 18
Asp Ile Gln Met Thr Gln Ser Pro Ser Ser Met Ser Val Ser Val Gly
1               5                   10                  15

Asp Arg Val Thr Ile Thr Cys His Ser Ser Gln Asp Ile Asn Ser Asn
            20                  25                  30

Ile Gly Trp Leu Gln Gln Lys Pro Gly Lys Ser Phe Lys Gly Leu Ile
        35                  40                  45

Tyr His Gly Thr Asn Leu Asp Asp Gly Val Pro Ser Arg Phe Ser Gly
    50                  55                  60

Ser Gly Ser Gly Thr Asp Tyr Thr Leu Thr Ile Ser Ser Leu Gln Pro
65                  70                  75                  80

Glu Asp Phe Ala Thr Tyr Tyr Cys Val Gln Tyr Ala Gln Phe Pro Trp
                85                  90                  95

Thr Phe Gly Gly Gly Thr Lys Leu Glu Ile Lys Arg
            100                 105

<210> 19
<211> 106
<212> PRT
<213> Artificial Sequence

<220>
<223> Description of Artificial Sequence: Synthetic
      polypeptide

<400> 19
Thr Val Ala Ala Pro Ser Val Phe Ile Phe Pro Pro Ser Asp Glu Gln
1               5                   10                  15

Leu Lys Ser Gly Thr Ala Ser Val Val Cys Leu Leu Asn Asn Phe Tyr
            20                  25                  30

Pro Arg Glu Ala Lys Val Gln Trp Lys Val Asp Asn Ala Leu Gln Ser
        35                  40                  45

Gly Asn Ser Gln Glu Ser Val Thr Glu Gln Asp Ser Lys Asp Ser Thr
    50                  55                  60

Tyr Ser Leu Ser Ser Thr Leu Thr Leu Ser Lys Ala Asp Tyr Glu Lys
65                  70                  75                  80

His Lys Val Tyr Ala Cys Glu Val Thr His Gln Gly Leu Ser Ser Pro
                85                  90                  95

Val Thr Lys Ser Phe Asn Arg Gly Glu Cys
            100                 105

<210> 20
<211> 11
<212> PRT
<213> Artificial Sequence

<220>
<223> Description of Artificial Sequence: Synthetic
      peptide

<400> 20
His Ser Ser Gln Asp Ile Asn Ser Asn Ile Gly
1               5                   10

<210> 21
<211> 7
<212> PRT
<213> Artificial Sequence

<220>
<223> Description of Artificial Sequence: Synthetic
      peptide

<400> 21
His Gly Thr Asn Leu Asp Asp
1               5

<210> 22
<211> 9
<212> PRT
<213> Artificial Sequence

<220>
<223> Description of Artificial Sequence: Synthetic
      peptide

<400> 22
Val Gln Tyr Ala Gln Phe Pro Trp Thr
1               5